# EUROPEAN PATENT SPECIFICATION

(11) **EP 1 382 687 B1**
(45) Date of publication and mention of the grant of the patent: **26.05.2010**
(21) Application number: 02724644.6
(22) Date of filing: 25.04.2002
(51) Int. Cl.: C12P 19/12, A23G 3/00, A23K 1/16, A23L 1/236, A23L 1/29

(54) **PROCESS FOR PRODUCING ISOMALTOSE AND USE THEREOF**
VERFAHREN ZUR HERSTELLUNG VON ISOMALTOSE UND DEREN VERWENDUNG
PROCESSUS DE PRODUCTION D'ISOMALTOSE ET UTILISATION DE CELUI-CI

(30) Priority: 27.04.2001 JP 2001130922
(43) Date of publication of application: 21.01.2004
(73) Proprietor: KABUSHIKI KAISHA HAYASHIBARA SEIBUTSU KAGAKU KENKYUJO, Okayama-shi, Okayama 700-0907 (JP)
(72) Inventor: KUBOTA, Michio, c/o K.K. Hayashibara, Okayama-shi, Okayama 700-0907 (JP); NISHIMOTO, Tomoyuki, c/o K.K. Hayashibara, Okayama-shi, Okayama 700-0907 (JP); HIGASHIYAMA, Takanobu, c/o K.K. Hayashibara, Okayama-shi, Okayama 700-0907 (JP); WATANABE, Hikaru, c/o K.K. Hayashibara, Okayama-shi, Okayama 700-0907 (JP); FUKUDA, Shigeharu, c/o K.K. Hayashibara, Okayama-shi, Okayama 700-0907 (JP); MIYAKE, Toshio, c/o K.K. Hayashibara, Okayama-shi, Okayama 700-0907 (JP)
(74) Representative: Daniels, Jeffrey Nicholas
(86) International application number: PCT/JP2002/004166
(87) International publication number: WO 2002/088374

(56) References cited:
- EP-A- 0 608 636
- EP-A- 0 875 585
- EP-A2- 0 990 704
- WO-A-99/27124
- WO-A1-01/90338
- WO-A1-02/10361
- JP-A- 63 216 493
- JP-A- 63 287 496
- US-A- 4 521 252
- KIM Y-K ET AL: "ENZYMATIC PREPARATION OF NOVEL NON-REDUCING OLIGOSACCHARIDES HAVINGAN ISOMALTOSYL RESIDUE BY USING THE TRANSFER ACTION OF ISOMALTODEXTRANASE FROM ARTHOBACTER GLOBIFORMIS T6" BIOSCIENCE, BIOTECHNOLOGY AND BIOCHEMISTRY, vol. 59, no. 7, 1995, pages 1367-1369, XP002948394 ISSN: 0916-8451
- COTE G.I. ET AL.: 'Enzymatically produced cyclic alpha-1,3-linked and alpha-1,6-linked oligosaccharides of D-glucose' EUR. J. BIOCHEM. vol. 226, 1994, pages 641 - 648, XP002954139
- HAYASHI A. ET AL.: 'The enzymatic reaction for the production of panose and isomaltose by glucosyltransferase from aureobasidium' LETTERS IN APPLIED MICROBIOLOGY vol. 19, 1994, pages 247 - 248, XP002954140

## Description

### TECHNICAL FIELD

The present invention relates to a novel process for producing isomaltose and uses thereof, more particularly, a process for producing isomaltose from saccharides, which have both a glucose polymerization degree of at least two and an α-1,4 glucosidic linkage as a linkage at the non-reducing end, in a relatively high yield.

### BACKGROUND ART

EP-A-0608636 discloses cycloisomaltooligosaccharides, an enzyme and process for producing said oligosaccharides, and a process for producing said enzyme.

WO99/27124A discloses enzymatic starch saccharification including a membrane separation step.

EP-A-0875585 discloses a method for the production of isomalto-oligosaccharide rich syrups.

Kim et al., (Biosci. Biotech. Biochem., 59(7), 1367-1369, 1995) discloses the enzymatic preparation of novel non-reducing oligosaccharides having an isomaltosyl residue by using the transfer action of isomaltodextranase from *Arthrobacter globiformis* T6.

Côté and Biely (Eur. J. Biochem. 226, 641-648 (1994) discloses enzymically produced cyclic α-1,3-linked and α-1,6-linked oligosaccharides of D-glucose.

JP63287496A discloses the production of high conversion syrup containing isomaltose.

JP63216493A discloses the production of high-purity isomaltose.

Isomaltose is a substantially non-crystallizable saccharide which is slightly present in fermented foods and has a relatively low sweetness and satisfactory humectancy. The saccharide has been widely used in a mixture form with saccharides such as glucose, maltose and panose in foods, cosmetics, pharmaceuticals, etc.

Isomaltose is a rare saccharide slightly present in fermented foods, etc., in the natural world. On an industrial-scale production, the following methods for producing isomaltose have been known; partial hydrolysis reaction of dextrans using acid catalysts, enzymatic reactions using dextranase or isomaltodextranase, etc., reverse synthetic reactions from glucose using glucoamylase or acid catalysts, and glucose saccharide-transferring reactions from maltose or maltodextrins using α-glucosidase. However, the isomaltose content of reaction mixtures obtained by conventional methods is only about 10 to about 25% (w/w), on a dry solid basis (d.s.b.) (throughout the specification, "% (w/w)" is abbreviated as "%", unless specified otherwise), and therefore it is far from satisfaction in view of the purity of isomaltose on an industrial-scale production. As a method for improving the drawback, a column chromatography, as disclosed in Japanese Patent Kokai No. 72,598/83, can be exemplified. According to the method, a high purity isomaltose is obtained from a material saccharide solution with an isomaltose content of about 10 to about 25%, d.s.b. However, the method has the drawback that the purity and yield of isomaltose inevitably depends on the isomaltose content in the material saccharide solutions used.

Under the background, it has been in a great demand a novel process for producing isomaltose on an industrial scale, at a lesser cost, and in a relatively high yield.

In view of the prior arts, the object of the present invention is to establish a process for producing isomaltose which produces isomaltose on an industrial scale, at a lesser cost, and in a relatively high yield.

### DISCLOSURE OF INVENTION

During the present inventors' energetic studying to solve the above object, it has reported in European Journal of Biochemistry, Vol. 226, pp. 641-648 (1994) a cyclic tetrasaccharide having the structure of cyclo{→6)-α-D-glucopyranosyl-(1→3)-α-D-glucopyranosyl-(1→6)-α-D-glucopyranosyl-(1→3)-α-D-glucopyranosyl-(1→} (abbreviated as "cyclotetrasaccharide" throughout the specification), formed by allowing a hydrolyzing enzyme, i.e., alternanase, to act on alternan linked with glucose residues via the alternating α-1,3 and α-1,6 linkages.

While, in the specification of Japanese Patent Application No. 229,557/00, the present inventors disclosed a process for producing cyclotetrasaccharide using an α-isomaltosyl-transferring enzyme which forms cyclotetrasaccharide from saccharides such as panose derived from starches; and disclosed in Japanese Patent Application No. 234,937/00 a process for producing cyclotetrasaccharide in a satisfactorily high yield by allowing the above α-isomaltosyl-transferring enzyme and an α-isomaltosylglucosaccharide-forming enzyme which forms α-isomaltosylglucosaccharide from maltooligosaccharides.

Thereafter, the present inventors focused on the fact that the above α-isomaltosylglucosaccharide and cyclotetrasaccharide have an isomaltose structure intramolecularly, and then studied a method for producing isomaltose from these saccharides. As the result of studying on the enzymatic reaction mechanisms of the above α-isomaltosylglucosaccharide-forming enzyme and α-isomaltosyl-transferring enzyme, the present inventors found that the production yield of isomaltose is outstandingly improved by allowing α-isomaltosylglucosaccharide-forming enzyme and isomaltose-releasing enzyme capable of releasing isomaltose, in the presence or the absence of α-isomaltosyl-transferring enzyme, to act on saccharides having both a glucose polymerization degree of at least two and α-1,4 glucosidic linkage as a linkage at the non-reducing end; and found that the method is easily feasible on an industrial scale. The present inventors also established the uses of isomaltose thus obtained, and accomplished this invention: They accomplished the following process and uses thereof and solved the object of the present invention.

Accordingly, the present invention provides a process for producing isomaltose as defined in claim 1 which comprises the steps of: (a) allowing α-isomaltosylglucosaccharide-forming enzyme which forms α-isomaltosylglucosaccharide having a glucose polymerization degree of at least three, α-1,6 glucosidic linkage as a linkage at the non-reducing end, and α-1,4 glucosidic linkage as a linkage other than the non-reducing end; to act on a saccharide having both a glucose polymerization degree of at least two and α-1,4 glucosidic linkage as a linkage at the non-reducing end to form said α-isomaltosylglucosaccharide;
(b) allowing isomaltodextranase to act on said α-isomaltosylglucosaccharide to release isomaltose; and
(c) collecting the released isomaltose. The present invention also provides a process as described above wherein said α-isomaltosylglucosaccharide-forming enzyme has the following physicochemical properties:
   (1) Action
      Forming a saccharide, which has a glucose polymerization degree of at least three, α-1,6 glucosidic linkage as a linkage at the non-reducing end, and α-1,4 glucosidic linkage other than the linkage at the non-reducing end, by catalyzing the α-glucosyl-transferring reaction from a saccharide having both a glucose polymerization degree of at least two and having α-1,4 glucosidic linkage as a linkage at the non-reducing end without substantially increasing the reducing power;
   (2) Molecular weight
      Having a molecular weight of 117,000 to 160,000 daltons when determined on SDS-PAGE;
   (3) Isoelectric point (pI)
      Having an isoelectric point of 4.7 to 5.7 when determined on isoelectrophoresis using ampholine;
   (4) Optimum temperature
      Having an optimum temperature of 40°C to 45°C when incubated at a pH of 6.0 for 60 min, or an optimum temperature of 45°C to 50°C when incubated in the presence of 1 mM Ca²⁺;
   (5) Optimum pH
      Having an optimum pH of 6.0 to 6.5 when incubated at 35°C for 60 min;
   (6) Thermal stability
      Stable up to a temperature of 35°C to 40°C, or a temperature of about 40°C to 45°C in the presence of 1 mM Ca²⁺; and
   (7) pH Stability
      Stable at a pH of 4.5 to 10.0 when incubated at 4°C for 24 hours.

Further the present invention provides a process for producing isomaltose as described above characterized in that one or more enzymes selected from the group consisting of α-isomaltosyl-transferring enzyme, cyclomaltodextrin glucanotransferase and starch debranching enzyme are used along with the α-isomaltosylglucosaccharide-forming enzyme in the step (a). Still further, the present invention provides the process of the above paragraph wherein said α-isomaltosyl-transferring enzyme has the following physicochemical properties:
(1) Action
   Acting on an α-isomaltosylglucosaccharide, which has a glucose polymerization degree of at least three, α-1,6 glucosidic linkage as a linkage at the non-reducing end, and α-1,4 glucosidic linkage as a linkage other than the non-reducing end, to form cyclotetrasaccharide having a structure of cyclo{→6) - α-D-glucopyranosyl- (1→3) - α-D-glucopyranosyl-(1→6)-α-D-glucopyranosyl-(1→3)-α-D-glucopyranosyl-(1→} through α-isomaltosyl transfer;
(2) Molecular weight
   Having a molecular weight of 82,000 to 132,000 daltons when determined on SDS-PAGE;
(3) Isoelectric point (pI)
   Having an isoelectric point of 5.0 to 6.1 when determined on isoelectrophoresis using ampholine;
(4) Optimum temperature
   Having an optimum temperature of 45°C to 50°C when incubated at a pH of 6.0 for 30 min;
(5) Optimum pH
   Having an optimum pH of 5.5 to 6.0 when incubated at 35°C for 30 min;
(6) Thermal stability
   Stable up to a temperature of 40°C when incubated at a pH of 6.0 for 60 min; and
(7) pH Stability
   Stable at a pH of 4.0 to 9.0 when incubated at 4°C for 24 hours.

The present invention provides a process for producing isomaltoseas described above wherein said saccharide having both a glucose polymerization degree of at least two and α-1,4 glucosidic linkage as a linkage at the non-reducing end is one selected from the group consisting of maltooligosaccharides, maltodextrins, amylodextrins, amyloses, amylopectins, soluble starches, liquefied starches, and glycogens.
The present invention also provides a process for producing isomaltose as first described above characterized in that a column chromatography using an alkaline metal and/or alkaline earth metal strong-acid cation exchange resin is used in step (c).

### BRIEF DESCRIPTION OF DRAWINGS

FIG. 1 shows the thermal influence on the enzymatic activity of α-isomaltosylglucosaccharide-forming enzyme from a microorganism of *Bacillus globiformis* C9 strain.
FIG. 2 shows the pH influence on the enzymatic activity of α-isomaltosylglucosaccharide-forming enzyme from a microorganism of *Bacillus globiformis* C9 strain.
FIG. 3 shows the thermal stability of α-isomaltosylglucosaccharide-forming enzyme from a microorganism of *Bacillus globiformis* C9 strain.
FIG. 4 shows the pH stability of α-isomaltosylglucosaccharide-forming enzyme from a microorganism of *Bacillus globiformis* C9 strain.
FIG. 5 shows the thermal influence on the enzymatic activity of α-isomaltosyl-transferring enzyme from a microorganism of *Bacillus globiformis* C9 strain.
FIG. 6 shows the pH influence on the enzymatic activity of α-isomaltosyl-transferring enzyme from a microorganism of *Bacillus globiformis* C9 strain.
FIG. 7 shows the thermal stability of α-isomaltosyl-transferring enzyme from a microorganism of *Bacillus globiformis* C9 strain.
FIG. 8 shows the pH stability of α-isomaltosyl-transferring enzyme from a microorganism of *Bacillus globiformis* C9 strain.
FIG. 9 shows the thermal influence on the enzymatic activity of α-isomaltosylglucosaccharide-forming enzyme from a microorganism of *Bacillus globisporus* C11 strain.
FIG. 10 shows the pH influence on α-isomaltosylglucosaccharide-forming enzyme from a microorganism of *Bacillus globisporus* C11 strain.
FIG. 11 shows the thermal stability of α-isomaltosylglucosaccharide-forming enzyme from a microorganism of *Bacillus globisporus* C11 strain.
FIG. 12 shows the pH stability of α-isomaltosylglucosaccharide-forming enzyme from a microorganism of *Bacillus globisporus* C11 strain.
FIG. 13 shows the thermal influence on the enzymatic activity of α-isomaltosyl-transferring enzyme from a microorganism of *Bacillus globisporus* C11 strain.
FIG. 14 shows the pH influence on the enzymatic activity of α-isomaltosyl-transferring enzyme from a microorganism of *Bacillus globisporus* C11 strain.
FIG. 15 shows the thermal stability of α-isomaltosyl-transferring enzyme from a microorganism of *Bacillus globisporus* C11 strain.
FIG. 16 shows the pH stability of α-isomaltosyl-transferring enzyme from a microorganism of *Bacillus globisporus* C11 strain.
FIG. 17 is a nuclear resonance spectrum (¹H-NMR) of α-isomaltosylmaltotriose, obtained by the enzymatic reaction using α-isomaltosylglucosaccharide-forming enzyme.
FIG. 18 is a nuclear resonance spectrum (¹H-NMR) of α-isomaltosylmaltotetraose, obtained by the enzymatic reaction using α-isomaltosylglucosaccharide-forming enzyme.
FIG. 19 is a nuclear resonance spectrum (¹³C-NMR) of α-isomaltosylmaltotriose, obtained by the enzymatic reaction using α-isomaltosylglucosaccharide-forming enzyme.
FIG. 20 is a nuclear resonance spectrum (¹³C-NMR) of α-isomaltosylmaltotetraose, obtained by the enzymatic reaction using α-isomaltosylglucosaccharide-forming enzyme.
FIG. 21 is a nuclear resonance spectrum (¹H-NMR) of the product A.
FIG. 22 is a nuclear resonance spectrum (¹³C-NMR) of the product A.

### BEST MODE FOR CARRYING OUT THE INVENTION

The α-isomaltosylglucosaccharide-forming enzyme usable in the present invention means an enzyme as defined in claim 1, which forms from amylaceous substances α-isomaltosylglucosaccharides such as α-isomaltosylglucose (alias panose), α-isomaltosylmaltose, α-isomaltosylmaltotriose, and α-isomaltosylmaltotetraose, and in particular is an α-isomaltosylglucosaccharide-forming enzyme from *Bacillus globisporus* C9, FERM BP-7143 (hereinafter may be designated as "Strain C9"), and *Bacillus globisporus* C11, FERM BP-7144 (hereinafter may be designated as "Strain C11"), which have been deposited on April 25, 2000, in International Patent Organism Depositary National Institute of Advanced Industrial Science and Technology Tsukuba Central 6, 1-1, Higashi 1-Chome Tsukuba-shi, Ibaraki-ken, 305-8566, Japan, and which are disclosed in Japanese Patent Application No. 234,937/00; and recombinant polypeptides having an α-isomaltosylglucosaccharide-forming enzyme activity, disclosed in Japanese Patent Application No. 5,441/01.

The α-isomaltosyl-transferring enzyme usable in the present invention means an enzyme as defined in claim 1 which forms cyclotetrasaccharide from α-isomaltosylglucosaccharides such as panose and α-isomaltosylmaltose. These enzymes are an α-isomaltosyl-transferring enzyme from *Bacillus globisporus* C9, FERM BP-7143, and *Bacillus globisporus* C11, FERM BP-7144, disclosed in Japanese Patent Application No. 229,557/00; and recombinant polypeptides having an α-isomaltosyl-transferring enzyme activity, disclosed in Japanese Patent Application No. 350,142/00.

The isomaltose-releasing enzyme usable in the present invention means an enzyme, which has an activity of releasing isomaltose from α-isomaltosylglucosaccharides or cyclotetrasaccharide, for example, isomaltodextranase (EC 3.2.1.94) from microorganisms such as *Arthrobacter globiformis* T6, NRRL B-4425, reported in Journal of Biochemistry, Vol. 75, pp. 105-112 (1974); *Arthrobacter globiformis,* IAM 12103, provided from Institute of Applied Microbiology (IAM), The University of Tokyo, Tokyo, Japan; and *Actinomadura* R10, NRRL B-11411, reported in Carbohydrate Research, Vol. 89, pp. 289-299 (1981).

The saccharides, which have both a glucose polymerization degree of at least two and α-1,4 glucosidic linkage as a linkage at the non-reducing end, usable in the present invention include, for example, terrestrial starches such as corns, rices, and wheats; and subterranean starches such as potatoes, sweet potatoes, and tapioca, as well as partial hydrolyzates thereof, i.e., partial starch hydrolyzates thereof. The partial starch hydrolyzates can be usually prepared by suspending the above terrestrial or subterranean starches in water, usually, into 10%, preferably, 15-65%, more preferably, 20-50% starch suspensions, and then liquefying the suspensions by heating or using acid agents or enzyme preparations. The degree of liquefaction is preferably set to a relatively low level, usually, less than DE (dextrose equivalent) 15, preferably, less than DE 10, and more preferably, DE 0.1-9. In the case of liquefaction with acid agents, there employed is a method comprising a step of liquefying the above starches with acid agents such as hydrochloric acid, phosphoric acid, and oxalic acid, and usually neutralizing the liquefied suspensions to the desired pHs with alkaline agents such as calcium carbonate, calcium oxide, and sodium carbonate. While in the case of liquefaction with enzyme preparations, α-amylases, particularly, thermostable liquefying α-amylases are preferably used in the present invention. Isomaltose can be obtained in a relatively high yield by allowing α-isomaltosylglucosaccharide-forming enzyme, in the presence or the absence of α-isomaltosyl-transferring enzyme, to act on saccharides, which have both a glucose polymerization degree of at least two and α-1,4 glucosidic linkage as a linkage at the non-reducing end, to form α-isomaltosylglucosaccharides, which have a glucose polymerization degree of at least three, α-1,6 glucosidic linkage as a linkage at the non-reducing end, and α-1,4 glucosidic linkage as a linkage other than the non-reducing end, and/or to form cyclo{→6)-α-D-glucopyranosyl-(1→3)-α-D-glucopyranosyl-(1→6)-α-D-glucopyranosyl-(1→3)-α-D-glucopyranosyl-(1→}; allowing isomaltosyl-releasing enzymes to act on the products to form isomaltose; and collecting the formed isomaltose. When allowed to act on substrates, α-isomaltosylglucosaccharide-forming enzyme can be used in combination with one or more another enzymes of α-isomaltosyl-transferring enzyme, cyclomaltodextrin glucanotransferase (hereinafter abbreviated as "CGTase"), α-glucosidase, glucoamylase, and starch debranching enzymes such as isoamylase and pullulanase to more increase the yield of isomaltose. Particularly, the yield of isomaltose from cyclotetrasaccharide can be increased up to a maximum yield of 100% in such a manner of allowing isomaltose-releasing enzyme to act on cyclotetrasaccharide obtained by allowing α-isomaltosylglucosaccharide-forming enzyme, in the presence of α-isomaltosyl-transferring enzyme, to act on saccharides having both a glucose polymerization degree of at least two and α-1,4 glucosidic linkage as a linkage at the non-reducing end. The order of a plurality of enzymes employed in the present invention can be decided depending on the yield of isomaltose, reaction times, reaction conditions, etc. These enzymes can be allowed to act on substrates at the same time or different timings after divided into several aliquots with the desired amount. Any pHs at which the enzymes used in the present invention are allowed to act on their substrates can be employed as long as the enzymes exert their enzymatic activities at the pHs, usually, those which are selected from pH 4-10, preferably, pH 5-8. The temperatures of allowing enzymes are usually selected from 10-80°C, preferably, 30-70°C. The amount of enzymes used can be appropriately altered in view of the reaction condition and time for each enzyme: Usually, the amounts of α-isomaltosyl-transferring enzyme and α-isomaltosylglucosaccharide-forming enzyme used are respectively selected from 0.01-100 units, the amounts of isomaltose-releasing enzyme and starch debranching enzyme used are selected from 1-10,000 units, and the amounts of CGTase, α-glucosidase, glucoamylase, and isoamylase used are selected from 0.05-7,000 units. Although the reaction time of enzymes used is varied depending on their amounts used, it is appropriately selected in view of the yield of isomaltose. Usually, the reaction time is set to 1-200 hours, preferably, 5-150 hours, and more preferably, 10-100 hours to complete the overall enzymatic reactions. The pHs and temperatures in the enzymatic reaction for each enzyme can be appropriately altered before termination of the enzymatic reactions of the present invention.

The content of isomaltose in the enzymatic reaction mixtures thus obtained is usually, on a dry solid basis, at least 30%, preferably, at least 40%, more preferably, at least 50%, and still more preferably, up to a maximum level of 99% or higher. Particularly, when α-isomaltosylglucosaccharide-forming enzyme, α-isomaltosyl-transferring enzyme, and isomaltose-releasing enzyme are simultaneously or in this order added to and allowed to act on saccharides having both a glucose polymerization degree of at least two and α-1,4 glucosidic linkage as a linkage at the non-reducing end, enzymatic reaction mixtures with an isomaltose content of at least 50%, d.s.b., can be easily obtained. In general, the reaction mixtures can be subjected to conventional methods such as filtration and centrifugation to remove impurities; decolored with an activated charcoal, desalted and purified, for example, by ion-exchange resins in a H- or OH-form; and concentrated into syrupy products; and optionally dried into powdery products. If necessary, the resulting products can be further purified into high isomaltose content products by appropriately using alone or in combination with two or more methods of column chromatographies such as ion-exchange column chromatography, column chromatography using an activated charcoal, and silica gel column chromatography; separation using organic solvents such as alcohols and acetone; and membrane separation. In particular, as an industrial-scale production method for high isomaltose content product, ion-exchange column chromatography is preferably employed. For example, high isomaltose content products can be produced on an industrial scale at a relatively high yield and amount and at a lesser cost by ion-exchange column chromatography using one or more styrene-divinylbenzene cross-linked copolymeric resins with sulfonyl group and strong-acid cation exchange resins in the form of alkaline metals such as Na⁺ and K⁺, and of alkaline earth metals such as Ca²⁺ and Mg²⁺, as disclosed in Japanese Patent Kokai Nos. 23,799/83 and 72,598/83. Examples of commercialized products of the above strong-acid cation exchange resins include "DOWEX 50WX2", "DOWEX 50WX4", and "DOWEX 50WX8", produced by Dow Chemical Co., Midland, Michigan, USA; "AMBERLITE CG-120", produced by Rohm & Hass Company, Philadelphia, PA., USA; "XT-1022E" produced by Tokyo Organic Chemical Industries, Ltd., Tokyo, Japan; and "DIAION SK1B", "DIAION SK102", and "DIAION SK104", produced by Mitsubishi Chemical Industries, Tokyo, Japan. In practicing the above ion-exchange column chromatography, any one of fixed-bed, moving bed, and semi-moving bed methods can be appropriately used. With these methods the purity of isomaltose can be increased, usually, to at least 60%, preferably, at least 80%, and more preferably, at least 99%, d.s.b., as the highest possible purity. Products of isomaltose except for the highest possible isomaltose, i.e., high isomaltose content products usually contain isomaltose and 1-60%, d.s.b., of one or more saccharides from glucose, maltose, maltotriose, maltotetraose, other starch hydrolyzates, α-isomaltosylglucosaccharides, and α-glucosyl-(1→6)-α-glucosyl-(1→3)-α-glucosyl-(1→6)-α-glucose (may be abbreviated as "open-ring tetrasaccharide", hereinafter).

The isomaltose and high isomaltose content products thus obtained can be suitably used as sweeteners which substantially do not induce dental caries because of their action of inhibiting the formation of dextran as a cause of dental caries, as well as satisfactory quality and good tastable sweetness. The isomaltose and high isomaltose content products of the present invention have also satisfactory storage stability. Particularly, products with a relatively high content of crystalline isomaltose can be advantageously used as sugar coatings for tablets in combination with conventional binders such as pullulan, hydroxyethyl starch, and polyvinylpyrrolidone. The isomaltose and high isomaltose content products of the present invention have useful properties of osmosis-controlling ability, filler-imparting ability, gloss-imparting ability, humectancy, viscosity, crystallization-preventing ability for saccharides, insubstantial fermentability, retrogradation-preventing ability for gelatinized starches, etc. Thus, the isomaltose and high isomaltose content products can be arbitrary used as a sweetener, taste-improving agent, flavor-improving agent, quality-improving agent, stabilizer, excipient, filler, etc., in a variety of compositions such as food products, feeds, pet foods, cosmetics, pharmaceuticals, tobaccos, and cigarettes.

The isomaltose and high isomaltose content products of the present invention can be used as seasonings to sweeten food products, and if necessary, they can be arbitrarily used in combination with one or more other sweeteners such as powdered syrup, glucose, fructose, lactosucrose, maltose, sucrose, isomerized sugar, honey, maple sugar, isomaltooligosaccharides, galactooligosaccharides, fructooligosaccharides, sorbitol, maltitol, lactitol, dihydrochalcone, stevioside, α-glycosyl stevioside, sweetener of *Momordica grosvenori,* glycyrrhizin, L-aspartyl L-phenylalanine methyl ester, sucralose, acesulfame K, saccharin, glycine, and alanine; and fillers such as dextrins, starches, and lactose.

Particularly, the isomaltose and high isomaltose content products of the present invention can be arbitrarily used intact or after mixing with appropriate fillers, excipients, binders, sweeteners, etc., and then formed into products with different shapes such as granules, spheres, plates, cubes, tablets, films, and sheets.

Since the isomaltose and high isomaltose content products of the present invention well harmonize with other tastable materials having sour-, acid-, salty-, astringent-, delicious-, or bitter-tastes, and have a satisfactorily high acid- and heat-tolerance, they can be favorably used in food products to sweeten and/or improve the taste or the quality of food products; amino acids, peptides, soy sauces, powdered soy sauces, *miso*, "*funmatsu-miso*" (a powdered *miso*), "*moromi*" (a refined sake), *"hishio"* (a refined soy sauce), "*furikake*" (a seasoned fish meal), mayonnaises, dressings, vinegars, *"sanbaizu*" (a sauce of sugar, soy sauce and vinegar), "*funmatsu-sushisu*" (powdered vinegar for sushi), "*chuka-no-moto*" (an instant mix for Chinese dish), "*tentsuyu*" (a sauce for Japanese deep-fat fried food), *"mentsuyu"* (a sauce for Japanese vermicelli), sauce, catsups, "*yakiniku-no-tare*" (a sauce for Japanese grilled meat), curry roux, instant stew mixes, instant soup mixes, "*dashi-no-moto*" (an instant stock mix), nucleotide seasonings, mixed seasonings, "*mirin*" (a sweet sake), "*shin-mirin*" (a synthetic mirin), table sugars, and coffee sugars. Also, the isomaltose and high isomaltose content products of the present invention can be arbitrarily used in "*wagashi*" (Japanese cakes) such as "*senbei*" (a rice cracker), *"arare"* (a rice cake cube), *"okoshi"* (a millet-and-rice cake), "*mochi*" (a rice paste) and the like, "*manju*" (a bun with a bean-jam), *"uiro"* (a sweet rice jelly), *"an"* (a bean jam) and the like, "*yokan*" (a sweet jelly of beans), "*mizu-yokan*" (a soft adzuki-bean jelly), "*kingyoku*" (a kind of yokan), jellies, *pao de Castella,* and "*amedama*" (a Japanese toffee); Western confectioneries such as a bun, biscuit, cracker, cookie, pie, pudding, butter cream, custard cream, cream puff, waffle, sponge cake, doughnut, chocolate, chewing gum, caramel, nougat, and candy; frozen desserts such as an ice cream and sherbet; syrups such as "*kajitsu-no-syrup-zuke"* (a preserved fruit) and "*korimitsu*" (a sugar syrup for shaved ice); pastes such as a flour paste, peanut paste, fruit paste, and spread; processed fruits and vegetables such as a jam, marmalade, *"syrup-zuke"* (fruit pickles), and "*toka*" (conserves); pickles and pickled products such as a "*fukujinzuke*" (red colored radish pickles), "*bettara-zuke*" (a kind of whole fresh radish pickles), *"senmai-zuke"* (a kind of sliced fresh radish pickles), and "*rakkyo-zuke*" (pickled shallots); premixes for pickles and pickled products such as a *"takuan-zuke-no-moto"* (a premix for pickled radish), and *"hakusai-zuke-no-moto*" (a premix for fresh white rape pickles); meat products such as a ham and sausage; products of fish meat such as a fish ham, fish sausage, "*kamaboko*" (a steamed fish paste), "*chikuwa*" (a kind of fish paste), and "*tenpura*" (a Japanese deep-fat fried fish paste); "*chinmi*" (relish) such as a "*uni-no-shiokara*" (salted guts of sea urchin), "*ika-no-shiokara*" (salted guts of squid), "*su-konbu*" (processed tangle), "*saki-surume*" (dried squid strips), "*fugu-no-mirin-boshi*" (a dried mirin-seasoned swellfish); *"tsukudani"* (foods boiled down in soy sauce) such as those of lavers, edible wild plants, dried squids, small fishes, and shellfishes; daily dishes such as a "*nimame*" (cooked beans), potato salad, and "*konbu-maki*" (a tangle roll); milk products such as yogurts and cheeses; canned and bottled products such as those of meat, fish meat, fruit, and vegetables; alcoholic beverages such as a *sake*, synthetic *sake*, liqueur, and foreign liquors and drinks; soft drinks such as a coffee, tea, cocoa, juice, carbonated beverage, sour milk beverage, and beverage containing lactic acid bacteria; instant food products such as an instant pudding mix, instant hot cake mix, "*sokuseki-shiruko*" (an instant mix of adzuki-bean soup with rice cake), and instant soup mix; and other foods and beverages such as a solid food for babies, food for therapy, health/tonic drink, peptide food, frozen food, and health food.

The isomaltose and high isomaltose content products of the present invention can be arbitrarily used to improve the taste preference of feeds and pet foods for animals and pets such as domestic animals, poultry, honey bees, silk worms, and fishes; and also they can be arbitrary used as a sweetener, taste-improving agent, flavoring substance, quality-improving agent, and stabilizer in products in a liquid or solid form such as a tobacco, cigarette, tooth paste, lipstick/rouge, lip cream, internal liquid medicine, tablet, troche, cod liver oil in the form of drop, cachou, oral refrigerant, and gargle.

Stable and high-quality health foods and pharmaceuticals in a liquid, paste or solid form can be obtained by incorporating, as a quality-improving agent and/or stabilizer, the isomaltose and high isomaltose content products of the present invention into health foods and pharmaceuticals which contain effective ingredients, active ingredients, or biologically active substances. Examples of such biologically active substances include lymphokines such as α-, β- and γ- interferons, tumor necrosis factor-α (TNF-α), tumor necrosis factor-β (TNF-β), macrophage migration inhibitory factor, colony-stimulating factor, transfer factor, and interleukins; hormones such as insulin, growth hormone, prolactin, erythropoietin, and follicle-stimulating hormone; biological preparations such as BCG vaccine, Japanese encephalitis vaccine, measles vaccine, live polio vaccine, smallpox vaccine, tetanus toxoid, Trimeresurus antitoxin, and human immunoglobulin; antibiotics such as penicillin, erythromycin, chloramphenicol, tetracycline, streptomycin, and kanamycin sulfate; vitamins such as thiamine, riboflavin, L-ascorbic acid, α-glycosyl ascorbic acid, cod liver oil, carotenoid, ergosterol, tocopherol, rutin, α-glycosyl rutin, naringin, α-glycosyl naringin, hesperidin, and α-glycosyl hesperidin; enzymes such as lipase, elastase, urokinase, protease, β-amylase, isoamylase, glucanase, and lactase; extracts such as a ginseng extract, bamboo leaf extract, Japanese apricot extract, pine leaf extract, snapping turtle extract, chlorella extract, aloe extract, and propolis extract; live microorganisms such as viruses, lactic acid bacteria, and yeasts; and royal jelly.

The methods for incorporating the isomaltose or the high isomaltose content products of the present invention into the aforesaid compositions are those which can complete the incorporation before completion of the processings of the compositions, and can be appropriately selected from the following conventional methods of mixing, kneading, dissolving, melting, soaking, penetrating, dispersing, applying, coating, spraying, injecting, crystallizing, and solidifying. The isomaltose or the high isomaltose content product can be preferably incorporated into the compositions in an amount, usually, of at least 0.1%, preferably, at least 1%, and more preferably, 2-99.99% (w/w).

The following experiments explain the present invention in more detail:

### Experiment 1

### Preparation of α-isomaltosylglucosaccharide-forming enzyme and α -isomaltosyl-transferring enzyme

A liquid culture medium consisting of 4.0% (w/v) of "PINE-DEX #4", a partial starch hydrolysate commercialized by Matsutani Chemical Ind., Tokyo, Japan, 1.8% (w/v) of "ASAHIMEAST", a yeast extract commercialized by Asahi Breweries, Ltd., Tokyo, Japan, 0.1% (w/v) of dipotassium phosphate, 0.06% (w/v) of sodium phosphate dodecahydrate, 0.05% (w/v) magnesium sulfate heptahydrate, and water was placed in 500-ml Erlenmeyer flasks in a respective volume of 100 ml, sterilized by autoclaving at 121°C for 20 min, cooled, and then seeded with *Bacillus globisporus* C9 strain, FERM BP-7143, followed by culturing under rotary-shaking conditions at 27°C and 230 rpm for 48 hours for a seed culture.

About 20 L of a fresh preparation of the same liquid culture medium as used in the above seed culture were placed in a 30-L fermentor, sterilized by heating, and then cooled to 27°C and inoculated with 1% (v/v) of the seed culture, followed by culturing at 27°C and pH 6.0-8.0 for 48 hours under aeration-agitation conditions. After completion of the culture, the resulting culture, which had about 0.45 unit/ml of the α-isomaltosylglucosaccharide-forming enzyme, about 1.5 units/ml of α-isomaltosyl-transferring enzyme, and about 0.95 unit/ml of cyclotetrasaccharide-forming activity, was centrifuged at 10,000 rpm for 30 min to obtain about 18 L of a supernatant. When measured for enzymatic activity, the supernatant had about 0.45 unit/ml of the α-isomaltosylglucosaccharide-forming enzyme, i.e., a total enzymatic activity of about 8,110 units; about 1.5 units/ml of α-isomaltosyl-transferring enzyme, i.e., a total enzymatic activity of about 26,900 units. The supernatant thus obtained can be used as an enzyme preparation of α-isomaltosylglucosaccharide-forming enzyme and α-isomaltosyl-transferring enzyme.

The activities of these enzymes were assayed as follows: The α-isomaltosylglucosaccharide-forming enzyme of the present invention was assayed for enzymatic activity by dissolving maltotriose in 100 mM acetate buffer (pH 6.0) to give a concentration of 2% (w/v) for a substrate solution, adding a 0.5 ml of an enzyme solution to a 0.5 ml of the substrate solution, enzymatically reacting the mixture solution at 35°C for 60 min, suspending the reaction mixture by heating at 100°C for 10 min, and quantifying maltose, among the isomaltosyl maltose and maltose formed in the reaction mixture, by high-performance liquid chromatography (abbreviated as "HPLC" hereinafter). HPLC was carried out using "YMC PACK ODS-AQ303 column" commercialized by YMC Co., Ltd., Tokyo, Japan, at a column temperature of °C and a flow rate of 0.5 ml/min of water; and using "RI-8012", a differential refractometer commercialized by Tosoh Corporation, Tokyo, Japan. One unit activity of the α-isomaltosylglucosaccharide-forming enzyme is defined as the enzyme amount that forms one micromole of maltose per minute under the above enzymatic reaction conditions.

The α-isomaltosyl-transferring enzyme was assayed for enzymatic activity by dissolving panose in 100 mM acetate buffer (pH 6.0) to give a concentration of 2% (w/v) for a substrate solution, adding a 0.5 ml of an enzyme solution to 0.5 ml of the substrate solution, enzymatically reacting the mixture solution at 35°C for 30 min, suspending the reaction mixture by boiling for 10 min, and quantifying glucose, among the cyclotetrasaccharide and glucose formed in the reaction mixture, by the glucose oxidase method. One unit activity of the α-isomaltosyl-transferring enzyme is defined as the enzyme amount that forms one micromole of glucose per minute under the above enzymatic reaction conditions.

The cyclotetrasaccharide-forming activity is assayed by dissolving "PINE-DEX #100", a partial starch hydrolysate commercialized by Matsutani Chemical Ind., Tokyo, Japan, in 50 mM acetate buffer (pH 6.0) to give a concentration of 2% (w/v) for a substrate solution, adding 0.5 ml of an enzyme solution to 0.5 ml of the substrate solution, enzymatically reacting the mixture solution at 35°C for 60 min, suspending the reaction mixture by boiling for 10 min, and then further adding to the resulting mixture one milliliter of 50 mM acetate buffer (pH 5.0) with 70 units/ml of "TRANSGLUCOSIDASE L AMANO™", an α-glucosidase commercialized by Amano Pharmaceutical Co., Ltd., Aichi, Japan, and 27 units/ml of glucoamylase, commercialized by Nagase Biochemicals, Ltd., Kyoto, Japan, and incubated at 50°C for 60 min, inactivating the remaining enzymes by heating at 100°C for 10 min, and quantifying cyclotetrasaccharide on HPLC similarly as above. One unit of cyclotetrasaccharide-forming activity is defined as the enzyme amount that forms one micromole of cyclotetrasaccharide per minute under the above enzymatic reaction conditions.

### Experiment 2

### Isolation of α-isomaltosylglucosaccharide-forming enzyme and α-isomaltosyl-transferring enzyme

### Experiment 2-1

### Isolation of α-isomaltosylglucosaccharide-forming enzyme

About 18 L of the supernatant in Experiment 1 was salted out with 80% saturated ammonium sulfate and allowed to stand at 4°C for 24 hours, and the formed precipitates were collected by centrifugation at 10,000 rpm for 30 min, dissolved in 10 mM phosphate buffer (pH 7.5), and dialyzed against a fresh preparation of the same buffer to obtain about 400 ml of a crude enzyme solution with 8,110 units of α-isomaltosylglucosaccharide-forming enzyme, 24,700 units of α-isomaltosyl-transferring enzyme, and about 15,600 units of cyclotetrasaccharide-forming activity. The crude enzyme solution was subjected to ion-exchange chromatography using 1,000 ml of "SEPABEADS FP-DA13" gel, an ion-exchange resin commercialized by Mitsubishi Chemical Industries, Ltd., Tokyo, Japan. The α-isomaltosylglucosaccharide-forming enzyme and cyclotetrasaccharide were eluted as non-adsorbed fractions without adsorbing on the ion-exchange resin. The resulting enzyme solution was dialyzed against 10 mM phosphate buffer (pH 7.0) with 1 M ammonium sulfate, and the dialyzed solution was centrifuged to remove impurities, and subjected to affinity chromatography using 500 ml of "SEPHACRYL HR S-200", a gel commercialized by Amersham Corp., Div. Amersham International, Arlington Heights, IL, USA. Enzymatically active components adsorbed on the gel and, when sequentially eluted with a linear gradient decreasing from 1 M to 0 M of ammonium sulfate and a linear gradient increasing from 0 mM to 100 mM of maltotetraose, the α-isomaltosylglucosaccharide-forming enzyme and the α-isomaltosyl-transferring enzyme were separately eluted, i.e., the former was eluted with the linear gradient of maltotetraose at about 30 mM and the latter was eluted with the linear gradient of ammonium sulfate at about 0 M. Thus, fractions with α-isomaltosyl-transferring activity and those with the α-isomaltosylglucosaccharide-forming activity were separatory collected.

The above α-isomaltosylglucosaccharide-forming enzyme fraction were pooled and dialyzed against 10 mM phosphate buffer (pH 7.0) containing 1 M ammonium sulfate. The dialyzed solution was centrifuged to remove insoluble impurities, and the resulting supernatant was fed to hydrophobic chromatography using 350 ml of "BUTYL-TOYOPEARL 650 M", a gel commercialized by Tosoh Corporation, Tokyo, Japan. The active enzyme was adsorbed on the gel and then eluted therefrom at about 0.3 M ammonium sulfate using a linear gradient decreasing from 1 M to 0 M of ammonium sulfate, followed by collecting fractions with the enzyme activity. The fractions were pooled and then dialyzed against 10 mM phosphate buffer (pH 7.0) containing 1 M ammonium sulfate. The resulting dialyzed solution was centrifuged to remove impurities and fed to affinity chromatography using "SEPHACRYL HR S-200" gel to purify the enzyme. The amount of enzyme activity, specific activity, and yield of the α-isomaltosylglucosaccharide-forming enzyme in each purification step are in Table 1.

**Table 1**

| Purification step | Enzyme* activity (unit) | Specific activity of enzyme* (unit/mg protein) | Yield (%) |
|---|---|---|---|
| Culture supernatant | 8,110 | 0.12 | 100 |
| Dialyzed solution after salting out with ammonium sulfate | 7,450 | 0.56 | 91.9 |
| Eluate from ion-exchange column chromatography | 5,850 | 1.03 | 72.1 |
| Eluate from affinity column chromatography | 4,040 | 8.72 | 49.8 |
| Eluate from hydrophobic column chromatography | 3,070 | 10.6 | 37.8 |
| Eluate from affinity column chromatography | 1,870 | 13.6 | 23.1 |

| | | | |
|---|---|---|---|
| Note : The symbol "*" means the α-isomaltosylglucosaccharide-forming enzyme. | | | |

The finally purified α-isomaltosylglucosaccharide-forming enzyme specimen was assayed for purity on gel electrophoresis using a 7.5% (w/v) polyacrylamide gel and detected on the gel as a single protein band, i.e., a high purity enzyme specimen.

### Experiment 2-2

### Property of α-isomaltosylglucosaccharide-forming enzyme

A purified specimen of α-isomaltosylglucosaccharide-forming enzyme, obtained by the method in Experiment 2-1, was subjected to SDS-PAGE using a 7.5% (w/v) of polyacrylamide gel and then determined for molecular weight by comparing with the dynamics of standard molecular markers electrophoresed in parallel, commercialized by Bio-Rad Laboratories Inc., Brussels, Belgium, revealing that the enzyme had a molecular weight of about 140,000±20,000 daltons.

A fresh preparation of the above purified specimen was subjected to isoelectrophoresis using a gel containing 2% (w/v) ampholine commercialized by Amersham Corp., Div. Amersham International, Arlington Heights, IL, USA, and then measured for pHs of protein bands and gels to determine the isoelectric point of the enzyme, revealing that the enzyme had an isoelectric point of about 5.2±0.5.

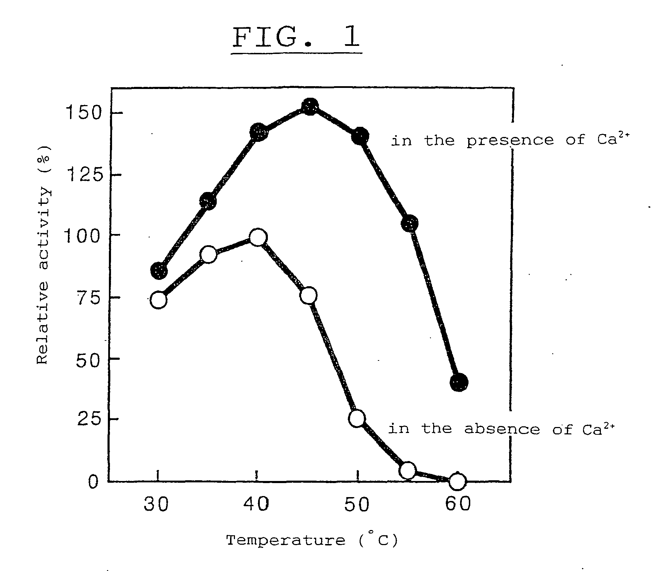

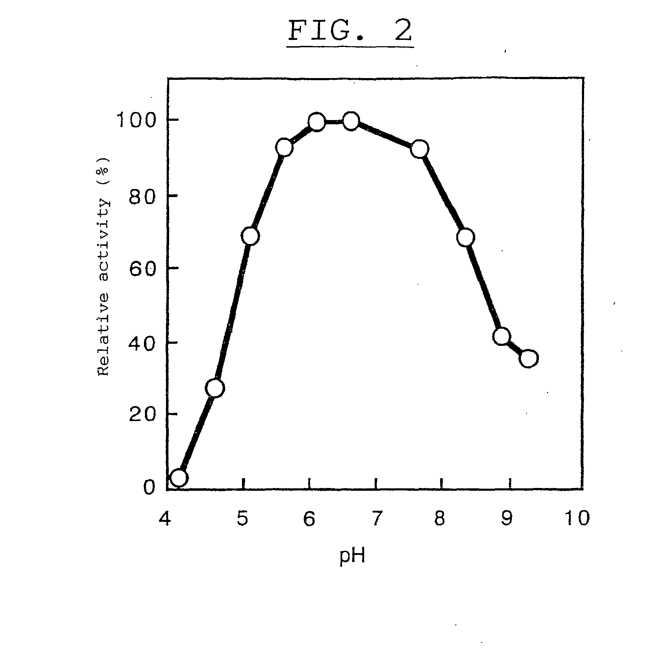

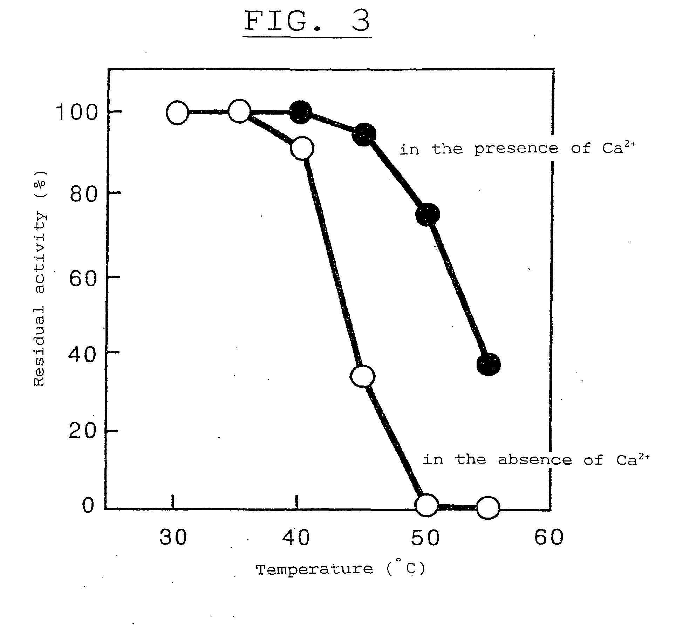

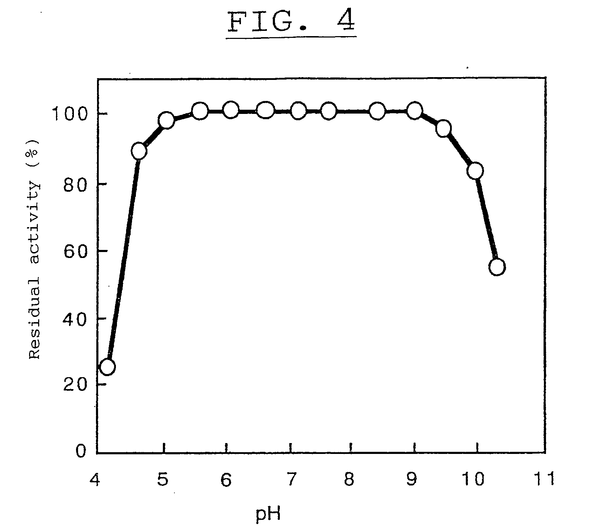

The influence of temperature and pH on the activity of α-isomaltosylglucosaccharide-forming enzyme was examined in accordance with the assay for the enzyme activity, where the influence of temperature was conducted in the presence or the absence of 1 mM Ca²⁺. These results are in FIG. 1 (influence of temperature) and FIG. 2 (influence of pH). The optimum temperature of the enzyme was about 40°C (in the absence of Ca²⁺) and about 45°C (in the presence of 1 mM Ca²⁺) when incubated at pH 6.0 for 60 min, and the optimum pH of the enzyme was about 6.0 to about 6.5 when incubated at 35°C for 60 min. The thermal stability of the enzyme was determined by incubating the testing enzyme solutions in 20 mM acetate buffer (pH 6.0) at prescribed temperatures for 60 min in the presence or the absence of 1 mM Ca²⁺, cooling the resulting enzyme solutions with water, and assaying the remaining enzyme activity of each solution. The pH stability of the enzymes was determined by keeping the testing enzyme solutions in 50 mM buffers having prescribed pHs at 4°C for 24 hours, adjusting the pH of each solution to 6.0, and assaying the remaining enzyme activity of each solution. These results are respectively in FIG. 3 (thermal stability) and FIG. 4 (pH stability). As a result, the enzyme had thermal stability of up to about 35°C in the absence of Ca²⁺ and about 40°C in the presence of 1 mM Ca²⁺, and pH stability of about 4.5 to about 9.0.

The influence of metal ions on the activity of α-isomaltosylglucosaccharide-forming enzyme was examined in the presence of 1 mM of each metal-ion according to the assay for the enzyme activity. The results are in Table 2.

**Table 2**

| Metal ion | Relative activity (%) | Metal ion | Relative activity (%) |
|---|---|---|---|
| None | 100 | Hg²⁺ | 4 |
| Zn²⁺ | 92 | Ba²⁺ | 65 |
| Mg²⁺ | 100 | Sr²⁺ | 80 |
| Ca²⁺ | 115 | Pb²⁺ | 103 |
| Co²⁺ | 100 | Fe²⁺ | 98 |
| Cu²⁺ | 15 | Fe³⁺ | 97 |
| Ni²⁺ | 98 | Mn²⁺ | 111 |
| Al³⁺ | 99 | EDTA | 20 |

As evident form the results in Table 2, the enzyme activity was greatly inhibited by Hg²⁺, Cu²⁺, and EDTA, and also inhibited by Ba²⁺ and Sr²⁺. It was also found that the enzyme was activated by Ca²⁺ and Mn²⁺.

### Experiment 2-3

### Property of α-isomaltosyl-transferring enzyme

A fraction with α-isomaltosyl-transferring enzyme, obtained in Experiment 2-1, was dialyzed against 10 mM phosphate buffer (pH 7.0) containing 1 M ammonium sulfate. The resulting dialyzed solution was centrifuged to remove impurities and subjected to hydrophobic chromatography using 350 ml of "BUTYL-TOYOPEARL 650 M", a gel commercialized by Tosoh Corporation, Tokyo, Japan. The enzyme adsorbed on the gel and then eluted with a linear gradient decreasing from 1 M to 0 M ammonium sulfate, resulting in an elution of the enzyme from the gel at a concentration of about 0.3 M ammonium sulfate and collecting fractions with the enzyme activity. Thereafter, the fractions were pooled and dialyzed against 10 mM phosphate buffer (pH 7.0) containing 1 M ammonium sulfate, and the dialyzed solution was centrifuged to remove impurities and purified on affinity chromatography using "SEPHACRYL HR S-200" gel. The amount of enzyme activity, specific activity, and yield of the α-isomaltosyl-transferring enzyme in each purification step are in Table 3.

**Table 3**

| Purification step | Enzyme* activity (unit) | Specific activity of enzyme* (unit/mg protein) | Yield (%) |
|---|---|---|---|
| Culture supernatant | 26,900 | 0.41 | 100 |
| Dialyzed solution after salting out with ammonium sulfate | 24,700 | 1.85 | 91.8 |
| Eluate from ion-exchange column chromatography | 19,400 | 3.41 | 72.1 |
| Eluate from affinity column chromatography | 13,400 | 18.6 | 49.8 |
| Eluate from hydrophobic column chromatography | 10,000 | 21.3 | 37.2 |
| Eluate from affinity column chromatography | 6,460 | 26.9 | 24.0 |

| | | | |
|---|---|---|---|
| Note : The symbol "*" means the α-isomaltosyl-transferring enzyme. | | | |

### Experiment 2-4

### Property of α-isomaltosyl-transferring enzyme

The purified specimen of α-isomaltosyl-transferring enzyme in Experiment 2-3 was subjected to SDS-PAGE using a 7.5% (w/v) of polyacrylamide gel and then determined for molecular weight by comparing with the dynamics of standard molecular markers electrophoresed in parallel, commercialized by Bio-Rad Laboratories Inc., Brussels, Belgium, revealing that the enzyme had a molecular weight of about 112,000±20,000 daltons.

A fresh preparation of the above purified specimen was subjected to isoelectrophoresis using a gel containing 2% (w/v) ampholine commercialized by Amersham Corp., Div., Amersham International, Arlington Heights, IL, USA, and then measured for pHs of protein bands and gel to determine the isoelectric point of the enzyme, revealing that the enzyme had an isoelectric point of about 5.5±0.5.

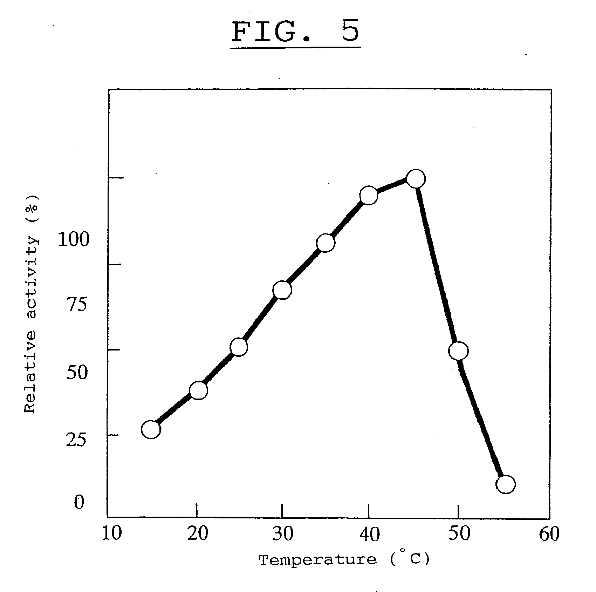

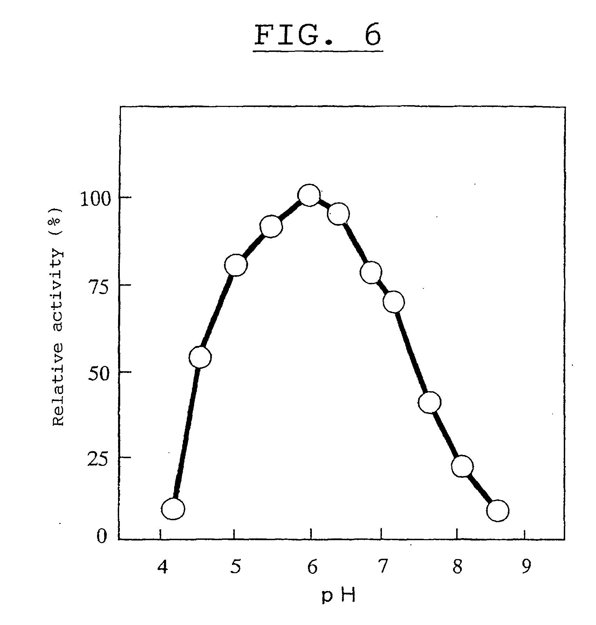

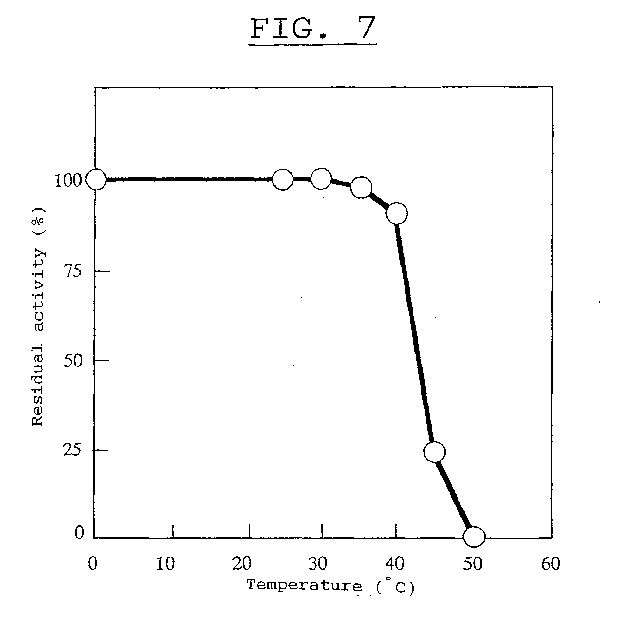

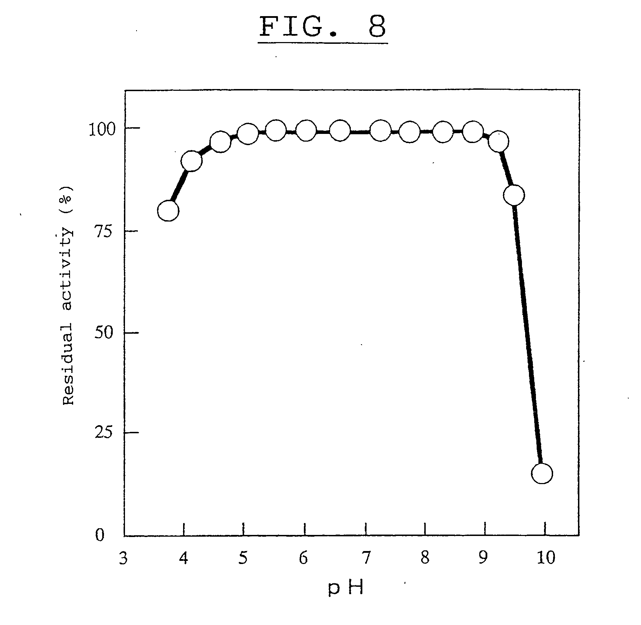

The influence of temperature and pH on the activity of α-isomaltosyl-transferring enzyme was examined in accordance with the assay for the enzyme activity. These results are in FIG. 5 (influence of temperature) and FIG. 6 (influence of pH). The optimum temperature of the enzyme was about 45°C when incubated at pH 6.0 for 30 min, and the optimum pH of the enzyme was about 6.0 when incubated at 35°C for 30 min. The thermal stability of the enzyme was determined by incubating the testing enzyme solutions in 20 mM acetate buffer (pH 6.0) at prescribed temperatures for 60 min, cooling the resulting enzyme solutions with water, and assaying the remaining enzyme activity of each solution. The pH stability of the enzyme was determined by keeping the testing enzyme solutions in 50 mM buffers having prescribed pHs at 4°C for 24 hours, adjusting the pH of each solution to 6.0, and assaying the remaining enzyme activity of each solution. These results are respectively in FIG. 7 (thermal stability) and FIG. 8 (pH stability). As a result, the enzyme had thermal stability of up to about 40°C and pH stability of about 4.0 to about 9.0.

The influence of metal ions on the activity of α-isomaltosyl-transferring enzyme was examined in the presence of 1 mM of each metal-ion according to the assay for the enzyme activity. The results are in Table 4.

**Table 4**

| Metal ion | Relative activity (%) | Metal ion | Relative activity (%) |
|---|---|---|---|
| None | 100 | Hg²⁺ | 1 |
| Zn²⁺ | 88 | Ba²⁺ | 102 |
| Mg²⁺ | 98 | Sr²⁺ | 101 |
| Ca²⁺ | 101 | Pb²⁺ | 89 |
| Co²⁺ | 103 | Fe²⁺ | 96 |
| Cu²⁺ | 57 | Fe³⁺ | 105 |
| Ni²⁺ | 102 | Mn²⁺ | 106 |
| Al³⁺ | 103 | EDTA | 104 |

As evident form the results in Table 4, the enzyme activity was greatly inhibited by Hg²⁺ and also inhibited by Cu²⁺. It was also found that the enzyme was not activated by Ca²⁺ and not inhibited by EDTA.

Both the α-isomaltosylglucosaccharide-forming enzyme and the α-isomaltosyl-transferring enzyme from *Bacillus globisporus* C9 strain, FERM BP-7143, can be suitably used in the present invention.

### Experiment 3

### Production of α-isomaltosylglucosaccharide-forming enzyme and α-isomaltosyl-transferring enzyme

A liquid nutrient culture medium, consisting of 4.0% (w/v) of "PINE-DEX #4", a partial starch hydrolysate, 1.8% (w/v) of "ASAHIMEAST", a yeast extract, 0.1% (w/v) of dipotassium phosphate, 0.06% (w/v) of sodium phosphate dodecahydrate, 0.05% (w/v) magnesium sulfate heptahydrate, and water was placed in 500-ml Erlenmeyer flasks in a respective volume of 100 ml each, autoclaved at 121°C for 20 minutes to effect sterilization, cooled, inoculated with a stock culture of *Bacillus globisporus* C11, FERM BP-7144, and incubated at 27°C for 48 hours under rotary shaking conditions of 230 rpm. The resulting cultures were pooled and used as a seed culture.

About 20 L of a fresh preparation of the same nutrient culture medium as used in the above culture were placed in a 30-L fermentor, sterilized by heating, cooled to 27°C, inoculated with 1% (v/v) of the seed culture, and incubated for about 48 hours while stirring under aeration agitation conditions at 27°C and pH 6.0-8.0. The resultant culture, having about 0.55 unit/ml of α-isomaltosylglucosaccharide-forming enzyme activity, about 1.8 units/ml of α-isomaltosyl-transferring enzyme activity, and about 1.1 units/ml of cyclotetrasaccharide-forming enzyme activity, was centrifuged at 10,000 rpm for 30 min to obtain about 18 L of a supernatant. Measurement of the supernatant revealed that it had about 0.51 unit/ml of α-isomaltosylglucosaccharide-forming enzyme activity, i.e., a total enzyme activity of about 9,180 units; and about 1.7 units/ml of α-isomaltosyl-transferring enzyme activity, i.e., a total enzyme activity of about 30,400 units.

An 18 L of the above supernatant was salted out with an 80% saturated ammonium sulfate solution and allowed to stand at 4°C for 24 hours. Then the salted out precipitates were collected by centrifugation at 10,000 for 30 min, dissolved in 10 mM phosphate buffer (pH 7.5), dialyzed against a fresh preparation of the same buffer to obtain about 416 ml of a crude enzyme solution. The crude enzyme solution was revealed to have 8,440 units of the α-isomaltosylglucosaccharide-forming enzyme, about 28,000 units of α-isomaltosyl-transferring enzyme, and about 17,700 units of cyclotetrasaccharide-forming enzyme. When subjected to ion-exchange chromatography using "SEPABEADS FP-DA13" gel, disclosed in Experiment 2-1, the above three types of enzymes were eluted as non-adsorbed fractions without adsorbing on the gel. The non-adsorbed fractions with those enzymes were pooled and dialyzed against 10 mM phosphate buffer (pH 7.0) containing 1 M ammonium sulfate, and the dialyzed solution was centrifuged to remove impurities. The resulting supernatant was fed to affinity chromatography using 500 ml of "SEPHACRYL HR S-200" gel to purify the enzyme. Active enzymes was adsorbed on the gel and was sequentially eluted with a linear gradient decreasing from 1 M to 0 M of ammonium sulfate and a linear gradient increasing from 0 mM to 100 mM of maltotetraose, followed by separate elutions of α-isomaltosyl-transferring enzyme and α-isomaltosylglucosaccharide-forming enzyme, where the former enzyme was eluted with the linear gradient of ammonium sulfate at a concentration of about 0.3 M and the latter enzyme was eluted with a linear gradient of maltotetraose at a concentration of about 30 mM. Therefore, fractions with the α-isomaltosylglucosaccharide-forming enzyme and those with α-isomaltosyl-transferring enzyme were separately collected and recovered.

### Experiment 4

### Isolation of α-isomaltosylglucosaccharide-forming enzyme and α-isomaltosyl-transferring enzyme

The pooled fraction of α-isomaltosylglucosaccharide-forming enzyme in Experiment 3 was dialyzed against 10 mM phosphate buffer (pH 7.0) containing 1 M ammonium sulfate. The dialyzed solution was centrifuged to remove insoluble impurities, and the resulting supernatant was fed to hydrophobic chromatography using 350 ml of "BUTYL-TOYOPEARL 650 M", a gel commercialized by Tosoh Corporation, Tokyo, Japan. The enzyme adsorbed on the gel was eluted therefrom at about 0.3 M ammonium sulfate using a linear gradient decreasing from 1 M to 0 M of ammonium sulfate, followed by collecting fractions with the enzyme activity. The fractions were pooled and dialyzed against 10 mM phosphate buffer (pH 7.0) containing 1 M ammonium sulfate. The resulting dialyzed solution was centrifuged to remove impurities and fed to affinity chromatography using "SEPHACRYL HR S-200" gel to purify the enzyme. The amount of enzyme activity, specific activity, and yield of the α-isomaltosylglucosaccharide-forming enzyme in each purification step are in Table 5.

**Table 5**

| Purification step | Enzyme* activity (unit) | Specific activity of enzyme* (unit/mg protein) | Yield (%) |
|---|---|---|---|
| Culture supernatant | 9,180 | 0.14 | 100 |
| Dialyzed solution after salting out with ammonium sulfate | 8,440 | 0.60 | 91.9 |
| Eluate from ion-exchange column chromatography | 6,620 | 1.08 | 72.1 |
| Eluate from affinity column chromatography | 4,130 | 8.83 | 45.0 |
| Eluate from hydrophobic column chromatography | 3,310 | 11.0 | 36.1 |
| Eluate from affinity column chromatography | 2,000 | 13.4 | 21.8 |

| | | | |
|---|---|---|---|
| Note : The symbol "*" means the α-isomaltosylglucosaccharide-forming enzyme. | | | |

The finally purified α-isomaltosylglucosaccharide-forming enzyme specimen was assayed for purity on gel electrophoresis using a 7.5% (w/v) polyacrylamide gel and detected on the gel as a single protein band, meaning a high purity enzyme specimen.

### Experiment 4-2

### Property of α-isomaltosylglucosaccharide-forming enzyme

The purified specimen of α-isomaltosylglucosaccharide-forming enzyme in Experiment 4-1 was subjected to SDS-PAGE using a 7.5% (w/v) of polyacrylamide gel and then determined for molecular weight by comparing with the dynamics of standard molecular markers electrophoresed in parallel, commercialized by Bio-Rad Laboratories Inc., Brussels, Belgium, revealing that the enzyme had a molecular weight of about 137,000±20,000 daltons.

A fresh preparation of the above purified specimen was subjected to isoelectrophoresis using a gel containing 2% (w/v) ampholine commercialized by Amersham Corp., Div., Amersham International, Arlington Heights, IL, USA, and then measured for pHs of protein bands and gel to determine the isoelectric point of the enzyme, revealing that the enzyme had an isoelectric point of about 5.2±0.5.

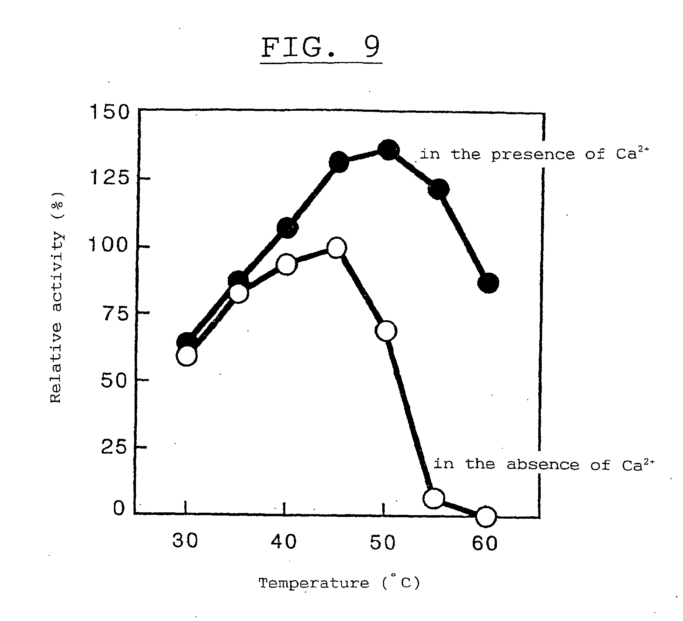

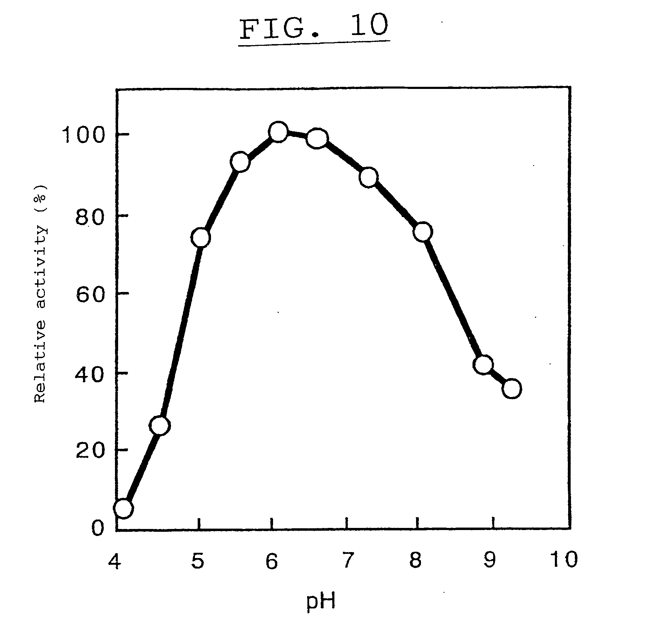

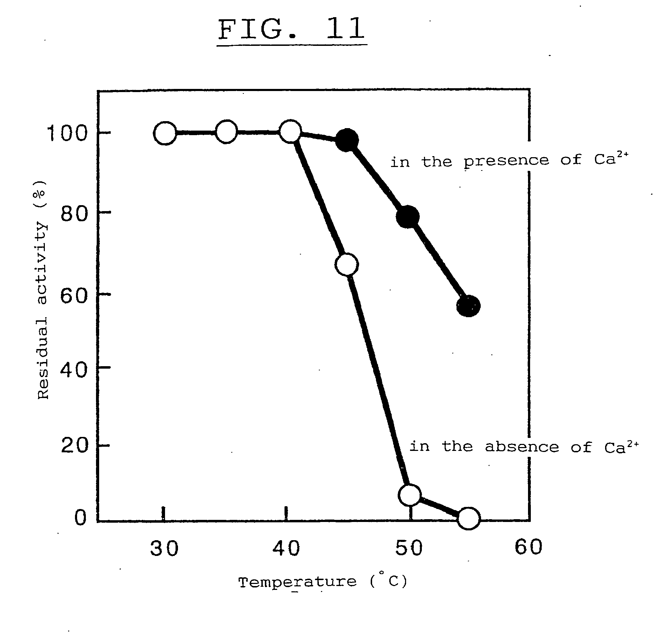

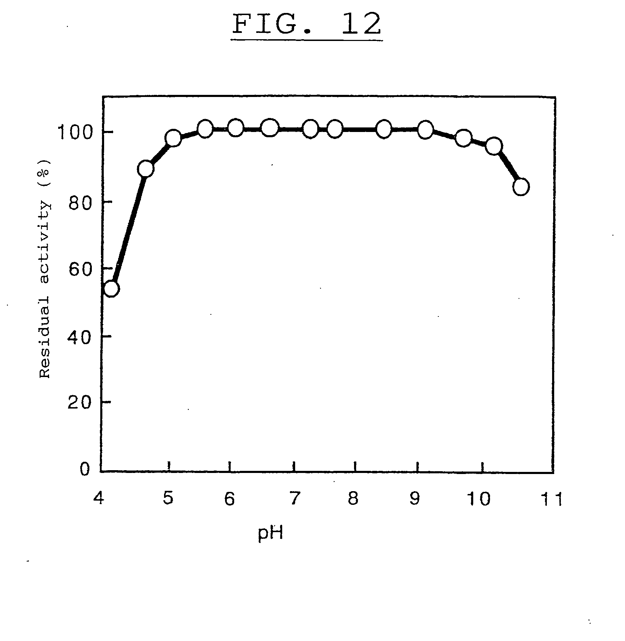

The influence of temperature and pH on the activity of α-isomaltosylglucosaccharide-forming enzyme was examined in accordance with the assay for the enzyme activity, where the influence of temperature was conducted in the presence or the absence of 1 mM Ca²⁺. These results are in FIG. 9 (influence of temperature) and FIG. 10 (influence of pH). The optimum temperature of the enzyme was about 45°C in the absence of Ca²⁺ and about 50°C in the presence of 1 mM Ca²⁺ when incubated at pH 6.0 for 60 min. The optimum pH of the enzyme was about 6.0 when incubated at 35°C for 60 min. The thermal stability of the enzyme was determined by incubating the testing enzyme solutions in 20 mM acetate buffer (pH 6.0) in the presence or the absence of 1 mM Ca²⁺ at prescribed temperatures for 60 min, cooling the resulting enzyme solutions with water, and assaying the remaining enzyme activity of each solution. The pH stability of the enzyme was determined by keeping the testing enzyme solutions in 50 mM buffers having prescribed pHs at 4°C for 24 hours, adjusting the pH of each solution to 6.0, and assaying the remaining enzyme activity of each solution. These results are respectively in FIG. 11 (thermal stability) and FIG. 12 (pH stability). As a result, the enzyme had thermal stability of up to about 40°C in the absence of Ca²⁺ and up to about 45°C in the presence of 1 mM Ca²⁺. The pH stability of enzyme was about 5.0 to about 10.0.

The influence of metal ions on the activity of α-isomaltosyl-transferring enzyme was examined in the presence of 1 mM of each metal-ion according to the assay for the enzyme activity. The results are in Table 6.

**Table 6**

| Metal ion | Relative activity (%) | Metal ion | Relative activity (%) |
|---|---|---|---|
| None | 100 | Hg²⁺ | 4 |
| Zn²⁺ | 91 | Ba²⁺ | 65 |
| Mg²⁺ | 98 | Sr²⁺ | 83 |
| Ca²⁺ | 109 | Pb²⁺ | 101 |
| Co²⁺ | 96 | Fe²⁺ | 100 |
| Cu²⁺ | 23 | Fe³⁺ | 102 |
| Ni²⁺ | 93 | Mn²⁺ | 142 |
| Al³⁺ | 100 | EDTA | 24 |

As evident form the results in Table 6, the enzyme activity was greatly inhibited by Hg²⁺, Cu²⁺, and EDTA and also inhibited by Ba²⁺ and Sr²⁺. It was also found that the enzyme was activated by Ca²⁺ and Mn²⁺.

### Experiment 4-3

### Amino acid sequence of α-isomaltosylglucosaccharide-forming enzyme

The specification does not describe in detail the method for analyzing the amino acid sequence of α-isomaltosylglucosaccharide-forming enzyme because it is disclosed in detail in Japanese Patent Application No. 5,441/01. Similarly as the polypeptide disclosed in the application, the α-isomaltosylglucosaccharide-forming enzyme in Experiment 4-1 has an amino acid sequence of the residues 36-1284 of SEQ ID NO:1 shown in parallel with nucleosides.

### Experiment 4-4

### Isolation of α-isomaltosyl-transferring enzyme

The faction of α-isomaltosyl-transferring enzyme in Experiment 3 was dialyzed against 10 mM phosphate buffer (pH 7.0) containing 1 M ammonium sulfate. The dialyzed solution was centrifuged to remove insoluble impurities, and the resulting supernatant was fed to hydrophobic chromatography using 350 ml of "BUTYL-TOYOPEARL 650 M", a gel commercialized by Tosoh Corporation, Tokyo, Japan. The enzyme adsorbed on the gel and then eluted therefrom at about 0.3 M ammonium sulfate using a linear gradient decreasing from 1 M to 0 M of ammonium sulfate, followed by collecting fractions with the enzyme activity. The fractions were pooled and dialyzed against 10 mM phosphate buffer (pH 7.0) containing 1 M ammonium sulfate. The resulting dialyzed solution was centrifuged to remove impurities and fed to affinity chromatography using "SEPHACRYL HR S-200" gel to purify the enzyme. The amount of enzyme activity, specific activity, and yield of the α-isomaltosyl-transferring enzyme in each purification step are in Table 7.

**Table 7**

| Purification step | Enzyme* activity (unit) | Specific activity of enzyme* (unit/mg protein) | Yield (%) |
|---|---|---|---|
| Culture supernatant | 30,400 | 0.45 | 100 |
| Dialyzed solution after salting out with ammonium sulfate | 28,000 | 1.98 | 92.1 |
| Eluate from ion-exchange column chromatography | 21,800 | 3.56 | 71.7 |
| Eluate from affinity column chromatography | 13,700 | 21.9 | 45.1 |
| Eluate from hydrophobic column chromatography | 10,300 | 23.4 | 33.9 |
| Eluate from affinity column chromatography | 5,510 | 29.6 | 18.1 |

| | | | |
|---|---|---|---|
| Note : The symbol "*" means α-isomaltosyl-transferring enzyme. | | | |

### Experiment 4-5

### Property of α-isomaltosyl-transferring enzyme

The purified specimen of α-isomaltosyl-transferring enzyme in Experiment 4-4 was subjected to SDS-PAGE using a 7.5% (w/v) of polyacrylamide gel and then determined for molecular weight by comparing with the dynamics of standard molecular markers electrophoresed in parallel, commercialized by Bio-Rad Laboratories Inc., Brussels, Belgium, revealing that the enzyme had a molecular weight of about 102,000±20,000 daltons.

A fresh preparation of the above purified specimen was subjected to isoelectrophoresis using a gel containing 2% (w/v) ampholine commercialized by Amersham Corp., Div., Amersham International, Arlington Heights, IL, USA, and then measured for pHs of protein bands and gel to determine the isoelectric point of the enzyme, revealing that the enzyme had an isoelectric point of about 5.6±0.5.

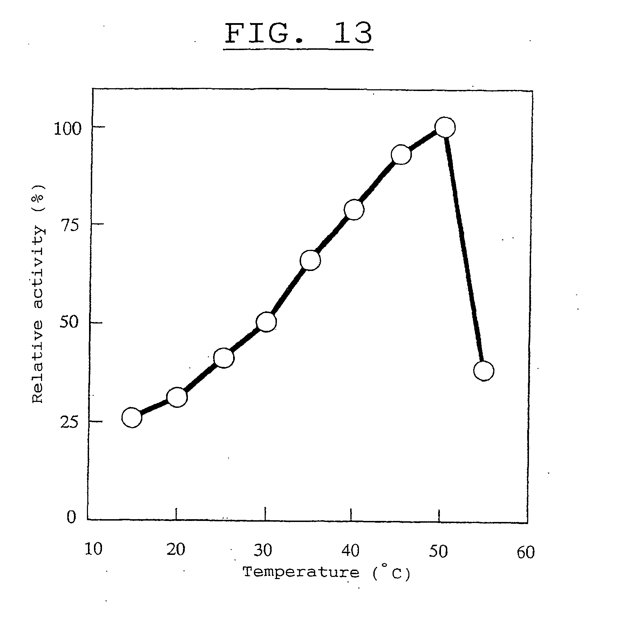

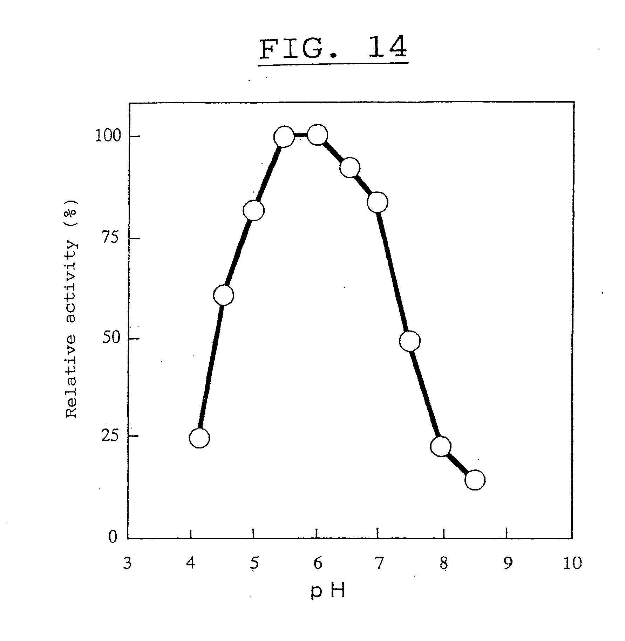

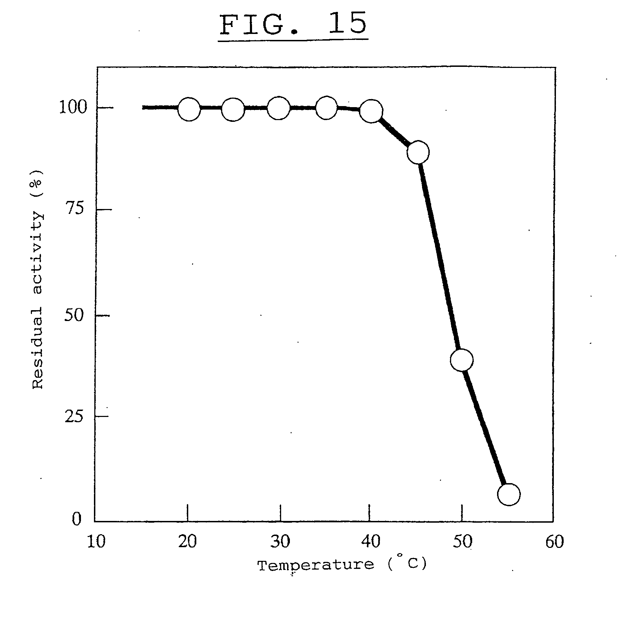

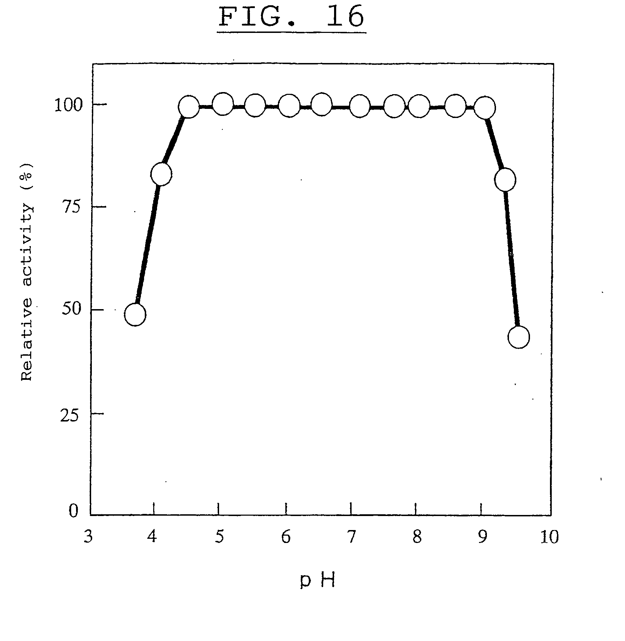

The influence of temperature and pH on the activity of α-isomaltosyl-transferring enzyme was examined in accordance with the assay for the enzyme activity. These results are in FIG. 13 (influence of temperature) and FIG. 14 (influence of pH). The optimum temperature of the enzyme was about 50°C when incubated at pH 6.0 for 30 min. The optimum pH of the enzyme was about 5.5 to about 6.0 when incubated at 35°C for 30 min. The thermal stability of the enzyme was determined by incubating the testing enzyme solutions in 20 mM acetate buffer (pH 6.0) at prescribed temperatures for 60 min, cooling with water the resulting enzyme solutions, and assaying the remaining enzyme activity of each solution. The pH stability of the enzyme was determined by keeping the testing enzyme solutions in 50 mM buffers having prescribed pHs at 4°C for 24 hours, adjusting the pH of each solution to 6.0, and assaying the remaining enzyme activity of each solution. These results are respectively in FIG. 15 (thermal stability) and FIG. 16 (pH stability). As a result, the enzyme had thermal stability of up to about 40°C and pH stability of about 4.5 to about 9.0.

The influence of metal ions on the activity of α-isomaltosyl-transferring enzyme was examined in the presence of 1 mM of each metal-ion according to the assay for the enzyme activity. The results are in Table 8.

**Table 8**

| Metal ion | Relative activity (%) | Metal ion | Relative activity (%) |
|---|---|---|---|
| None | 100 | Hg²⁺ | 2 |
| Zn²⁺ | 83 | Ba²⁺ | 90 |
| Mg²⁺ | 91 | Sr²⁺ | 93 |
| Ca²⁺ | 91 | Pb²⁺ | 74 |
| Co²⁺ | 89 | Fe²⁺ | 104 |
| Cu²⁺ | 56 | Fe³⁺ | 88 |
| Ni²⁺ | 89 | Mn²⁺ | 93 |
| Al³⁺ | 89 | EDTA | 98 |

As evident form the results in Table 8, the enzyme activity was greatly inhibited by Hg²⁺ and also inhibited by Cu²⁺. It was also found that the enzyme was not activated by Ca²⁺ and not inhibited by EDTA.

### Experiment 4-6

### Amino acid sequence of α-isomaltosyl-transferring enzyme

The specification does not describe in detail the method for analyzing the amino acid sequence of α-isomaltosyl-transferring enzyme because it is disclosed in detail in Japanese Patent Application No. 350,142/00. Similarly as the polypeptide disclosed in the application, the α-isomaltosylglucosaccharide-forming enzyme in Experiment 4-4 has an amino acid sequence of the residues 30-1093 of SEQ ID NO:2 shown in parallel with nucleosides.

### Experiment 5

### Action of α-isomaltosylglucosaccharide-forming enzyme on saccharides

The action of α-isomaltosylglucosaccharide-forming enzyme on saccharides as substrates was tested. First, a solution of maltose, maltotriose, maltotetraose, maltopentaose, maltohexaose, maltoheptaose, isomaltose, isomaltotriose, panose, isopanose, α,α-trehalose (may be abbreviated as "trehalose" hereinafter), kojibiose, nigerose, neotrehalose, cellobiose, gentibiose, maltitol, maltotriitol, lactose, sucrose, erlose, selaginose, maltosyl glucoside, or isomaltosyl glucoside was prepared. To each of the above solutions was added two units/g substrate of the purified specimen of α-isomaltosylglucosaccharide-forming enzyme from *Bacillus globisporus* C9 in Experiment 2-1 or *Bacillus globisporus* C11 in Experiment 4-1, and the resulting each solution was adjusted to give a substrate concentration of 2% (w/v) and incubated at 30°C and pH 6.0 for 24 hours. The solutions before and after the enzymatic reactions were respectively subjected to thin-layer chromatography (abbreviated as "TLC" hereinafter). TLC was carried out in such a manner of separating saccharides by developing the solutions twice each using, as a developer, a mixture solution of n-butanol, pyridine, and water (=6:4:1), and, as a thin-layer plate, "KIESELGEL 60", an aluminum plate (20 x 20 cm) for TLC commercialized by Merck & Co., Inc., Rahway, USA.; detecting the total saccharides in each mixture solution by spraying a mixture of sulfuric acid and methanol onto the aluminum plates to develop color of the total saccharides and detecting non-reducing saccharides in each mixture solution by the diphenylamine-aniline method. The results on TLC are in Table 9.

**Table 9**

| Substrate | Enzymatic action | | Substrate | Enzymatic action | |
|---|---|---|---|---|---|
| | Enzyme of Strain C9 | Enzyme of Strain C11 | | Enzyme of Strain C9 | Enzyme of Strain C11 |
| Maltose | + | + | Nigerose | + | + |
| Maltotriose | ++ | ++ | Neotrehalose | + | + |
| Maltotetraose | +++ | +++ | Cellobiose | - | - |
| Maltopentaose | +++ | +++ | Gentibiose | - | - |
| Maltohexaose | +++ | +++ | Maltitol | - | - |
| Maltoheptaose | +++ | +++ | Maltotriitol | + | + |
| Isomaltose | - | - | Lactose | - | - |
| Isomaltotriose | - | - | Sucrose | - | - |
| Panose | - | - | Erlose | + | + |
| Isopanose | ++ | ++ | Selaginose | - | - |
| Trehalose | - | - | Maltosylglucoside | ++ | ++ |
| Kojibiose | + | + | Isomaltosylglucoside | - | - |

| | | | | | |
|---|---|---|---|---|---|
| Note : Before and after the enzymatic reaction, the symbols "-", "+", "++", and "+++" mean that it showed no change, it showed a slight reduction of the color of substrate spot and the formation of other reaction product, it showed a high reduction of the color of substrate spot and the formation of other reaction product, and it showed a substantial disappearance of the color of substrate spot and the formation of other reaction product, respectively. | | | | | |

As evident from the results in Table 9, it was revealed that the α-isomaltosylglucosaccharide-forming enzyme well acted on saccharides having both a glucose polymerization degree of at least three and a maltose structure at their non-reducing ends, among the saccharides tested. It was also found that the enzyme slightly acted on saccharides, having a glucose polymerization degree of two, such as maltose, kojibiose, nigerose, neotrehalose, maltotriitol, and erlose.

### Experiment 6

### Reaction product from maltooligosaccharide

To an aqueous solution containing one percent (w/v) of maltose, maltotriose, maltotetraose, or maltopentaose as a substrate was added the purified specimen of α-isomaltosylglucosaccharide-forming enzyme in Experiment 4-1 in an amount of two units/g solid for the aqueous solution of maltose or maltotriose, 0.2 unit/g solid for that of maltotetraose, and 0.1 unit/g solid for that of maltopentaose, followed by incubation at °C and pH 6.0 for eight hours. After a 10-min incubation at 100°C, the enzymatic reaction was suspended. The resulting reaction solutions were respectively measured for saccharide composition on HPLC using "YMC PACK ODS-AQ303", a column commercialized by YMC Co., Ltd., Tokyo, Japan, at a column temperature of 40°C and a flow rate of 0.5 ml/min of water, and using as a detector "RI-8012", a differential refractometer commercialized by Tosoh Corporation, Tokyo, Japan. The results are in Table 10.

**Table 10**

| Saccharide as reaction product | Substrate | | | |
|---|---|---|---|---|
| | Maltose | Maltotriose | Maltotetraose | Maltopentaose |
| Glucose | 8.5 | 0.1 | 0.0 | 0.0 |
| Maltose | 78.0 | 17.9 | 0.3 | 0.0 |
| Maltotriose | 0.8 | 45.3 | 22.7 | 1.9 |
| Maltotetraose | 0.0 | 1.8 | 35.1 | 19.2 |
| Maltopentaose | 0.0 | 0.0 | 3.5 | 34.4 |
| Maltohexaose | 0.0 | 0.0 | 0.0 | 4.6 |
| Isomaltose | 0.5 | 0.0 | 0.0 | 0.0 |
| Glucosylmaltose | 8.2 | 1.2 | 0.0 | 0.0 |
| Glucosylmaltotriose | 2.4 | 31.5 | 6.8 | 0.0 |
| X | 0.0 | 2.1 | 30.0 | 11.4 |
| Y | 0.0 | 0.0 | 1.4 | 26.8 |
| Z | 0.0 | 0.0 | 0.0 | 1.7 |
| Others | 0.6 | 0.1 | 0.2 | 0.0 |

| | | | | |
|---|---|---|---|---|
| Note : In the table, glucosylmaltose means α-isomaltosylglucose, alias 6²-O-α-glucosylmaltose or panose; glucosylmaltotriose means α-isomaltosylmaltose, alias 6³-O-α-glucosylmaltotriose; X means the α-isomaltosylglucotriose in Experiment 7, alias 6⁴-O-α-glucosylmaltotetraose; Y means the α-isomaltosylglucotetraose in Experiment 7, alias 6⁵-O-α-glucosylmaltopentaose; and Z means an unidentified saccharide. | | | | |

As evident from the results in Table 10, it was revealed that, after the action of the enzyme, glucose and α-isomaltosylglucose, alias 6²-O-α-glucosylmaltose or panose, were mainly formed and maltotriose, isomaltose, and α-isomaltosylmaltose, alias 6³-O-α-glucosylmaltotriose were formed in a small amount when the enzyme acted on maltose as a substrate. Also, it was revealed that, from maltotriose as a substrate, maltose and α-isomaltosylmaltose were mainly formed along with small amounts of glucose, maltotetraose, α-isomaltosylglucose alias 6²-O-α-glucosylmaltose or panose, and the product X. It was also found that, from maltotetraose as a substrate, maltotriose and the product X were mainly formed along with small amounts of maltose, maltopentaose, α-isomaltosylmaltose alias 6³-O-α-glucosylmaltotriose or panose, and the product Y. Further, it was revealed that, from maltopentaose as a substrate, maltotetraose and the product Y were mainly formed along with small amounts of maltotriose, maltohexaose, and the products X and Z.

The product X as a main product from maltotetraose as a substrate and the product Y as a main product from maltopentaose as a substrate were respectively isolated and purified as follows: The products X and Y were respectively purified on HPLC using "YMC PACK ODS-A R355-15S-15 12A", a separatory HPLC column commercialized by YMC Co., Ltd., Tokyo, Japan, to isolate a specimen of the product X having a purity of at least 99.9% from the reaction product from maltotetraose in a yield of about 8.3%, d.s.b., and a specimen of the product Y having a purity of at least 99.9% from the reaction product from maltotetraose in a yield of about 11.5%, d.s.b.

### Experiment 7

### Structural analysis on reaction product

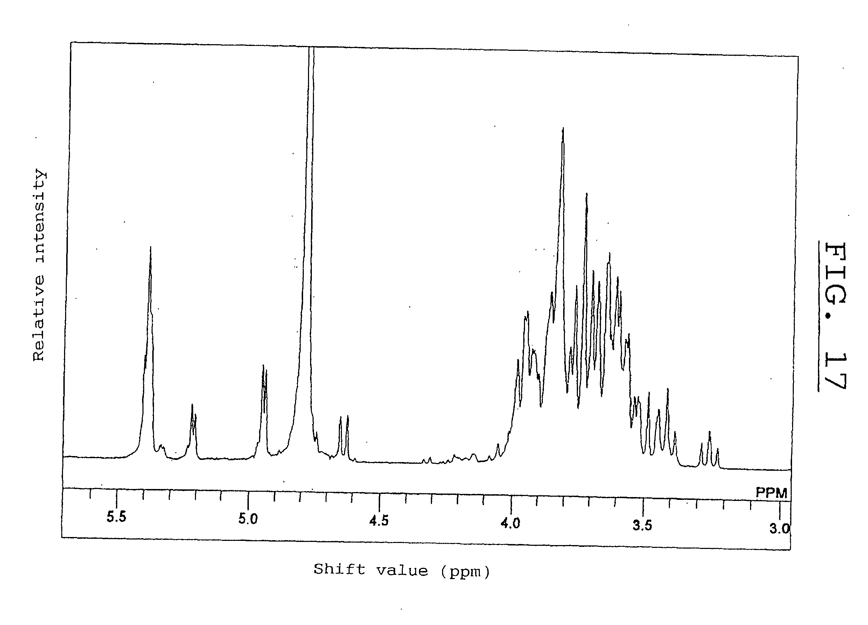

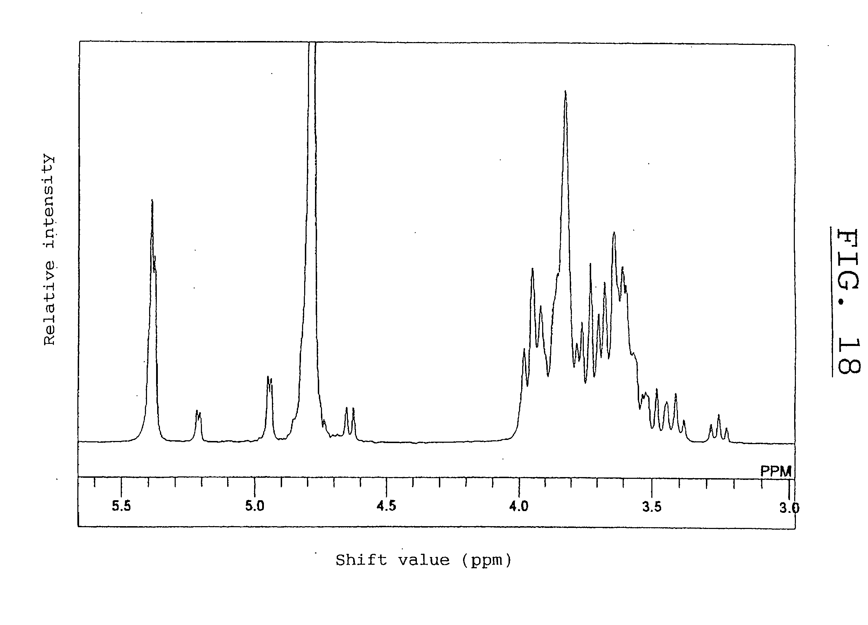

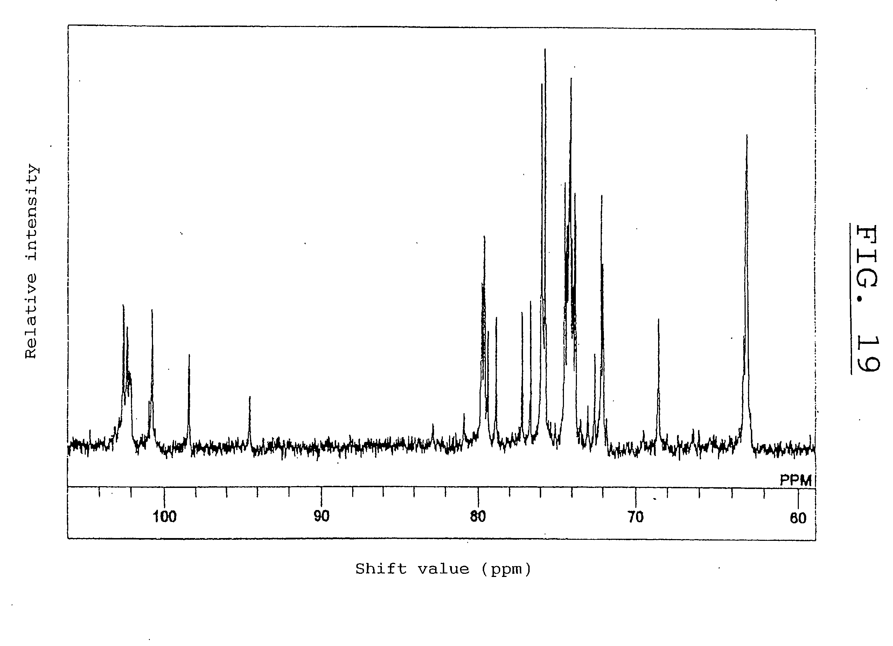

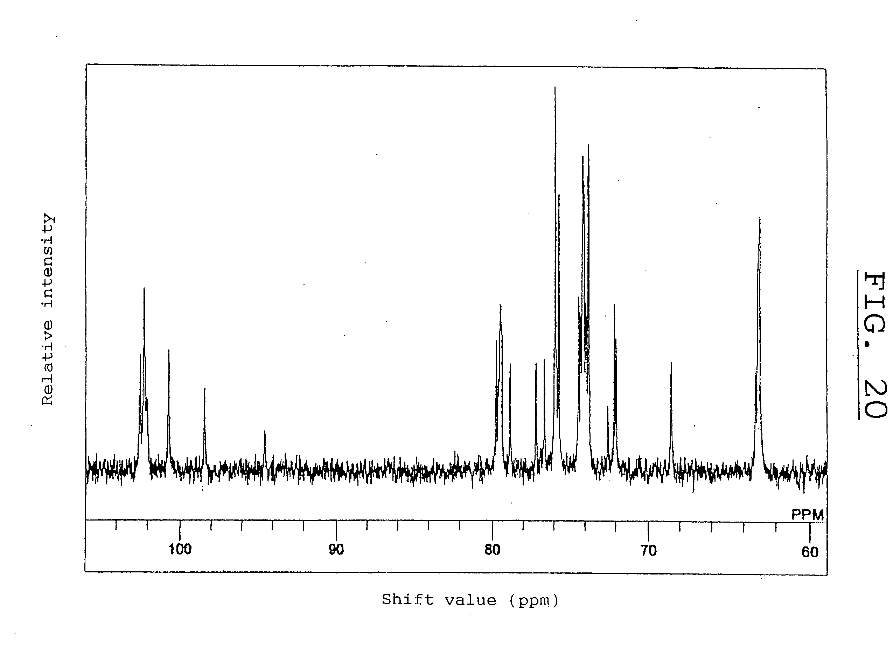

Using the products X and Y obtained by the method in Experiment 6, they were subjected to methyl analysis and NMR analysis in a usual manner. The results on their methyl analyses are in Table 11. For the results on their NMR analyses, FIG. 17 is a ¹H-NMR spectrum for the product X and FIG. 18 is for the product Y. The ¹³C-NMR spectra for the products X and Y are respectively in FIGs. 19 and 20, and their assignments are in Table 12.

**Table 11**

| Analyzed methyl compound | Ratio | |
|---|---|---|
| | Product X | Product Y |
| 2,3,4-trimethyl compound | 1.00 | 1.00 |
| 2,3,6-trimethyl compound | 3.05 | 3.98 |
| 2,3,4,6-tetramethyl compound | 0.82 | 0.85 |

**Table 12**

| | | NMR chemical shift value (ppm) | |
|---|---|---|---|
| Glucose number | Carbon number | Product X | Product Y |
| a | 1a | 100.8 | 100.8 |
| | 2a | 74.2 | 74.2 |
| | 3a | 75.8 | 75.7 |
| | 4a | 72.2 | 72.2 |
| | 5a | 74.5 | 74.5 |
| | 6a | 63.2 | 63.1 |
| b | 1b | 102.6 | 102.6 |
| | 2b | 74.2 | 74.2 |
| | 3b | 75.8 | 75.7 |
| | 4b | 72.1 | 72.1 |
| | 5b | 74.0 | 74.0 |
| | 6b | 68.6 | 68.6 |
| c | 1c | 102.3 | 102.3 |
| | 2c | 74.2 | 74.2 |
| | 3c | 76.0 | 76.0 |
| | 4c | 79.6 | 79.5 |
| | 5c | 73.9 | 73.9 |
| | 6c | 63.2 | 63.1 |
| d | 1d | 102.2 | 102.3 |
| | 2d | 74.0 (α), 74.4 (β) | 74.2 |
| | 3d | 76.0 | 76.0 |
| | 4d | 79.8 | 79.5 |
| | 5d | 73.9 | 73.9 |
| | 6d | 63 2 | 63.1 |
| e | 1e | 94.6 (α), 98.5 (β) | 102.1 |
| | 2e | 74.2 (α), 76.7 (β) | 74.0 (α), 74.4 (β) |
| | 3e | 75.9 (α), 78.9 (β) | 76. 0 |
| | 4e | 79.6 (α), 79.4 (β) | 79.8 |
| | 5e | 72.6 (α), 77.2 (β) | 73.9 |
| | 6e | 63.4 (α), 63.4 (β) | 63.1 |
| f | 1f | | 94.6 (α), 98.5 (β) |
| | 2f | | 79.2 (α), 76.7 (β) |
| | 3f | | 76.0 (α), 78.9 (β) |
| | 4f | | 79.6 (α), 79.5 (β) |
| | 5f | | 72.6 (α), 77.2 (β) |
| | 6f | | 63.3 (α), 63.3 (β) |

Based on these results, the product X formed from maltotetraose via the action of the α-isomaltosylglucosaccharide-forming enzyme was revealed as a pentasaccharide, in which a glucose residue binds via the α-linkage to OH-6 of glucose at the non-reducing end of maltotetraose, i.e., α-isomaltosylmaltotriose, alias 6⁴-O-α-glucosylmaltotetraose, represented by Formula 1. Formula 1:

α-D-Glcp-(1→6)-α-D-Glcp-(1→4)-α-D-Glcp-(1→4)-α-D-Glcp-(1→4)-D-Glcp

The product Y formed from maltopentaose was revealed as a hexasaccharide, in which a glucosyl residue binds via the α-linkage to OH-6 of glucose at the non-reducing end of maltopentaose, i.e., α-isomaltosylglucotetraose alias 6⁵-O-α-glucosylmaltopentaose, represented by Formula 2. Formula 2:

α-D-Glcp-(1→6)-α-D-Glcp-(1→4)-α-D-Glcp-(1→4)-α-D-Glcp-(1→4)-α-D-Glcp-(1→4)-D-Glcp

Based on these results, it was concluded that the α-isomaltosylglucosaccharide-forming enzyme acts on maltooligosaccharides as shown below:
(1) The enzyme acts on as substrates maltooligosaccharides having a glucose polymerization degree of at least two where glucoses are linked together via the α-1,4 linkage, and catalyzes the intermolecular 6-glucosyl-transferring reaction in such a manner of transferring a glucosyl residue at the non-reducing end of a maltooligosaccharide molecule to C-6 of the non-reducing end of other maltooligosaccharide molecule to form both an α-isomaltosylglucosaccharide alias 6-O-α-glucosylmaltooligosaccharide, having a 6-O-α-glucosyl residue and a higher glucose polymerization degree by one as compared with the intact substrate, and a maltooligosaccharide with a reduced glucose polymerization degree by one as compared with the intact substrate; and
(2) The enzyme slightly catalyzes the 4-glucosyl-transferring reaction and forms both a maltooligosaccharide, having an increased glucose polymerization degree by one as compared with the intact substrate, and a maltooligosaccharide having a reduced glucose polymerization degree by one as compared with the intact substrate.

### Experiment 8

### Specificity of saccharide transferring reaction acceptor

Using different saccharides, it was tested whether the saccharides were used as saccharide transferring reaction acceptors for the α-isomaltosylglucosaccharide-forming enzyme. A 1.6% solution, as a solution of saccharide transferring reaction acceptor, of D-glucose, D-xylose, L-xylose, D-galactose, D-fructose, D-mannose, D-arabinose, D-fucose, L-sorbose, L-rhamnose, methyl-α-glucopyranoside (methyl-α-glucose), methyl-β-glucopyranoside (methyl-β-glucose), N-acetylglucosamine, sorbitol, α,α-trehalose, isomaltose, isomaltotriose, cellobiose, gentibiose, maltitol, lactose, sucrose, α-cyclodextrin, β-cyclodextrin, or γ-cyclodextrin, was prepared. To each solution with a saccharide concentration was added "PINE-DEX #100", a partial starch hydrolysate, as a saccharide donor, to give a concentration of 4%, and admixed with one unit/g saccharide donor, d.s.b., of either of purified specimens of α-isomaltosylglucosaccharide-forming enzyme from *Bacillus globisporus* C9 strain obtained by the method in Experiment 2-1, *Bacillus globisporus* C11 strain obtained by the method in Experiment 4-1. The resulting mixture solutions were incubated at °C and pH 6.0 except that the enzyme from *Arthrobacter globiformis* A19 strain was incubated at pH 8.4 for 24 hours. The reaction mixtures of the post-enzymatic reactions were analyzed on gas chromatography (abbreviated as "GLC" hereinafter) for monosaccharides and disaccharides as acceptors, and on HPLC for trisaccharides as acceptors to confirm whether these saccharides could be used as their saccharide transferring reaction acceptors. In the case of performing GLC, the following apparatuses and conditions are used: GLC apparatus, "GC-16A" commercialized by Shimadzu Corporation, Tokyo, Japan; column, a stainless-steel column, 3 mm in diameter and 2 m in length, packed with 2% "SILICONE OV-17/CHROMOSOLV W", commercialized by GL Sciences Inc., Tokyo, Japan; carrier gas, nitrogen gas at a flow rate of 40 ml/min under temperature conditions of increasing from 160°C to 320°C at an increasing temperature rate of 7.5°C/min; and detection, a hydrogen flame ionization detector. In the case of HPLC analysis, the apparatuses and conditions used were: HPLC apparatus, "CCPD" commercialized by Tosoh Corporation, Tokyo, Japan; column, "ODS-AQ-303" commercialized by YMC Co., Ltd., Tokyo, Japan; eluent, water at a flow rate of 0.5 ml/min; and detection, a differential refractometer. The results are in Table 13.

**Table 13**

| Saccharide | Product of saccharide transferring reaction | | Saccharide | Product of saccharide transferring reaction | |
|---|---|---|---|---|---|
| | Enzyme of Strain C9 | Enzyme of Strain C11 | | Enzyme of Strain C9 | Enzyme of Strain C11 |
| D-Glucose | + | + | Sorbitol | - | - |
| D-Xylose | ++ | ++ | Trehalose | ++ | ++ |
| L-Xylose | ++ | ++ | Isomaltose | ++ | ++ |
| D-Galactose | + | + | Isomaltotriose | ++ | ++ |
| D-Fructose | + | + | Cellobiose | ++ | ++ |
| D-Mannose | - | - | Gentibiose | ++ | ++ |
| D-Arabinose | ± | ± | Maltitol | ++ | ++ |
| D-Fucose | + | + | Lactose | ++ | ++ |
| L-Sorbose | + | + | Sucrose | ++ | ++ |
| L-Rhamnose | - | - | α-Cyclodextrin | - | - |
| Methyl-α-glucopyranoside | ++ | ++ | β-Cyclodextrin | - | - |
| Methyl-β-glucopyranoside | ++ | ++ | γ-Cyclodextrin | - | - |
| N-Acetyl-glucosamine | + | + | | | |

| | | | | | |
|---|---|---|---|---|---|
| Note : In the table, the symbols "-", "±", "+", and "++" mean that no saccharide-transferre product was detected through transferring reaction to acceptor; a saccharide-transferred product was detected in an amount of less than one percent through transfer reaction to acceptor; a saccharide-transferred product was detected in an amount of at least one percent but less than ten percent through transferring reaction to acceptor; and a saccharide transferred product was detected in an amount of at least ten percent through transferring reaction to acceptor. | | | | | |

As evident from the results in Table 13, α-isomaltosylglucosaccharide-forming enzyme utilizes different types of saccharides as saccharide transfer acceptors, particularly, the enzyme has a higher saccharide transferring action, particularly, on D-/L-xylose, methyl-α-glucopyranoside, methyl-β-glucopyranoside, α,α-trehalose, isomaltose, isomaltotriose, cellobiose, gentibiose, maltitol, lactose, and sucrose; then on D-glucose, D-fructose, D-fucose, L-sorbose, and N-acetylglucosamine, as well as D-arabinose.

### Experiment 9

### Preparation of cyclotetrasaccharide from culture

A liquid medium consisting of 5% (w/v) of "PINE-DEX #1", a partial starch hydrolysate commercialized by Matsutani Chemical Ind., Tokyo, Japan, 1.5% (w/v) of "ASAHIMEAST", a yeast extract commercialized by Asahi Breweries, Ltd., Tokyo, Japan, 0.1% (w/v) of dipotassium phosphate, 0.06% (w/v) of sodium phosphate dodecahydrate, 0.05% (w/v) magnesium sulfate heptahydrate, and water was placed in a 500-ml Erlenmeyer flask in an amount of 100 ml, sterilized by autoclaving at 121°C for 20 min, cooled, and then seeded with *Bacillus globisporus* C9 strain, FERM BP-7143, followed by culturing under rotary-shaking conditions at 27°C and 230 rpm for 48 hours and centrifuging the resulting culture to remove cells to obtain a supernatant. The supernatant was autoclaved at 120°C for 15 min and then cooled, and the resulting insoluble substances were removed by centrifugation to obtain a supernatant. About 90 ml of the supernatant was adjusted to pH 5.0 and 45°C and then incubated for 24 hours after admixed with 1,500 units per gram of solids of "TRANSGLUCOSIDASE L AMANO^{™}", an α-glucosidase commercialized by Amano Pharmaceutical Co., Ltd., Aichi, Japan, and 75 units per gram of solids of a glucoamylase commercialized by Nagase Biochemicals, Ltd., Kyoto, Japan. Thereafter, the resulting culture was adjusted to pH 12 by the addition of sodium hydroxide and boiled for two hours to decompose the remaining reducing sugars. After removing insoluble substances by filtration, the resulting solution was decolored and desalted with "DIAION PK218" and "DIAION WA30", cation exchange resins commercialized by Mitsubishi Chemical Industries, Ltd., Tokyo, Japan, and further desalted with "DIAION SK-1B", commercialized by Mitsubishi Chemical Industries, Ltd., Tokyo, Japan, and "AMBERLITE IRA411", an anion exchange resin commercialized by Japan Organo Co., Ltd., Tokyo, Japan, followed by decoloring with an activated charcoal, membrane filtered, concentrated by an evaporator, and lyophilized *in vacuo* to obtain about 0.6 g, d.s.b., of a saccharide powder with a cyclotetrasaccharide content of 99.9% or higher.

### Experiment 10

### Formation of cyclotetrasaccharide

The formation test on cyclotetrasaccharide by the action of α-isomaltosylglucosaccharide-forming enzyme and α-isomaltosyl-transferring enzyme was conducted using saccharides. Using as saccharides maltose, maltotriose, maltotetraose, maltopentaose, amylose, soluble starch, "PINE-DEX #100", a partial starch hydrolyzate commercialized by Matsutani Chemical Ind., Tokyo, Japan, or glycogen from oyster commercialized by Wako Pure Chemical Industries Ltd., Tokyo, Japan, solutions containing each of the saccharides were respectively prepared.

To each of these solutions with a respective concentration of 0.5%, one unit/g solid of a purified specimen of α-isomaltosylglucosaccharide-forming enzyme from Strain C11 obtained by the method in Experiment 4-1 and 10 units/g solid of a purified specimen of α-isomaltosyl-transferring enzyme from Strain C11 obtained by the method in Experiment 4-4, and the resulting mixture was subjected to an enzymatic reaction at 30°C and pH 6.0. The enzymatic conditions were the following four systems:
(1) After the α-isomaltosylglucosaccharide-forming enzyme was allowed to act on a saccharide solution for 24 hours, the enzyme was inactivated by heating, and then the α-isomaltosyl-transferring enzyme was allowed to act on the resulting mixture for 24 hours and inactivated by heating;
(2) After the α-isomaltosylglucosaccharide-forming enzyme and the α-isomaltosyl-transferring enzyme were allowed in combination to act on a saccharide solution for 24 hours, then the saccharides were inactivated by heating;
(3) After only the α-isomaltosylglucosaccharide-forming enzyme was allowed to act on a saccharide solution for 24 hours, then the enzyme was inactivated by heating; and
(4) After only the α-isomaltosyl-transferring enzyme was allowed to act on a saccharide solution for 24 hours, then the enzyme was inactivated by heating.

To determine the formation level of cyclotetrasaccharide in each reaction mixture after heating, the reaction mixture was treated with α-glucosidase and glucoamylase similarly as in Experiment 1 to hydrolyze the remaining reducing oligosaccharides, followed by quantitation of cyclotetrasaccharide on HPLC. The results are in Table 14.

**Table 14**

| Substrate | Formation yield of cyclotetrasaccharide (%) | | | |
|---|---|---|---|---|
| | A | B | C | D |
| Maltose | 4.0 | 4.2 | 0.0 | 0.0 |
| Maltotriose | 10.2 | 12.4 | 0.0 | 0.0 |
| Maltotetraose | 11.3 | 21.5 | 0.0 | 0.0 |
| Maltopentaose | 10.5 | 37.8 | 0.0 | 0.0 |
| Amylose | 3.5 | 31.6 | 0.0 | 0.0 |
| Soluble starch | 5.1 | 38.2 | 0.0 | 0.0 |
| Partial starch hydrolyzate | 6.8 | 63.7 | 0.0 | 0.0 |
| Glycogen | 10.2 | 86.9 | 0.0 | 0.0 |

| | | | | |
|---|---|---|---|---|
| Note : The symbols "A", "B", "C" and "D" mean that α-isomaltosylglucosaccharide-forming enzyme was first allowed to act on a substrate and then α-isomaltosyl-transferring enzyme was allowed to act on the resulting mixture, the α-isomaltosylglucosaccharide-forming enzyme and α-isomaltosyl-transferring enzyme were allowed to coact on a substrate, only α-isomaltosylglucosaccharide-forming enzyme was allowed to act on a substrate, and only α-isomaltosyl-transferring enzyme was allowed to act on a substrate. | | | | |

As evident from the results in Table 14, no cyclotetrasaccharide was formed from any of the saccharides tested by the action of only α-isomaltosylglucosaccharide-forming enzyme or α-isomaltosyl-transferring enzyme, but cyclotetrasaccharide was formed by the coaction of these enzymes. It was revealed that the formation level of cyclotetrasaccharide was relatively low as below about 11% when α-isomaltosyl-transferring enzyme was allowed to act on the substrate saccharides after the action of α-isomaltosylglucosaccharide-forming enzyme, while the formation level was increased by simultaneously allowing the enzymes to act on every saccharide tested, particularly, increased to about 87% and about 64% when the enzymes were allowed to act on glycogen and partial starch hydrolyzate, respectively.

Based on the reaction properties of α-isomaltosylglucosaccharide-forming enzyme and α-isomaltosyl-transferring enzyme, the formation mechanism of cyclotetrasaccharide by the coaction of these enzymes is estimated as follows:
(1) α-Isomaltosylglucosaccharide-forming enzyme acts on a glucose residue at the non-reducing end of an α-1,4 glucan chain of glycogen and partial starch hydrolyzates, etc., and intermolecularly transfers the glucose residue to OH-6 of a glucose residue at the non-reducing end of other α-1,4 glucan chain of glycogen to form an α-1,4 glucan chain having an α-isomaltosyl residue at the non-reducing end;
(2) α-Isomaltosyl-transferring enzyme acts on the α-1,4 glucan chain having an α-isomaltosyl residue at the non-reducing end and intermolecularly transfers the isomaltosyl residue to C-3 of glucose residue at the non-reducing end of other α-1,4 glucan chain having isomaltosyl residue at the non-reducing end to form an α-1,4 glucan chain having an isomaltosyl-1,3-isomaltosyl residue at the non-reducing end;
(3) Then, α-isomaltosyl-transferring enzyme acts on the α-1,4 glucan chain having an isomaltosyl-1,3-isomaltosyl residue at the non-reducing end and releases the isomaltosyl-1,3-isomaltosyl residue from the α-1,4 glucan chain via the intramolecular transferring reaction to cyclize the released isomaltosyl-1,3-isomaltosyl residue into cyclotetrasaccharide;
(4) From the released α-1,4 glucan chain, cyclotetrasaccharide is newly formed through the sequential steps (1) to (3). Thus, it is estimated that the coaction of α-isomaltosylglucosaccharide-forming enzyme and α-isomaltosyl-transferring enzyme increases the formation of cyclotetrasaccharide in the above sequential manner.

### Experiment 11

### Influence of liquefaction degree of starch

A 15% corn starch suspension was prepared, admixed with 0.1% calcium carbonate, adjusted to pH 6.0, and then mixed with 0.2-2.0% per gram starch of "TERMAMYL 60L", an α-amylase specimen commercialized by Novo Indutri A/S, Copenhagen, Denmark, followed by the enzymatic reaction at 95°C for 10 min. Thereafter, the reaction mixture was autoclaved at 120°C for 20 min, promptly cooled to about 35°C to obtain a liquefied starch with a DE (dextrose equivalent) of 3.2-20.5. To the liquefied starch were added two units/g solid of a purified specimen of α-isomaltosylglucosaccharide-forming enzyme from Strain C11 obtained by the method in Experiment 4-1, and 20 units/g solid of a purified specimen of α-isomaltosyl-transferring enzyme from Strain C11 obtained by the method in Experiment 4-4, followed by the incubation at 35°C for 24 hours. After completion of the reaction, the reaction mixture was heated at 100°C for 15 min to inactivate the remaining enzymes. Then, the reaction mixture thus obtained was treated with α-glucosidase and glucoamylase similarly as in Experiment 1 to hydrolyze the remaining reducing oligosaccharides, followed by quantifying the formed cyclotetrasaccharide on HPLC. The results are in Tale 15.

**Table 15**

| Amount of α-amylase per starch (%) | DE | Yield of cyclotetrasaccharide (%) |
|---|---|---|
| 0.2 | 3.2 | 54.5 |
| 0.4 | 4.8 | 50.5 |
| 0.6 | 7.8 | 44.1 |
| 1.0 | 12.5 | 39.8 |
| 1.5 | 17.3 | 34.4 |
| 2.0 | 20.5 | 30.8 |

As evident from the results in Table 15, it was revealed that the formation of cyclotetrasaccharide by the coaction of α-isomaltosylglucosaccharide-forming enzyme and α-isomaltosyl-transferring enzyme is influenced by the liquefaction degree of starch, i.e., the lower the liquefaction degree or the lower the DE, the more the yield of cyclotetrasaccharide from starch becomes. On the contrary, the higher the liquefaction degree or the high the DE, the lower the yield of cyclotetrasaccharide from starch becomes. It was revealed that a suitable liquefaction degree is a DE of about 20 or lower, preferably, DE of about 12 or lower, more preferably, DE of about five or lower.

### Experiment 12

### Influence of concentration of partial starch hydrolyzate

Aqueous solutions of "PINE-DEX #100", a partial starch hydrolyzate with a DE of about two to about five, having a final concentration of 0.5-40%, were prepared and respectively admixed with one unit/g solid of a purified specimen of α-isomaltosylglucosaccharide-forming enzyme from Strain C11 obtained by the method in Experiment 4-1 and 10 units/g solid of a purified specimen of α-isomaltosyl-transferring enzyme from Strain C11 obtained by the method in Experiment 4-4, followed by the coaction of the enzymes at 30°C and pH 6.0 for 48 hours. After completion of the reaction, the reaction mixture was heated at 100°C for 15 min to inactivate the remaining enzymes, and then treated with α-glucosidase and glucoamylase similarly as in Experiment 1 to hydrolyze the remaining reducing oligosaccharides, followed by quantifying the formed cyclotetrasaccharide on HPLC. The results are in Table 16.

**Table 16**

| Concentration of PINE-DEX (%) | Formation yield of cyclotetrasaccharide (%) |
|---|---|
| 0.5 | 63.6 |
| 2.5 | 62.0 |
| 5 | 60.4 |
| 10 | 57.3 |
| 15 | 54.6 |
| 20 | 51.3 |
| 30 | 45.9 |
| 40 | 35.9 |

As evident from the results in Table 16, the formation yield of cyclotetrasaccharide was about 64% at a low concentration of 0.5%, while it was about 40% at a high concentration of 40%. The fact indicated that the formation yield of cyclotetrasaccharide increased depending on the concentration of partial starch hydrolyzate as a substrate. The result revealed that the formation yield of cyclotetrasaccharide increased as the decrease of partial starch hydrolyzate.

### Experiment 13

### Influence of the addition of cyclodextrin glucanotransferase

A 15% aqueous solution of "PINE-DEX #100", a partial starch hydrolyzate was prepared and admixed with one unit/g solid of a purified specimen of α-isomaltosylglucosaccharide-forming enzyme from Strain C11 obtained by the method in Experiment 4-1, 10 units/g solid of a purified specimen of α-isomaltosyl-transferring enzyme from Strain C11 obtained by the method in Experiment 4-4, and 0-0.5 unit/g solid of cyclodextrin glucanotransferase (CGTase) from a microorganism of the species *Bacillus stearothermophilus*, followed by the coaction of these enzymes at 30°C and pH 6.0 for 48 hours. After completion of the reaction, the reaction mixture was heated at 100°C for 15 min to inactivate the remaining enzymes, and then treated with "TRANSGLUCOSIDASE L AMANO^{™}", an α-glucosidase commercialized by Amano Pharmaceutical Co., Ltd., Aichi, Japan, and a glucoamylase specimen commercialized by Nagase Biochemicals, Ltd., Kyoto, Japan, to hydrolyze the remaining reducing oligosaccharides, followed by quantifying the formed cyclotetrasaccharide on HPLC. The results are in Table 17.

**Table 17**

| Amount of CGTase added (unit) | Formation yield of cyclotetrasaccharide (%) |
|---|---|
| 0 | 54.6 |
| 2.5 | 60.1 |
| 5 | 63.1 |
| 10 | 65.2 |

As evident from the results in Table 17, it was revealed that the addition of CGTase increased the formation yield of cyclotetrasaccharide.

### Experiment 15

### Preparation of isomaltose-releasing enzyme

A liquid nutrient culture medium, consisting of 3.0% (w/v) of dextran, 0.7% (w/v) of peptone, 0.2% (w/v) of dipotassium phosphate, 0.05% (w/v) magnesium sulfate heptahydrate, and water was placed in 500-ml Erlenmeyer flasks in a volume of 100 ml each, autoclaved at 121°C for 20 minutes to effect sterilization, cooled, inoculated with a stock culture of *Arthrobacter globiformis,* IAM 12103, and incubated at 27°C for 48 hours under rotary shaking conditions of 230 rpm for use as a seed culture. About 20 L of a fresh preparation of the same nutrient culture medium as used in the above culture were placed in a 30-L fermentor, sterilized by heating, cooled to 27°C, inoculated with 1% (v/v) of the seed culture, and incubated for about 72 hours while stirring under aeration agitation conditions at 27°C and a pH of 6.0-8.0. The resultant culture, having an activity of about 16.5 units/ml of α-isomaltodextranase as an isomaltose-releasing enzyme, was centrifuged at 10,000 rpm for 30 min to obtain about 18 L of a supernatant having an activity of about 16 units/ml of the enzyme and a total enzyme activity of about 288,000 units. The activity of isomaltodextranase was assayed as follows: Provide as a substrate solution a 1.25% (w/v) aqueous dextran solution containing 0.1M acetate buffer (pH 5.5), add one milliliter of an enzyme solution to the substrate solution, react the mixture solution at 40°C for 20 min, collect one milliliter of the reaction mixture, added the collected reaction mixture to two milliliters of Somogyi reagent to suspend the enzymatic reaction, and quantify the reducing power of the formed isomaltose by the Somogyi-Nelson's method. One unit of isomaltodextranase activity was defined as the enzyme amount that exhibits a reducing power corresponding to that of one micromole of isomaltose per minute under the above enzymatic reaction conditions. About 18 L of the resulting supernatant was concentrated with a UF membrane into an about two liter solution which was then dialyzed against 80% saturated ammonium sulfate solution at 4°C for 24 hours. The salted out precipitates were collected by centrifugation at 10,000 rpm for 30 min and dissolved in 5 mM phosphate buffer (pH 6.8), followed by dialyzing the resulting solution against a fresh preparation of the same phosphate buffer to obtain about 400 ml of a crude enzyme solution. The crude enzyme solution was subjected to ion-exchange chromatography using two liters of "SEPABEADS FP-DA13" gel. Isomaltodextranase was eluted in non-adsorbed fractions without adsorbing on the gel. The fractions with isomaltodextranase activity were collected, pooled and dialyzed against 80% saturated ammonium solution at 4°C for 24 hours. The resulting precipitates were collected by centrifugation at 10,000 rpm for 30 min and dissolved in 5 mM phosphate buffer (pH 6.8), and the solution was dialyzed against a fresh preparation of the same phosphate buffer to obtain about 500 ml of a partially purified enzyme solution having an activity of 161,000 units of isomaltodextranase.

### Experiment 16

### Preparation of isomaltose from α-isomaltosylglucosaccharide and cyclotetrasaccharide

To an aqueous solution having a final solid concentration of 0.2% (w/v) of panose, α-isomaltosylmaltose, α-isomaltosyltriose, α-isomaltosyltetraose, or cyclotetrasaccharide, were added 100 units/g solid of an isomaltodextranase specimen, obtained by the method in Experiment 15, except for using 100 or 3,000 units of the specimen for the aqueous solution of cyclotetrasaccharide, allowed to react at 40°C and pH 5.5 for 24 hours, and kept at 100°C for 20 min to suspend the enzymatic reactions. The saccharide composition for each reaction mixture was determined on HPLC. The conditions used in HPLC were: Column, "MCIGEL CK04SS" comercialized by Mitsubishi Chemical Industries, Ltd., Tokyo, Japan; 80°C, inner column temperature; 0.5 ml/min, a flow rate of water as an eluent; and detection, "RI-8012", a diffraction refractometer commercialized by Tosoh Corporation, Tokyo, Japan. The results are in Table 18.

**Table 18**

| Substrate | Amount of enzyme (unit) | Saccharide formed (peak area (%) on HPLC) | | | | | |
|---|---|---|---|---|---|---|---|
| | | G1 | IM | G2 | G3 | G4 | A |
| IMG1 | 100 | 35 | 65 | 0 | 0 | 0 | 0 |
| IMG2 | 100 | 0 | 51 | 49 | 0 | 0 | 0 |
| IMG3 | 100 | 0 | 41 | 0 | 59 | 0 | 0 |
| IMG4 | 100 | 0 | 35 | 0 | 0 | 65 | 0 |
| Cyclotetra-saccharide | 100 | 0 | 22 | 0 | 0 | 0 | 78 |
| | 3,000 | 0 | 100 | 0 | 0 | 0 | 0 |

| | | | | | | | |
|---|---|---|---|---|---|---|---|
| Note: The symbols "IMG1", "IMG2", "IMG3" and "IMG4" mean panose, α-isomaltosylmaltose, α-isomaltosyltriose, and isomaltosyltetraose, respectively; the symbols "G1", "1M", "G2", "G3", and "G4" mean glucose, isomaltose, maltose, maltotriose, and maltoteraose, respectively; and the symbol "A" means an intermediate product formed during the formation of isomaltose from cyclotetrasaccharide. | | | | | | | |

As evident from the result in Table 18, it was revealed that, when acts on α-isomaltosylglucosaccharides, isomaltodextranase forms only glucose and isomaltose from panose as a substrate; only isomaltose and maltose from α-isomaltosylmaltose as a substrate; only isomaltose and maltotriose from α-isomaltosyltriose as a substrate; and forms only isomaltose and maltotetraose from α-isomaltosyltetraose as a substrate. While it was revealed that the enzyme forms only isomaltose from cyclotetrasaccharide as a substrate through the product A as the intermediate.

Thereafter, the product A, as an intermediate formed from cyclotetrasaccharide as a substrate, was purified and isolated as follows: Using "YMC-PACK ODS-A R355-15S-15 12A", a separatory HPLC column commercialized by YMC Co., Ltd., Tokyo, Japan, the product A was purified and isolated, resulting in an isolation of the product A, having a purity of at least 98.2% in a yield of about 7.2%, from the reaction products formed from the material cyclotetrasaccharide.

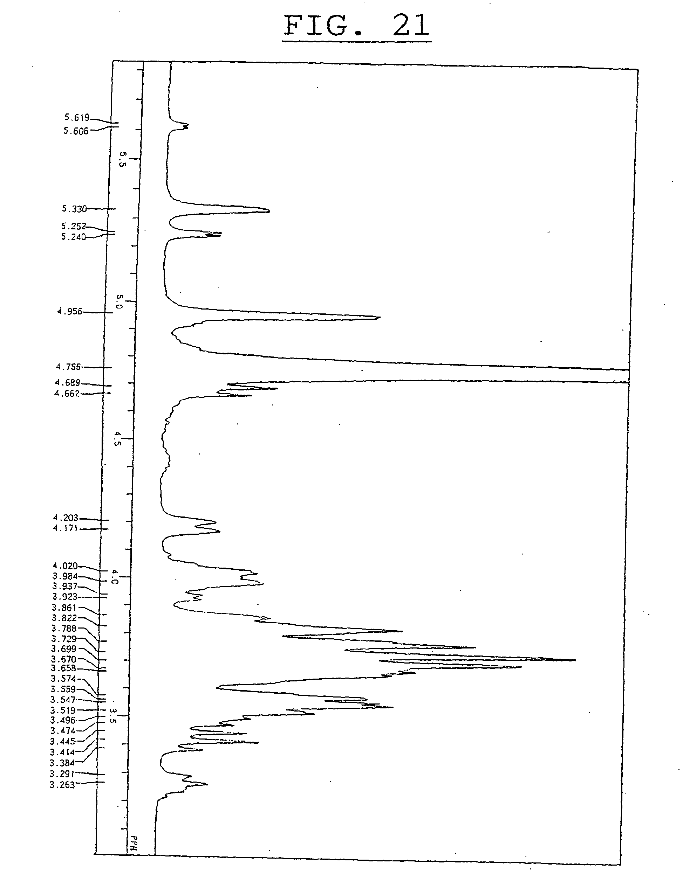

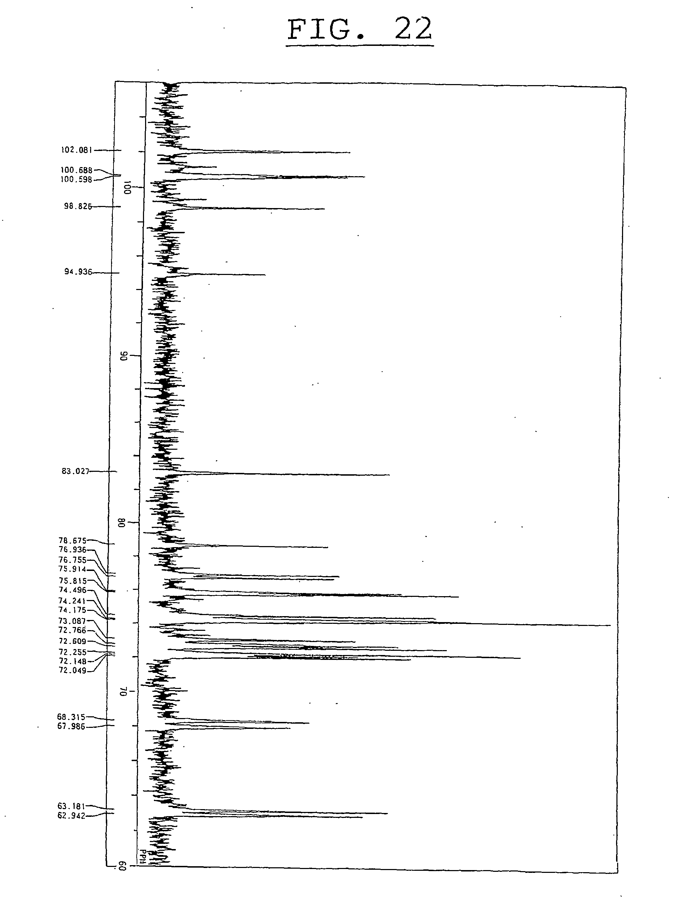

Upon the product A, it was subjected to methyl analysis and NMR analysis in a usual manner. The results on the methyl analysis is in Table 19. For the result on the NMR analysis, the ¹H-NMR spectrum is FIG. 21. The ¹³C-NMR spectrum for the product A is in FIG. 22, and the assignment thereof is tabulated in Table 20.

**Table 19**

| Analyzed methyl compound | Ratio |
|---|---|
| 2,3,4-trimethyl compound | 2.00 |
| 2,3,6-trimethyl compound | 0.92 |
| 2,3,4,6-tetramethyl compound | 0.88 |

**Table 20**

| Glucose number | Carbon Number | NMR Chemical shift (ppm) |
|---|---|---|
| | 1a | 100.7 |
| | 2a | 74.2 |
| a | 3a | 75.2 |
| | 4a | 72.3 |
| | 5a | 74.5 |
| | 6a | 63.2 |
| | 1b | 102.1 |
| | 2b | 74.3 |
| b | 3b | 75.9 |
| | 4b | 72.6 |
| | 5b | 74.2 |
| | 6b | 68.0 |
| | 1c | 100.6 |
| | 2c | 72.8 |
| c | 3c | 83.0 |
| | 4c | 72.0 |
| | 5c | 73.1 |
| | 6c | 62.9 |
| | 1e | 94.9 (α), 98.8 (β) |
| | 2e | 74.1 (α), 76.6 (β) |
| e | 3e | 75.8 (α), 78.7 (β) |
| | 4e | 72.1 (α), 72.1 (β) |
| | 5e | 72.6 (α), 76.9 (β) |
| | 6e | 68.3 (α), 68.3 (β) |

Based on these results, it was revealed that the product A, as an intermediate, formed during the formation of isomaltose from cyclotetrasaccharide via the action of isomaltodextranase was a tetrasaccharide represented by Formula 3, α-D-glucosyl-(1→6)-α-D-glucosyl-(1→3)-α-D-glucosyl-(1→6)-α-D-glucose (designated as "ring-opened tetrasaccharide" hereinafter), obtained by hydrolyzing either of the α-1,3 linkages in cyclotetrasaccharide for ring opening. Formula 3:

α-D-Glcp-(1→6)-α-D-Glcp-(1→3)-α-D-Glcp-(1→6)-α-D-Glcp

Based on these results, the action of isomaltodextranase on α-isomaltosylglucosaccharide is judged as follows:

Isomaltodextranase acts on α-isomaltosylglucosaccharides having a 6-O-α-glucosyl residue as substrates to specifically hydrolyze the α-1,4 linkage between the isomaltosyl residue at the non-reducing end and the glucose residue (or a maltooligosaccharide residue) to form isomaltose and glucose (or a maltooligosaccharide). The enzyme also acts on cyclotetrasaccharide as a substrate and hydrolyzes its α-1,3 linkage, and further acts on a ring-opened tetrasaccharide and hydrolyzes its α-1,3 linkage to form isomaltose.

### Experiment 17

### Formation of isomaltose from substrates

Using different substrates, the isomaltose formation by the action of α-isomaltosylglucosaccharide-forming enzyme and isomaltodextranase was tested. Using calcium chloride and maltose, maltotriose, maltotetraose, maltopentaose, maltohexaose, maltoheptaose, amylose, or "PINE-DEX #100", a partial starch hydrolyzate commercialized by Matsutani Chemical Ind., Tokyo, Japan, aqueous solutions for saccharides each were prepared to give a final saccharide concentration of 5% and a final calcium chloride concentration of 1 mM. Then, to each solution were added 0.2 unit/g solids of the purified α-isomaltosylglucosaccharide-forming enzyme from Strain C11 obtained in Experiment 4-1 and 100 units/g solids of the isomaltodextranase obtained in Experiment 15, followed by reacting at 40°C at pH 5.5. The reaction conditions were conducted in the following two reaction systems:
(1) After contacting α-isomaltosylglucosaccharide-forming enzyme with each saccharide for 65 hours, the enzyme was inactivated by heating, and then isomaltodextranase was further allowed to act on the saccharide for 65 hours and inactivated by heating.
(2) After contacting α-isomaltosylglucosaccharide-forming enzyme in combination with isomaltodextranase with each saccharide for 65 hours, the enzymes were inactivated by heating.

After the above enzymatic reactions, the formation yield of isomaltose in the resulting reaction mixtures received with heat treatment was quantified on HPLC. The results are in Table 21.

**Table 21**

| Substrate | Formation yield of isomaltose (%) | |
|---|---|---|
| | A | B |
| Maltose | 6.6 | 7.0 |
| Maltotriose | 15.7 | 18.7 |
| Maltotetraose | 15.8 | 45.4 |
| Maltopentaose | 15.3 | 55.0 |
| Maltohexaose | 10.1 | 58.1 |
| Maltoheptaose | 8.5 | 63.6 |
| Amylose | 4.0 | 64.9 |
| Partial starch hydrolyzate | 3.8 | 62.7 |

| | | |
|---|---|---|
| Note: The symbol "A" means that after contacting α-isomaltosylglucosaccharide-forming enzyme with each saccharide, isomaltodextranase was further allowed to act on the resulting mixture. The symbol "B" means that α-isomaltosylglucosaccharide-forming enzyme and isomaltodextranase were used in combination. | | |

As evident from the results in Table 21, from every saccharide tested, isomaltose was formed via the action of α-isomaltosylglucosaccharide-forming enzyme and isomaltodextranase. It was revealed that, in the case of sequentially contacting α-isomaltosylglucosaccharide-forming enzyme and isomaltodextranase with each saccharide, the formation yield of isomaltose was relatively low as about 15%, while in the case of contacting these enzymes in a combinative manner with any of the saccharides, the formation yield of isomaltose was improved, particularly, it was improved up to 60% or higher when acted on maltoheptaose, amylose, and partial starch hydrolyzate. The mechanism of forming isomaltose by the combination use of α-isomaltosylglucosaccharide-forming enzyme and isomaltodextranase would be as follows:
(1) α-Isomaltosylglucosaccharide-forming enzyme acts on a glucose residue at the non-reducing end of an α-1,4 glucan chain such as amylose and partial starch hydrolyzate and transfers the glucose residue to the C-6 hydroxyl group of another glucose residue at the non-reducing end of another α-1,4 glucan chain to form a α-1,4 glucan chain having an α-isomaltosyl residue at the non-reducing end;
(2) Isomaltodextranase acts on an α-1,4 glucan chain having an isomaltosyl residue at the non-reducing end and hydrolyzes the α-1,4 linkage between the isomaltosyl residue and hydrolyzes the α-1,4 linkage between the isomaltosyl residue and the α-1,4 glucan chain to form a glucan chain, free of the isomaltose, with a lowered glucose polymerization degree by two; and
(3) The released α-1,4 glucan chain is again sequentially received the steps (1) and (2) to newly form isomaltose.

It would be estimated that, through the combination use of α-isomaltosylglucosaccharide-forming enzyme and isomaltodextranase, the formation yield of isomaltose would be increased by the repeated action of the enzymes on α-1,4 glucan chains as described above.

### Experiment 18

### Effect of the addition of isoamylase

Aqueous solutions of "PINE-DEX #100", a partial starch hydrolyzate, having a final concentration of 5% and 1 mM calcium chloride, were prepared, admixed with 0.2 unit/g starch of the purified α-isomaltosylglucosaccharide-forming enzyme from Strain C11 in Experiment 4-1, 100 units/g starch of the isomaltodextranase in Experiment 15, and 0-250 units/g starch of an isoamylase specimen from a microorganism of the species *Pseudomonas amyloderamosa* commercialized by Hayashibara Biochemical Laboratories, Inc., Okayama, Japan, incubated at 40°C and pH 5.5 for 65 hours, and then heated at 100°C for 15 min to inactivate the enzymes used. The formed isomaltose was quantified on HPLC. The results are in Table 22.

**Table 22**

| Amount of isoamylase added (unit) | Formation yield of isomaltose (%) |
|---|---|
| 0 | 62.7 |
| 50 | 65.1 |
| 250 | 71.1 |

As evident form the results in Table 22, it was revealed that the addition of isoamylase increases the formation yield of isomaltose.

### Experiment 19

### Influence of the concentration of partial starch hydrolyzate

Eight types of aqueous solutions with different concentrations of "PINE-DEX #100", a partial starch hydrolyzate with a DE of about 2-5, having final concentrations of 1-40% and 1 mM calcium chloride, were prepared. To each aqueous solution 0.2 unit/g starch of the purified α-isomaltosylglucosaccharide-forming enzyme from Strain C11 in Experiment 4-1, 100 units/g starch of the isomaltodextranase in Experiment 15, and 250 units/g starch of an isoamylase specimen from a microorganism of the species *Pseudomonas amyloderamosa* commercialized by Hayashibara Biochemical Laboratories, Inc., Okayama, Japan, incubated at 40°C and pH 5.5 for 65 hours, and then heated at 100°C for 15 min to inactivate the enzymes used. The formed isomaltose was quantified on HPLC. The results are in Table 23.

**Table 23**

| Concentration of PINE-DEX (%) | Formation yield of isomaltose (%) |
|---|---|
| 1 | 73.0 |
| 2.5 | 72.8 |
| 5 | 71.1 |
| 10 | 67.0 |
| 15 | 63.7 |
| 20 | 60.7 |
| 30 | 55.4 |
| 40 | 50.7 |

As evident from the results in Table 23, it was revealed that the formation of yield of isomaltose was about 73% at a concentration of one percent of partial starch hydrolyzate, while it was about 51% at a relatively high concentration of 40%. Thus, the formation of yield of isomaltose changes depending on the concentration of partial starch hydrolyzate as a substrate.

### Experiment 20

### Influence of the liquefaction degree of starch

Corn starch was prepared into a 15% starch suspension which was then mixed with 0.1% calcium carbonate, adjusted to pH 6.0, admixed with 0.2-2.0% per gram starch of "TERMAMYL 60L", an α-amylase specimen commercialized by Novo Indutri A/S, Copenhagen, Denmark, allowed to react at 95°C for 10 min, and autoclaved at 120°C. Thereafter, the reaction mixture was promptly cooled to about 40°C to obtain a liquefied solution with a DE of 3.2-20.5 which was then adjusted to pH 5.5, admixed with 0.2 unit/g starch of the purified α-isomaltosylglucosaccharide-forming enzyme from Strain C11 in Experiment 4-1, 100 units/g starch of the isomaltodextranase in Experiment 15, and 250 units/g starch of an isoamylase specimen from a microorganism of the species *Pseudomonas amyloderamosa* commercialized by Hayashibara Biochemical Laboratories, Inc., Okayama, Japan, incubated at 40°C for 65 hours, and then heated at 100°C for 15 min to inactivate the enzymes used. The formed isomaltose was quantified on HPLC. The results are in Table 24.

**Table 24**

| Amount of α-amylase used (% (w/w) per gram starch) | DE | Formation yield of isomaltose (%) |
|---|---|---|
| 0.2 | 3.2 | 71.5 |
| 0.4 | 4.8 | 71.0 |
| 0.6 | 7.8 | 66.2 |
| 1.0 | 12.5 | 59.8 |
| 1.5 | 17.3 | 53.2 |
| 2.0 | 20.5 | 47.9 |

As evident from the results in Table 24, it was revealed that the liquefaction degree of starch influences the formation yield of isomaltose using α-isomaltosylglucosaccharide-forming enzyme and isomaltodextranase; the lower the liquefaction degree or the lower the DE, the higher the formation yield of isomaltose becomes, in reverse, the higher the liquefaction degree or the higher the DE, the lower the formation yield of isomaltose becomes; it was revealed that the liquefaction degree should preferably be a DE not higher than 20, preferably, DE not higher than 12, more preferably, DE not higher than five.

### Experiment 23

### Effect of the addition of cyclodextrin glucanotransferase and glucoamylase

Aqueous solutions of "PINE-DEX #100", a partial starch hydrolyzate, having a final concentration of 20% and 1 mM calcium chloride, were prepared and admixed with 0.2 unit/g starch of the purified α-isomaltosylglucosaccharide-forming enzyme from Strain C11 in Experiment 4-1, 100 units/g starch of the isomaltodextranase in Experiment 15, 250 units/g starch of an isoamylase specimen from a microorganism of the species *Pseudomonas amyloderamosa* commercialized by Hayashibara Biochemical Laboratories, Inc., Okayama, Japan, and 0-0.5 unit/g starch of a CGTase specimen from a microorganism of the species *Bacillus stearothermophilus*, commercialized by Hayashibara Biochemical Laboratories, Inc., Okayama, Japan, and incubated with these enzymes at 40°C and pH 5.5 for 65 hours. Thereafter, each reaction mixture was heated at 100°C for 15 min to inactivate the enzymes, admixed with 20 units/g starch of "XL-4", a glucoamylase commercialized by Nagase Biochemicals, Ltd., Kyoto, Japan, incubated at 50°C for 24 hours, and heated at 100°C for 20 min to inactivate the remaining enzyme. The formed isomaltose was quantified on HPLC. The results are in Table 25.

**Table 25**

| Amount of CGTase added (unit per gram starch) | Formation yield of isomaltose (%) |
|---|---|
| 0 | 60.7 |
| 0.1 | 62.9 |
| 0.25 | 65.0 |
| 0.5 | 66.4 |

As evident from the results in Table 25, it was revealed that the addition of CGTase to the enzymatic system of α-isomaltosylglucosaccharide-forming enzyme and isomaltodextranase increases the formation yield of isomaltose. The object of using the glucoamylase was to increase the formation of isomaltose by releasing D-glucose residue(s) from a saccharide composed of isomaltose and at least one D-glucose residue.

With reference to the following Examples A and B, the process for producing isomaltose or high isomaltose content products according to the present invention and uses thereof are disclosed in detail:

### Example A-1

About 100 L of an aqueous solution of phytoglycogen from corn, commercialized by Q.P. Corporation, Tokyo, Japan, were adjusted to give a concentration of 4% (w/v), pH 6.0, and a temperature of 30°C, admixed with one unit/g starch of a purified α-isomaltosylglucosaccharide-forming enzyme from Strain C11 obtained by the method in Experiment 4-1, and 10 units/g starch of an α-isomaltosyl-transferring enzyme from Strain C11 obtained by the method in Experiment 4-4, allowed to react for 48 hours, and heated at 100°C for 10 min to inactivate the remaining enzymes. The resulting mixture was sampled and quantified the formation yield of cyclotetrasaccharide on HPLC to be about 84% with respect to the saccharide composition, wherein HPLC was carried out under the conditions of: Column, "SHODEX KS-801 COLUMN" comercialized by Showa Denko K.K., Tokyo, Japan; 60°C, an inner column temperature; 0.5 ml/min, a flow rate of water as an eluent; and detection by "RI-8012", a diffraction refractometer commercialized by Tosoh Corporation, Tokyo, Japan. After adjusted to pH 5.0 and 45°C, the resulting reaction mixture was admixed with 1,500 units/g starch "TRANSGLUCOSIDASE L AMANO^{™}", an α-glucosidase commercialized by Amano Pharmaceutical Co., Ltd., Aichi, Japan, and 75 units/g starch of "XL-4", a glucoamylase specimen commercialized by Nagase Biochemicals, Ltd., Kyoto, Japan, to hydrolyze the remaining reducing-oligosaccharides. Then, the resulting mixture was adjusted to give a pH 5.8 by the addition of sodium hydroxide, kept at 90°C for one hour to inactivate the remaining enzymes, and filtered to remove insoluble substances. The filtrate was concentrated using "HOLLOSEP^{®} HR5155PI", a reverse osmosis membrane commercialized by Toyobo Co., Ltd., Tokyo, Japan, up to give a concentration of about 16% (w/v). Then, the concentrate was in a usual manner decolored, desalted, filtered, and concentrated into about 6.2 kg of a saccharide solution having about 3,700 g of solid contents. The saccharide solution was fed to a column packed with about 225 L of "AMBERLITE CR-1310 (Na⁺-form)", a strong-acid cation-exchanger commercialized by Japan Organo Co., Ltd., Tokyo, Japan, and chromatographed at a column temperature of 60°C and a flow rate of about 45 L/h. While monitoring the saccharide composition of the eluate by the above HPLC, fractions of cyclotetrasaccharide with a purity of 98% or higher were collected and pooled, and then in a usual manner, desalted, decolored, filtered, and concentrated to obtain about 7.5 kg saccharide solution with a solid content of about 2,500 g. The HPLC revealed that the saccharide solution had a purity of about 99.5% of cyclotetrasaccharide. The obtained saccharide solution containing cyclotetrasaccharide was concentrated by an evaporator to give a concentration of about 50%, and about 5 kg of the concentrate was placed in a cylindrical plastic container and cooled from 65°C to 20°C over about 20 hours under gentle stirring conditions to crystallize cyclotetrasaccharide. Thereafter, the resulting massecuite was centrifuged to separate 1,360 g of cyclotetrasaccharide crystal on a wet weight, which was then dried at 60°C for three hours to obtain 1,170 g of a powdery cyclotetrasaccharide crystal. The powdery crystal was analyzed for saccharide composition on HPLC to reveal that it had a quite high purity of at least 99.9% of cyclotetrasaccharide.

The powdery cyclotetrasaccharide crystal thus obtained was dissolved in deionized water to give a concentration of one percent, pH 5.5, and 50°C, followed by admixing with 500 units/g solids of an isomaltodextranase specimen obtained by the method in Experiment 15, and incubating the mixture at pH 5.5 and 50°C for 70 hours. After completion of the enzymatic reaction, the reaction mixture was heated to 95°C and kept at the temperature for 10 min, cooled, and filtered. The resulting filtrate was in a usual manner decolored with an activated charcoal, desalted and purified using ion-exchange resins in H- and OH-forms, and further concentrated to give a concentration of 75%. Thus, a high isomaltose content syrup was obtained in a yield of about 95%, d.s.b.

The product contained 96.1% isomaltose, 2.8% ring-opened tetrasaccharide, and 1.1% other saccharides, d.s.b. Since the product substantially free of crystallization has a satisfactory humectancy, low-sweetness, osmosis controllability, filler-imparting ability, gloss-imparting ability, viscosity, ability of preventing crystallization of other saccharides, insubstantial fermentability, ability of preventing retrogradation of starches, etc., it can be arbitrarily used in foods, beverages, health foods, feeds, pet foods, cosmetics, pharmaceuticals, tobaccos, and cigarettes.

### Example A-2

A high isomaltose content syrup, obtained by the method in Example A-1, was subjected to column chromatography using "AMBERLITE CR-1310 (Na⁺-form)", a strong-acid cation exchanger commercialized by Japan Organo Co., Ltd., Tokyo, Japan. The resin was packed into 10 jacketed stainless steel columns having a diameter of 12.5 cm, which were then cascaded in series to give a total gel bed depth of 16 m. Under the conditions of keeping the inner column temperature at 40°C, the above saccharide syrup was fed to the columns in a volume of 1.5% (v/v) and fractionated by feeding to the columns hot water heated to 40°C at an SV (space velocity) of 0.2 to obtain high isomaltose content fractions while monitoring the saccharide composition of eluate on HPLC. Then, the fractions were pooled and purified to obtain a high isomaltose content solution in a yield of about 80%, d.s.b. The solution was in a usual manner decolored, desalted, and concentrated into an about 75%, d.s.b., of high isomaltose content syrup.

The product contained a high purity isomaltose with a purity of at least 99.9%, d.s.b. Since the product substantially free of crystallization has a satisfactory humectancy, low-sweetness, osmosis controllability, filler-imparting ability, gloss-imparting ability, viscosity, ability of preventing crystallization of other saccharides, insubstantial fermentability, ability of preventing retrogradation of starches, etc., it can be arbitrarily used in foods, beverages, health foods, feeds, pet foods, cosmetics, pharmaceuticals, tobaccos, and cigarettes.

### Example A-3

A tapioca starch was prepared into an about 20% starch suspension, admixed with calcium carbonate to give a concentration of 0.1%, adjusted to pH 6.5, further admixed with 0.3% per gram starch, d.s.b., of "TERMAMYL 60L", an α-amylase commercialized by Novo Industri A/S, Copenhagen, Denmark, and then heated at 95°C for about 15 min. Thereafter, the mixture was autoclaved at 120°C for 20 min and then promptly cooled to about 40°C to obtain a liquefied solution with a DE of about four. To the liquefied solution was added 0.2 unit/g starch of an α-isomaltosylglucosaccharide-forming enzyme obtained by the method in Experiment 2-1, 100 units/g starch of an isomaltodextranase obtained by the method in Experiment 15, 250 units/g starch of an isomaltodextranase specimen commercialized by Hayashibara Biochemical Laboratories, Inc., Okayama, Japan, and 0.5 unit/g starch of a CGTase specimen commercialized by Hayashibara Biochemical Laboratories, Inc., Okayama, Japan, and then enzymatically reacted at pH 5.5 and 40°C for 64 hours. The reaction mixture was kept at 95°C for 30 min, adjusted to 50°C, 10 units/g solids of "GLUCOZYME", a glucoamylase preparation commercialized by Nagase Biochemicals, Ltd., Kyoto, Japan, and then enzymatically reacted for 24 hours. The reaction mixture thus obtained was heated to and kept at 95°C for 30 min, and then cooled and filtered. The filtrate was in a conventional manner decolored with an activated charcoal, desalted and purified with ion exchangers in H- and OH-forms, and then concentrated, dried, pulverized, and granulated into isomaltose granules in a yield of about 95%, d.s.b.

The product contains, on a dry solid basis, 11.0% glucose, 66.5% isomaltose, 2.4% other disaccharides, and 20.1% trisaccharides or higher. Since the product has a satisfactory humectancy, low-sweetness, osmosis controllability, filler-imparting ability, gloss-imparting ability, viscosity, ability of preventing crystallization of other saccharides, insubstantial fermentability, ability of preventing retrogradation of starches, etc., it can be arbitrarily used in foods, beverages, health foods, feeds, pet foods, cosmetics, pharmaceuticals, tobaccos, and cigarettes.

### Example A-4

*Bacillus globisporus* C9 strain, FERM BP-7143, was cultured by a fermentor for 48 hours in accordance with the method in Experiment 1. After completion of the culture, the resulting culture was filtered with an SF membrane to remove cells and to collect about 18 L of a culture supernatant. Then the culture supernatant was concentrated with a UF membrane to collect about one liter of a concentrated enzyme solution containing 8.8 units/ml of an α-isomaltosylglucosaccharide-forming enzyme and 26.7 units/ml of an α-isomaltosyl-transferring enzyme. A potato starch was prepared into an about 27% starch suspension which was then admixed with 0.1% calcium carbonate, adjusted to pH 6.5, admixed with 0.3% per gram starch, d.s.b., of "TERMAMYL 60L", an α-amylase commercialized by Novo Industri A/S, Copenhagen, Denmark, and then sequentially heated at 95°C for 15 min, autoclaved at 120°C for 20 min, and promptly cooled to about 40°C to obtain a liquefied solution with a DE of about four. To the liquefied solution were added 0.25 ml per gram of starch of the above concentrated enzyme solution containing the α-isomaltosylglucosaccharide-forming enzyme and the α-isomaltosyl-transferring enzyme, 100 units/g starch of an isomaltodextranase obtained by the method in Experiment 15, 250 units/g starch of an isoamylase specimen commercialized by Hayashibara Biochemical Laboratories, Inc., Okayama, Japan, and 0.5 unit/g starch of a CGTase specimen commercialized by Hayashibara Biochemical Laboratories, Inc., Okayama, Japan, and then the resulting mixture was subjected to enzymatic reaction at pH 5.5 and 40°C for 70 hours. The reaction mixture was heated to and kept at 95°C for 10 min, adjusted to 50°C, admixed with 20 units/g starch of "GLUCOZYME", a glucoamylase preparation commercialized by Nagase Biochemicals, Ltd., Kyoto, Japan, and then enzymatically reacted for 24 hours. The reaction mixture thus obtained was heated to and kept at 95°C for 30 min, and then cooled and filtered. The filtrate was in a conventional manner decolored with an activated charcoal, desalted and purified with ion exchangers in H- and OH-forms and concentrated to obtain a 75% high isomaltose content syrup in a yield of about 95%, d.s.b.

The product contains, on a dry solid basis, 32.6% glucose, 59.4% isomaltose, 1.2% other disaccharides, and 6.8% trisaccharides or higher. Since the product substantially free of crystallization has a satisfactory humectancy, low-sweetness, osmosis controllability, filler-imparting ability, gloss-imparting ability, viscosity, ability of preventing crystallization of other saccharides, insubstantial fermentability, ability of preventing retrogradation of starches, etc., it can be arbitrarily used in foods, beverages, health foods, feeds, pet foods, cosmetics, pharmaceuticals, tobaccos, and cigarettes.

### Example A-5

To increase the isomaltose content in the high isomaltose content syrup in Example A-4 as a material saccharide solution, in accordance with the method in Example A-2, the syrup was subjected to column chromatography using a strong-acid cation exchange resin, followed by collecting the resulting high isomaltose content fractions which were then pooled and concentrated to obtain a high isomaltose content syrup in a yield of about 60%, d.s.b.

The product contains, on a dry solid basis, 4.8% glucose, 85.3% isomaltose, 3.9% other disaccharides, and 6.0% trisaccharides or higher. Since the product substantially free of crystallization has a satisfactory humectancy, low-sweetness, osmosis controllability, filler-imparting ability, gloss-imparting ability, viscosity, ability of preventing crystallization of other saccharides, insubstantial fermentability, ability of preventing retrogradation of starches, etc., it can be arbitrarily used in foods, beverages, health foods, feeds, pet foods, cosmetics, pharmaceuticals, tobaccos, and cigarettes.

### Example B-1

### Sweetener

To 0.8 part by weight of a high isomaltose content powder, obtained by the method in Example A-1, were homogeneously added 0.2 part by weight of "TREHA^{®}", a crystalline trehalose hydrate commercialized by Hayashibara Shoji Inc., Okayama, Japan, 0.01 part by weight of "αG SWEET^{™}" (α-glycosyl stevioside commercialized by Toyo Sugar Refining Co., Tokyo, Japan), and 0.01 part by weight of "ASPARTAME" (L-aspartyl-L-phenylalanine methyl ester). The resulting mixture was fed to a granulator to obtain a granular sweetener. The product has a satisfactory sweetness and about 2-fold higher sweetening power of sucrose. The product is a low-sweetener composition containing isomaltose which is substantially free of crystallization and has satisfactory humectancy and low-sweetness. The product has a satisfactory stability with lesser fear of causing quality deterioration even when stored at ambient temperature.

### Example B-2

### Hard candy

One hundred parts by weight of a 55% sucrose solution was admixed while heating with 50 parts by weight of a high isomaltose content syrup obtained by the method in Example A-2. The mixture was then concentrated by heating under reduced pressure to give a moisture content of less than 2%, and the concentrate was mixed with 0.6 part by weight of citric acid and adequate amounts of a lemon flavor and a color, followed by forming in a usual manner the resultant mixture into the desired product. The product is a stable, high quality hard candy which has a satisfactory mouth feel, taste, and flavor, less adsorbs moisture, and does not cause crystallization of sucrose.

### Example B-3

### Chewing gum

Three parts by weight of a gum base were melted by heating to an extent to be softened and then admixed with two parts by weight of anhydrous crystalline maltitol anhydride, two parts by weight of xylitol, two parts by weight of a high isomaltose content syrup obtained by the method in Example A-5, and one part by weight of trehalose, and further mixed with adequate amounts of a flavor and a color. The mixture was in a usual manner kneaded by a roll and then shaped and packed to obtain the desired product. The product was a relatively low cariogenic and caloric chewing gum having a satisfactory texture, taste, and flavor.

### Example B-4

### Powdery peptide

One part by weight of 40% of "HINUTE S", a peptide solution of edible soy beans commercialized by Fuji Oil Co., Ltd., Tokyo, Japan, was mixed with two parts by weight of a high isomaltose content syrup obtained by the method in Example A-4, and the resultant mixture was placed in a plastic vat, dried *in vacuo* at 50°C, and pulverized to obtain a powdery peptide. The product, having a satisfactory flavor and taste, can be arbitrary used as a material for low-caloric confectioneries such as premixes, sherbets and ice creams, as well as a material for controlling intestinal conditions, health food, and substantially non-digestible edible fibers used for fluid diets for oral administration and intubation feeding.

### Example B-5

### Bath salt

One part by weight of a peel juice of "*yuzu*" (a Chinese lemon) was mixed with 10 parts by weight of a high isomaltose content syrup obtained by the method in Example A-3, and one part by weight of cyclotetrasaccharide, and the mixture was pulverized into an isomaltose powder containing a peel juice of *yuzu*.

A bath salt was obtained by mixing five parts by weight of the above powder with 90 parts by weight of grilled salt, two parts by weight of crystalline trehalose hydrate, one part by weight of silicic anhydride, and 0.5 part by weight of "αG HESPERIDIN", α-glucosyl hesperidin commercialized by Hayashibara Shoji, Inc., Okayama, Japan.

The product is a high quality bath salt enriched with *yuzu* flavor and used by diluting in hot bath water by 100-10,000 folds, and it moisturizes and smooths the skin and does not make you feel cold after taking a bath therewith.

### Example B-6

### Cosmetic cream

Two parts by weight of polyoxyethylene glycol monostearate, five parts by weight of glyceryl monostearate, self-emulsifying, two parts by weight of a high isomaltose content syrup obtained by the method in Example A-2, one part by weight of "αG RUTIN", α-glucosyl rutin commercialized by Hayashibara Shoji, Inc., Okayama, Japan, one part by weight of liquid petrolatum, 10 parts by weight of glyceryl tri-2-ethylhexanoate, and an adequate amount of an antiseptic were dissolved by heating in a usual manner. The resulting solution was admixed with two parts by weight of L-lactic acid, five parts by weight of 1,3-butylene glycol, and 66 parts by weight of refined water, followed by emulsifying the mixture with a homogenizer and further admixing by stirring with an adequate amount of a flavor stirring to obtain a cosmetic cream. The product has an antioxidant activity and a relatively high stability, and these render it advantageously useful as a high quality sunscreen, skin-refining agent, and skin-whitening agent.

### Example B-7

### Toothpaste

A toothpaste was obtained by mixing 45 parts by weight of calcium secondary phosphate, 1.5 parts by weight of sodium lauryl sulfate, 25 parts by weight of glycerine, 0.5 part by weight of polyoxyethylene sorbitan laurate, 15 parts by weight of a high isomaltose content syrup obtained by the method in Example A-5, 0.02 part by weight of saccharine, 0.05 part by weight of an antiseptic, and 13 parts by weight of water. The product has an improved after taste and a satisfactory feeling after use without deteriorating the washing power of the surfactant.

### Example B-8

### Solid preparation for fluid diet

One hundred parts by weight of a high isomaltose content syrup obtained by the method in Example A-1, 200 parts by weight of crystalline trehalose hydrate, 200 parts by weight of high maltotetraose content powder, 270 parts by weight of an egg yolk powder, 209 parts by weight of a skim milk powder, 4.4 parts by weight of sodium chloride, 1.8 parts by weight of potassium chloride, four parts by weight of magnesium sulfate, 0.01 part by weight of thiamine, 0.1 part by weight of sodium L-ascorbate, 0.6 part by weight of vitamin E acetate, and 0.04 part by weight of nicotinamide were mixed. Twenty-five grams aliquots of the resulting composition were injected into moisture-proof laminated small bags which were then heat sealed to obtain the desired product.

The product is a fluid diet that has a satisfactory intestinal-controlling action. One bag of the product is dissolved in about 150-300 ml of water into a fluid diet and arbitrarily used by administering orally or intubationally into nasal cavity, stomach, intestines, etc., to supplement energy to living bodies.

### Example B-9

### Tablet

To 50 parts by weight of aspirin were sufficiently admixed with 14 parts by weight of a high isomaltose content syrup obtained by the method in Example A-2, and four parts by weight of corn starch. The resulting mixture was in a usual manner tabletted by a tabletting machine to obtain a tablet, 680 mg each, 5.25 mm in thickness.

The tablet, processed using the filler-imparting ability of isomaltose, has substantially no hygroscopicity, a sufficient physical strength and a quite satisfactory degradability in water.

### Example B-10

### Sugar coated tablet

A crude tablet as a core, 150 mg weight, was sugar coated with a first solution consisting of 40 parts by weight of a high isomaltose content syrup obtained by the method in Example A-1, two parts by weight of pullulan having an average molecular weight of 200,000, 30 parts by weight of water, 25 parts by weight of talc, and three parts by weight of titanium oxide until the total weight reached about 230 mg. The resultant was then sugar coated with a second solution consisting of 65 parts by weight of crystalline cyclotetrasaccharide, one part by weight of pullulan, and 34 parts by weight of water, and glossed with a liquid wax to obtain a sugar coated tablet having a satisfactory gloss and appearance. The product has a relatively high shock tolerance and retains its high quality for a relatively-long period of time.

### Example B-11

### Ointment for treating trauma

To 100 parts by weight of a high isomaltose content syrup obtained by the method in Example A-5 and 300 parts by weight of maltose was added 50 parts by weight of methanol dissolving three parts by weight of iodine. The resulting mixture was admixed with 200 parts by weight of a 10% (w/v) aqueous pullulan solution to obtain the captioned product with an adequate extensibility and adhesiveness. The product is a high-valued ointment in which the dispersion of iodine and methanol is well inhibited by isomaltose and which is relatively low in change during storage.

Because the product exerts a sterilizing action by iodine and acts, based on maltose, as an energy-supplementing agent to living cells, it shortens the curing term and well cures the affected parts and surfaces.

### INDUSTRIAL APPLICABILITY

As described above, the present invention relates to a novel process for producing isomaltose and uses thereof , more particularly, to a process for producing isomaltose characterized in that it comprises the steps of allowing α-isomaltosylglucosaccharide-forming enzyme, in the presence or the absence of α-isomaltosyl-transferring enzyme, to act on saccharides having both a glucose polymerization degree of at least two and α-1,4 glucosidic linkage as a linkage at the non-reducing end to form α-isomaltosylglucosaccharides, which have a glucose polymerization degree of at least three, α-1,6 glucosidic linkage as a linkage at the non-reducing end, and α-1,4 glucosidic linkage as a linkage other than the non-reducing end, and/or to form cyclo{→6)-α-D-glucopyranosyl-(1→3)-α-D-glucopyranosyl-(1→6)-α-D-glucopyranosyl-(1→3)-α-D-glucopyranosyl- (1→}; allowing isomaltose-releasing enzyme to act on the formed saccharides to release isomaltose; and collecting the released isomaltose; and relates to uses thereof. The isomaltose and high isomaltose content products of the present invention do not substantially crystallize and have useful properties of humectancy, low-sweetness, osmosis-controlling ability, filler-imparting ability, gloss-imparting ability, viscosity, crystallization-preventing ability for saccharides, insubstantial fermentability, retrogradation-preventing ability for gelatinized starches, etc. Thus, the isomaltose and high isomaltose content products can be arbitrarily used in foods, beverages, health foods, feeds, pet foods, cosmetics, pharmaceuticals, tobaccos, and cigarettes.

The present invention having these outstanding effects and functions is a significant invention that will greatly contribute to this art.

## Claims

1. A process for producing isomaltose, which comprises the steps of:
(a) allowing α-isomaltosylglucosaccharide-forming enzyme which forms α-isomaltosylglucosaccharide having a glucose polymerization degree of at least three, α-1,6 glucosidic linkage as a linkage at the non-reducing end, and α-1,4 glucosidic linkage as a linkage other than the non-reducing end; to act on a saccharide having, both a glucose polymerization degree of at least two and α-1,4 glucosidic linkage as a linkage at the non-reducing end to form said α-isomaltosylglucosaccharide;
(b) allowing isomaltodextranase to act on said α-isomaltosylglucosaccharide to release isomaltose; and
(c) collecting the released isomaltose,
wherein the step of allowing the α-isomaltosylglucosaccharide-forming enzyme to act on a saccharide is in the presence or absence of an α-isomaltosyl-transferring enzyme,
wherein the α-isomaltosylglucosaccharide-forming enzyme and the α-isomaltosyl-transferring enzyme are obtainable from Bacillus globisporus C9 (FERM BP-7143) or Bacillus globisporus C11 (FERM BP-7144).

2. The process of claim 1, wherein said α-isomaltosylglucosaccharide-forming enzyme has the following physicochemical properties:
(1) Action
Forming a saccharide, which has a glucose polymerization degree of at least three, α-1,6 glucosidic linkage as a linkage at the non-reducing end, and α-1,4 glucosidic linkage other than the linkage at the non-reducing end, by catalyzing the α-glucosyl-transferring reaction from a saccharide having both a glucose polymerization degree of at least two and having α-1,4 glucosidic linkage as a linkage at the non-reducing end without substantially increasing the reducing power;
(2) Molecular weight
Having a molecular weight of 117,000 to 160,000 daltons when determined on SDS-PAGE;
(3) Isoelectric point (pI)
Having an isoelectric point of 4.7 to 5.7 when determined on isoelectrophoresis using ampholine;
(4) Optimum temperature
Having an optimum temperature of 40°C to 45°C when incubated at a pH of 6.0 for 60 min, or an optimum temperature of 45°C to 50°C when incubated in the presence of 1 mM Ca²⁺;
(5) Optimum pH
Having an optimum pH of 6.0 to 6.5 when incubated at 35°C for 60 min;
(6) Thermal stability
Stable up to a temperature of 35°C to 40°C, or a temperature of about 40°C to 45°C in the presence of 1 mM Ca²⁺; and
(7) pH Stability
Stable at a pH of 4.5 to 10.0 when incubated at 4°C for 24 hours.

3. The process of claim 1, wherein said α-isomaltosyl-transferring enzyme has the following physicochemical properties:
(1) Action
Acting on an α-isomaltosylglucosaccharide, which has a glucose polymerization degree of at least three, α-1,6 glucosidic linkage as a linkage at the non-reducing end, and α-1,4 glucosidic linkage as a linkage other than the non-reducing end, to form cyclotetrasaccharide having a structure of cyclo{→6) - α-D-glucopyranosyl- (1→3) - α-D- glucopyranosyl-(1→6)-α-D-glucopyranosyl-(1→3) - α - D-glucopyranosyl- (1→} through α-isomaltosyl transfer;
(2) Molecular weight
Having a molecular weight of 82,000 to 132,000 daltons when determined on SDS-PAGE;
(3) Isoelectric point (pI)
Having an isoelectric point of 5.0 to 6.1 when determined on isoelectrophoresis using ampholine;
(4) Optimum temperature
Having an optimum temperature of 45°C to 50°C when incubated at a pH of 6.0 for 30 min;
(5) Optimum pH
Having an optimum pH of 5.5 to 6.0 when incubated at 35°C for 30 min;
(6) Thermal stability
Stable up to a temperature of 40°C when incubated at a pH of 6.0 for 60 min; and
(7) pH Stability
Stable at a pH of 4.0 to 9.0 when incubated at 4°C for 24 hours.

4. The process of claim 1, **characterized in that** one or more enzymes selected from the group consisting of cyclomaltodextrin glucanotransferase and starch debranching enzyme are used along with the α-isomaltosylglucosaccharide-forming enzyme in the step (a).

5. The process of claim 1, wherein said saccharide having both a glucose polymerization degree of at least two and α-1,4 glucosidic linkage as a linkage at the non-reducing end is one selected from the group consisting of maltooligosaccharides, maltodextrins, amylodextrins, amyloses, amylopectins, soluble starches, liquefied starches, and glycogens.

6. The process of claim 1, **characterized in that** a column chromatography using an alkaline metal and/or alkaline earth metal strong-acid cation exchange resin is used in step (c).

## Patentansprüche

1. Verfahren zur Herstellung von Isomaltose, wobei das Verfahren die Schritte umfasst:
(a) die Einwirkung von α-Isomaltosylglucosaccharid bildendem Enzym, welches α-Isomaltosylglucosaccharid bildet, das einen Glukose-Polymerisationsgrad von mindestens 3, eine α-1,6 glukosidische Bindung als Bindung am nicht-reduzierenden Ende und eine α-1,4 glukosidische Bindung als Bindung am vom nicht-reduzierenden Ende abweichenden Ende besitzt, auf ein Saccharid, das sowohl einen Glukose-Polymerisationsgrad von mindestens 2 als auch eine α-1,4 glukosidische Bindung als Bindung am nicht-reduzierenden Ende besitzt, um das besagte α-Isomaltosylglucosaccharid zu bilden;
(b) die Einwirkung von Isomaltodextranase auf das α-Isomaltosylglucosaccharid, um Isomaltose freizusetzen; und
(c) Auffangen der freigesetzten Isomaltose,
wobei der Schritt der Einwirkung des α-Isomaltosylglucosaccharid bildenden Enzyms in Gegenwart oder Abwesenheit eines α-Isomaltosyl-Transfer-Enzyms stattfindet,
wobei das α-Isomaltosylglucosaccharid bildende Enzym und das α-Isomaltosyl-Transfer-Enzym aus dem Bacillus globisporus C9 (FERM BP-7143) oder Bacillus globisporus C11 (FERM-7144) erhältlich sind.

2. Verfahren nach Anspruch 1, wobei das α-Isomaltosylglucosaccharid bildende Enzym die folgenden physikochemischen Eigenschaften aufweist:
(1) Reaktion
Es bildet eine Saccharid, das einen Glukose-Polymerisationsgrad von mindestens 3, eine α-1,6 glukosidische Bindung als Bindung am nicht-reduzierenden Ende und eine α-1,4 glukosidische Bindung als Bindung am vom nicht-reduzierenden Ende abweichenden Ende besitzt, durch Katalyse der α-Glucosyl-Transfer-Reaktion aus einem Saccharid, das sowohl einen Glukose-Polymerisationsgrad von mindestens 2 als auch eine α-1,4 glukosidische Bindung als Bindung am nicht-reduzierenden Ende besitzt, ohne die Reduktionskraft wesentlich zu erhöhen;
(2) Molekulargewicht
Es besitzt ein Molekulargewicht von 117,000 bis 160,000 Dalton bei einer Bestimmung über SDS-PAGE;
(3) Isoelektrischer Punkt (pI)
Es besitzt einen isoelektrischen Punkt von 4.7 bis 5.7 bei einer Bestimmung über Isoelektrophorese unter Einsatz von Ampholin;
(4) Optimale Temperatur
Es besitzt eine optimale Temperatur von 40 °C bis 45 °C bei einer Inkubation bei einem pH von 6.0 für 60 Minuten; oder eine optimale Temperatur von 45 °C bis 50 °C bei einer Inkubation in Gegenwart von 1 mM Ca²⁺;
(5) Optimaler pH
Es besitzt einen optimalen pH von 6.0 bis 6.5 bei einer Inkubation bei 35 °C für 60 Minuten;
(6) Thermische Stabilität
Es ist stabil bis zu einer Temperatur von 35 °C bis 40 °C; oder einer Temperatur von ca. 40 °C bis 45 °C in Gegenwart von 1 mM Ca2⁺; und
(7) pH-Stabilität
Es ist stabil bei einem pH von 4.5 bis 10.0 bei einer Inkubation bei 4 °C für 24 Stunden.

3. Verfahren nach Anspruch 1, wobei das das α-Isomaltosyl-Transfer-Enzym die folgenden physikochemischen
Eigenschaften aufweist:
(1) Reaktion
Bei der Einwirkung auf α-Isomaltosylglucosaccharid, das einen Glukose-Polymerisationsgrad von mindestens 3, eine α-1,6 glukosidische Bindung als Bindung am nicht-reduzierenden Ende und eine α-1,4 glukosidische Bindung als Bindung am vom nicht-reduzierenden Ende abweichenden Ende besitzt, bildet es durch α-Isomaltosyl-Transfer Cyclotetrasaccharid, das eine Struktur von {→6)-α-D-Glucopyranosyl-(1→3)-α-D-glucoppyranosyl-(1→} besitzt;
(2) Molekulargewicht
Es besitzt ein Molekulargewicht von 82,000 bis 132,000 Dalton bei einer Bestimmung über SDS-PAGE;
(3) Isoelektrischer Punkt (pI)
Es besitzt einen isoelektrischen Punkt von 5.0 bis 6.1 bei einer Bestimmung über Isoelektrophorese unter Einsatz von Ampholin;
(4) Optimale Temperatur
Es besitzt eine optimale Temperatur von 45 °C bis 50 °C bei einer Inkubation bei einem pH von 6.0 für 30 Minuten;
(5) Optimaler pH
Es besitzt einen optimalen pH von 5.5 bis 6.0 bei einer Inkubation bei 35 °C für 30 Minuten;
(6) Thermische Stabilität
Es ist stabil bis zu einer Temperatur von 40 °C bei einer Inkubation bei einem pH von 6.0 für 60 Minuten; und
(7) pH-Stabilität
Es ist stabil bei einem pH von 4.0 bis 9.0 bei einer Inkubation bei 4 °C für 24 Stunden.

4. Verfahren nach Anspruch 1, **dadurch gekennzeichnet, dass** eines oder mehrere Enzyme, ausgewählt aus der Gruppe bestehend aus Cyclomaltodextrin Glucanotransferase und Stärke abbauendes Enzym, zusammen mit dem α-Isomaltosylglucosaccharid bildenden Enzym in Schritt (a) eingesetzt werden.

5. Verfahren nach Anspruch 1, wobei das Saccharid, das sowohl einen Glukose-Polymerisationsgrad von mindestens 2 als auch eine α-1,4 glukosidische Bindung als Bindung am nicht-reduzierenden Ende besitzt, ein Saccharid ist, ausgewählt aus der Gruppe bestehend aus Maltooligosaccharide, Maltodextrine, Amylodextrine, Amylosen, Amylopektine, lösliche Stärken, verflüpssiogte Stärken und Glykogene.

6. Verfahren nach Anspruch 1, **dadurch gekennzeichnet, dass** eine Säulenchromatographie unter Einsatz von Alkalimetall und/oder Erdalkalimetall in Schritt (c) eingesetzt wird.

## Revendications

1. Procédé pour la production d'isomaltose, qui comprend les étapes consistant à :
(a) laisser une enzyme de formation d'α-isomaltosylglucosaccharide, qui forme de l'α-isomaltosylglucosaccharide ayant un degré de polymérisation du glucose d'au moins trois, une liaison glucosidique α-1,6 comme liaison à l'extrémité non réductrice et une liaison glucosidique α-1,4 comme liaison autre que celle l'extrémité non réductrice, agir sur un saccharide ayant à la fois un degré de polymérisation du glucose d'au moins 2 et une liaison glucosidique α-1,4 comme liaison à l'extrémité non réductrice pour former ledit α-isomaltosylglucosaccharide ;
(b) laisser de l'isomaltodextranase agir sur ledit α-isomaltosylglucosaccharide pour libérer l'isomaltose ; et
(c) recueillir l'isomaltose libéré,
dans lequel l'étape consistant à laisser l'enzyme de formation d'α-isomaltosylglucosaccharide agir sur un saccharide est mise en oeuvre en la présence ou en l'absence d'une enzyme de transfert d'α-isomaltosyle,
et dans lequel l'enzyme de formation d'α-isomaltosylglucosaccharide et l'enzyme de transfert d'α-isomaltosyle peuvent être obtenues à partir de Bacillus globisporus C9 (FERM BP-7143) ou de Bacillus globisporus C11 (FERM BP-7144).

2. Procédé suivant la revendication 1, dans lequel ladite enzyme de formation d'α-isomaltosylglucosaccharide a les propriétés physicochimiques suivantes :
(1) Action
Formation d'un saccharide, qui a un degré de polymérisation du glucose d'au moins trois, une liaison glucosidique α-1,6 comme liaison à l'extrémité non réductrice et une liaison glucosidique α-1,4 autre que la liaison à l'extrémité non réductrice, par catalyse de la réaction de transfert d'α-glucosyle à partir d'un saccharide ayant à la fois un degré de polymérisation du glucose d'au moins deux et une liaison glucosidique α-1,4 comme liaison à l'extrémité non réductrice sans augmenter substantiellement le pouvoir réducteur ;
(2) Poids moléculaire
Possession d'un poids moléculaire de 117 000 à 160 000 daltons lors de sa détermination par SDS-PAGE ;
(3) Poids isoélectrique (pI)
Possession d'un poids isoélectrique de 4,7 à 5,7 lors de sa détermination par isoélectrophorèse au moyen d'ampholine ;
(4) Température optimale
Possession d'une température optimale de 40°C à 45°C lors de la mise en incubation à un pH de 6,0 pendant 60 min, ou d'une température optimale de 45°C à 50°C lors de la mise en incubation en présence de Ca²⁺ 1 mM ;
(5) pH optimal
Possession d'un pH optimal de 6,0 à 6,5 lors de la mise en incubation à 35°C pendant 60 min ;
(6) Stabilité thermique
Stable jusqu'à une température de 35°C à 40°C, ou une température d'environ 40°C à 45°C en présence de Ca²⁺ 1 mM ; et
(7) Stabilité au pH
Stable à un pH de 4, 5 à 10,0 lors de la mise en incubation à 4°C pendant 24 heures.

3. Procédé suivant la revendication 1, dans lequel ladite enzyme de transfert d'α-isomaltosyle a les propriétés physicochimiques suivantes :
(1) Action
Action sur un α-isomaltosylglucosaccharide, qui a un degré de polymérisation du glucose d'au moins trois, une liaison glucosidique α-1,6 comme liaison à l'extrémité non réductrice et une liaison glucosidique α-1,4 comme liaison autre que celle à l'extrémité non réductrice, pour former un cyclotétrasaccharide ayant une structure cyclo-{→6)-α-D-glucopyrannosyl-(1→3)-α-D-glucopyrannosyl-(1→6)-α-D-glycopyrannosyl-(1→3)-α-D-glucopyrannosyl-(1→} par transfert d'α-isomaltosyle ;
(2) Poids moléculaire
Possession d'un poids moléculaire de 82 000 à 132 000 daltons lors de sa détermination par SDS-PAGE ;
(3) Poids isoélectrique (pI)
Possession d'un poids isoélectrique de 5,0 à 6,1 lors de sa détermination par isoélectrophorèse au moyen d'ampholine ;
(4) Température optimale
Possession d'une température optimale de 45°C à 50°C lors de la mise en incubation à un pH de 6,0 pendant 60 min ;
(5) pH optimal
Possession d'un pH optimal de 5,5 à 6,0 lors de la mise en incubation à 35°C pendant 30 min ;
(6) Stabilité thermique
Stable jusqu'à une température de 40°C, lors de la mise en incubation à un pH de 6,0 pendant 60 min ; et
(7) Stabilité au pH
Stable à un pH de 4,0 à 9,0 lors de la mise en incubation à 4°C pendant 24 heures.

4. Procédé suivant la revendication 1, **caractérisé en ce qu'**une ou plusieurs enzymes choisies dans le groupe consistant en la cyclomaltodextrine-glucanotransférase et une enzyme de déramification de l'amidon sont utilisées avec l'enzyme de formation d'α-isomaltosylglucosaccharide dans l'étape (a).

5. Procédé suivant la revendication 1, dans lequel ledit saccharide ayant à la fois un degré de polymérisation du glucose d'au moins deux et une liaison glucosidique α-1,4 comme liaison à l'extrémité non réductrice est un saccharide choisi dans le groupe consistant en des maltooligosaccharides, des maltodextrines, des amylodextrines, des amyloses, des amylopectines, des amidons solubles, des amidons liquéfiés et des glycogènes.

6. Procédé suivant la revendication 1, **caractérisé en ce qu'**une chromatographie sur colonne utilisant une résine échangeuse de cations du type acide fort avec métal alcalin et/ou métal alcalino-terreux est utilisée dans l'étape (c).


### SEQUENCE LISTING

<110> Kabushiki Kaisha Hayashibara Seibutsu Kagaku Kenkyujo
<120> Process for producing isomaltose and uses thereof
<130> W0899
<150> JP 130,922/01
   <151> 2001-4-27
<160> 2
<210> 1
   <211> 5234
   <212> DNA
   <213> Microorganism
<220>
   <221> CDS
   <222> (937)... (4785)
<400> 1 
atctaccggt ttttgtgaag tttggcagta ttcttccgat gaatttgaac gcgcaatatc 60
aagtgggcgg gaccattggc aacagcttga cgagctacac gaatctcgcg ttccgcattt 120
atccgcttgg gacaacaacg tacgactgga atgatgatat tggcggttcg gtgaaaacca 180
taacttctac agagcaatat gggttgaata aagaaaccgt gactgttcca gcgattaatt 240
ctaccaagac attgcaagtg tttacgacta agccttcctc tgtaacggtg ggtggttctg 360
tgatgacaga gtacagtact ttaactgccc taacgggagc gtcgacaggc tggtactatg 420
atactgtaca gaaattcact tacgtcaagc ttggttcaag tgcatctgct caatccgttg 480
tgctaaatgg cgttaataag gtggaatatg aagcagaatt cggcgtgcaa agcggcgttt 540
caacgaacac gaaccatgca ggttatactg gtacaggatt tgtggacggc tttgagactc 600
ttggagacaa tgttgctttt gatgtttccg tcaaagccgc aggtacttat acgatgaagg 660
ttcggtattc atccggtgca ggcaatggct caagagccat ctatgtgaat aacaccaaag 720
tgacggacct tgccttgccg caaacaacaa gctgggatac atgggggact gctacgttta 780
gcgtctcgct gagtacaggt ctcaacacgg tgaaagtcag ctatgatggt accagttcac 840
 
ttggcattaa tttcgataac atcgcgattg tagagcaata aaaggtcggg agggcaagtc 900
cctcccttaa tttctaatcg aaagggagta tccttg 936
atg cgt cca cca aac aaa gaa att cca cgt att ctt gct ttt ttt aca 978
Met Arg Pro Pro Asn Lys Glu Ile Pro Arg Ile Leu Ala Phe Phe Thr
1               5                   10                  15
gcg ttt acg ttg ttt ggt tca acc ctt gcc ttg ctt cct gct ccg cct 1026
Ala Phe Thr Leu Phe Gly Ser Thr Leu Ala Leu Leu Pro Ala Pro Pro
            20                  25                  30
gcg cat gcc tat gtc agc agc cta gga aat ctc att tct tcg agt gtc 1074
Ala His Ala Tyr Val Ser Ser Leu Gly Asn Leu Ile Ser Ser Ser Val
        35                  40                  45
acc gga gat acc ttg acg cta act gtt gat aac ggt gcg gag ccg agt 1122
Thr Gly Asp Thr Leu Thr Leu Thr Val Asp Asn Gly Ala Glu Pro Ser
    50                  55                  60
gat gac ctc ttg att gtt caa gcg gtg caa aac ggt att ttg aag gtg 1170
Asp Asp Leu Leu Ile Val Gln Ala Val Gln Asn Gly Ile Leu Lys Val
65                  70                  75                  80
gat tat cgt cca aat agc ata acg ccg agc gcg aag acg ccg atg ctg 1218
Asp Tyr Arg Pro Asn Ser Ile Thr Pro Ser Ala Lys Thr Pro Met Leu
                85                  90                  95
gat ccg aac aaa act tgg tca gct gta gga gct acg att aat acg aca 1266
Asp Pro Asn Lys Thr Trp Ser Ala Val Gly Ala Thr Ile Asn Thr Thr
            100                 105                 110
gcc aat cca atg acc atc acg act tcc aat atg aag att gag att acc 1314
Ala Asn Pro Met Thr Ile Thr Thr Ser Asn Met Lys Ile Glu Ile Thr
        115                 120                 125
aag aat cca gta cga atg acg gtc aag aag gcg gac ggc act acg cta 1362
Lys Asn Pro Val Arg Met Thr Val Lys Lys Ala Asp Gly Thr Thr Leu
    130                 135                 140
ttc tgg gaa cca tca ggc gga ggg gta ttc tca gac ggt gtg cgc ttc 1410
Phe Trp Glu Pro Ser Gly Gly Gly Val Phe Ser Asp Gly Val Arg Phe
145                 150                 155                 160
ctt cat gcc aca ggg gat aat atg tat ggc atc cgg agc ttc aat gct 1458
Leu His Ala Thr Gly Asp Asn Met Tyr Gly Ile Arg Ser Phe Asn Ala
                165                 170                 175
ttt gat agc ggg ggt gac ctg ctg cgg aat tcg tcc aat cat gcc gcc 1506
 
Phe Asp Ser Gly Gly Asp Leu Leu Arg Asn Ser Ser Asn His Ala Ala
            180                 185                 190
cat gcg ggt gaa cag gga gat tcc ggt ggt ccg ctt att tgg agt acg 1554
His Ala Gly Glu Gln Gly Asp Ser Gly Gly Pro Leu Ile Trp Ser Thr
        195                 200                 205
gca gga tat gga cta tta gtc gat agc gat ggc ggc tac ccc tat aca 1602
Ala Gly Tyr Gly Leu Leu Val Asp Ser Asp Gly Gly Tyr Pro Tyr Thr
210 21 220
gat agc aca acc ggt caa atg gag ttt tat tat ggt ggg acc cct cct 1650
Asp Ser Thr Thr Gly Gln Met Glu Phe Tyr Tyr Gly Gly Thr Pro Pro
225                 230                 235                 240
gag gga cgt cgt tat gcg aaa caa aac gtg gaa tat tat att atg ctc 1698
Glu Gly Arg Arg Tyr Ala Lys Gln Asn Val Glu Tyr Tyr Ile Met Leu
                245                 250                 255
gga acc ccc aag gaa att atg acc gac gta ggg gaa atc aca ggg aaa 1746
Gly Thr Pro Lys Glu Ile Met Thr Asp Val Gly Glu Ile Thr Gly Lys
            260                 265                 270
ccg cct atg ctg cct aag tgg tcg ctt gga ttc atg aac ttt gag tgg 1794
Pro Pro Met Leu Pro Lys Trp Ser Leu Gly Phe Met Asn Phe Glu Trp
        275                 280                 285
gat acg aat caa acg gag ttt acg aat aat gtg gat acg tat cgt gcc 1842
Asp Thr Asn Gln Thr Glu Phe Thr Asn Asn Val Asp Thr Tyr Arg Ala
    290                 295                 300
aaa aat atc ccc ata gat gct tac gcc ttc gac tat gac tgg aaa aag 1890
Lys Asn Ile Pro Ile Asp Ala Tyr Ala Phe Asp Tyr Asp Trp Lys Lys
305                 310                 315                 320
tac ggg gaa acc aac tat ggt gaa ttc gcg tgg aat acg act aat ttc 1938
Tyr Gly Glu Thr Asn Tyr Gly Glu Phe Ala Trp Asn Thr Thr Asn Phe
                325                 330                 335
cct tct gcg tca acg act tct tta aag tca aca atg gat gct aaa ggc 1986
Pro Ser Ala Ser Thr Thr Ser Leu Lys Ser Thr Met Asp Ala Lys Gly
            340                 345                 350
atc aaa atg atc gga att aca aaa ccc cgc atc gtt acg aag gat gct 2034
Ile Lys Met Ile Gly Ile Thr Lys Pro Arg Ile Val Thr Lys Asp Ala
        355                 360                 365
tca gcg aat gtg acg acc caa ggg acg gac gcg aca aat ggc ggt tat 2082
 
Ser Ala Asn Val Thr Thr Gln Gly Thr Asp Ala Thr Asn Gly Gly Tyr
    370                 375                 380
ttt tat cca ggc cat aac gag tat cag gat tat ttc att ccc gta act    2130
Phe Tyr Pro Gly His Asn Glu Tyr Gln Asp Tyr Phe Ile Pro Val Thr
385                 390                 395                 400
gtg cgt agt atc gat cct tac aat gct aac gaa cgt gct tgg ttc tgg    2178
Val Arg Ser Ile Asp Pro Tyr Asn Ala Asn Glu Arg Ala Trp Phe Trp
                405                 410                 415
aat cat tcc aca gat gcg ctt aat aaa ggg atc gta ggt tgg tgg aat    2226
Asn His Ser Thr Asp Ala Leu Asn Lys Gly Ile Val Gly Trp Trp Asn
            420                 425                 430
gac gag acg gat aaa gta tct tcg ggt gga gcg tta tat tgg ttt ggc    2274
Asp Glu Thr Asp Lys Val Ser Ser Gly Gly Ala Leu Tyr Trp Phe Gly
        435                 440                 445
aat ttc aca aca ggc cac atg tct cag acg atg tac gaa ggg ggg cgg    2322
Asn Phe Thr Thr Gly His Met Ser Gln Thr Met Tyr Glu Gly Gly Arg
    450                 455                 460
gct tac acg agt gga gcg cag cgt gtt tgg caa acg gct aga acc ttc    2370
Ala Tyr Thr Ser Gly Ala Gln Arg Val Trp Gln Thr Ala Arg Thr Phe
465                 470                 475                 480
tac cca ggt gcc cag cgg tat gcg act acg ctt tgg tct ggc gat att    2418
Tyr Pro Gly Ala Gln Arg Tyr Ala Thr Thr Leu Trp Ser Gly Asp Ile
                485                 490                 495
ggc att caa tac aat aaa ggc gaa cgg atc aat tgg gct gcc ggg atg    2466
Gly Ile Gln Tyr Asn Lys Gly Glu Arg Ile Asn Trp Ala Ala Gly Met
            500                 505                 510
cag gag caa agg gca gtt atg cta tcc tcc gtg aac aat ggc cag gtg    2514
Gln Glu Gln Arg Ala Val Met Leu Ser Ser Val Asn Asn Gly Gln Val
        515                 520                 525
aaa tgg ggc atg gat acc ggc gga ttc aat cag cag gat ggc acg acg    2562
Lys Trp Gly Met Asp Thr Gly Gly Phe Asn Gln Gln Asp Gly Thr Thr
    530                 535                 540
aac aat ccg aat ccc gat tta tac gct cgg tgg atg cag ttc agt gcc    2610
Asn Asn Pro Asn Pro Asp Leu Tyr Ala Arg Trp Met Gln Phe Ser Ala
545                 550                 555                 560
cta acg cct gtt ttc cga gtg cat ggg aac aac cat cag cag cgc cag    2658
 
Leu Thr Pro Val Phe Arg Val His Gly Asn Asn His Gln Gln Arg Gln
                565                 570                 575
cca tgg tac ttc gga tcg act gcg gag gag gcc tcc aaa gag gca att      2706
Pro Trp Tyr Phe Gly Ser Thr Ala Glu Glu Ala Ser Lys Glu Ala Ile
            580                 585                 590
cag ctg cgg tac tcc ctg atc cct tat atg tat gcc tat gag aga agt      2754
Gln Leu Arg Tyr Ser Leu Ile Pro Tyr Met Tyr Ala Tyr Glu Arg Ser
        595                 600                 605
gct tac gag aat ggg aat ggg ctc gtt cgg cca ttg atg caa gcc tat      2802
Ala Tyr Glu Asn Gly Asn Gly Leu Val Arg Pro Leu Met Gln Ala Tyr
    610                 615                 620
cca aca gat gcg gcc gtc aaa aat tac acg gat gct tgg atg ttt ggt      2850
Pro Thr Asp Ala Ala Val Lys Asn Tyr Thr Asp Ala Trp Met Phe Gly
625                 630                 635                 640
gac tgg ctg ctg gct gca cct gtg gta gat aaa cag cag acg agt aag      2898
Asp Trp Leu Leu Ala Ala Pro Val Val Asp Lys Gln Gln Thr Ser Lys
                645                 650                 655
gat atc tat tta ccg tct ggg tca tgg att gac tat gcg cga ggc aat      2946
Asp Ile Tyr Leu Pro Ser Gly Ser Trp Ile Asp Tyr Ala Arg Gly Asn
            660                 665                 670
gca ata act ggc ggt caa acc atc cga tat tcg gtt aat ccg gac acg      2994
Ala Ile Thr Gly Gly Gln Thr Ile Arg Tyr Ser Val Asn Pro Asp Thr
        675                 680                 685
ttg aca gac atg cct ctc ttt att aaa aaa ggt gcc att att cca aca      3042
Leu Thr Asp Met Pro Leu Phe Ile Lys Lys Gly Ala Ile Ile Pro Thr
    690                 695                 700
cag aaa gtg cag gat tac gta ggg cag gct tcc gtc act tcc gtt gat      3090
Gln Lys Val Gln Asp Tyr Val Gly Gln Ala Ser Val Thr Ser Val Asp
705                 710                 715                 720
gtg gat gtg ttt ccg gat acg acg cag tcg agt ttc acg tac tac gat      3138
Val Asp Val Phe Pro Asp Thr Thr Gln Ser Ser Phe Thr Tyr Tyr Asp
                725                 730                 735
gat gat ggc gcc agt tat aac tat gag agc ggc act tat ttt aag caa      3186
Asp Asp Gly Ala Ser Tyr Asn Tyr Glu Ser Gly Thr Tyr Phe Lys Gln
            740                 745                 750
aat atg act gct cag gat aat ggg tca ggc tcg tta agt ttt act tta      3234
 
Asn Met Thr Ala Gln Asp Asn Gly Ser Gly Ser Leu Ser Phe Thr Leu
        755                 760                 765
gga gca aag agt ggc agt tac acg ccg gct ctc caa tcc tat atc gtt 3282
Gly Ala Lys Ser Gly Ser Tyr Thr Pro Ala Leu Gln Ser Tyr Ile Val
    770                 775                 780
aag ctg cac ggt tct gct gga act tct gtt acg aat aac agc gca gct 3330
Lys Leu His Gly Ser Ala Gly Thr Ser Val Thr Asn Asn Ser Ala Ala
785                 790                 795                 800
atg aca tct tat gca agc ttg gaa gca tta aaa gct gct gct ggg gaa 3378
Met Thr Ser Tyr Ala Ser Leu Glu Ala Leu Lys Ala Ala Ala Gly Glu
                805                 810                 815
ggc tgg gcg act ggg aag gac att tat ggg gat gtc acc tat gtg aaa 3428
Gly Trp Ala Thr Gly Lys Asp Ile Tyr Gly Asp Val Thr Tyr Val Lys
            820                 825                 830
gtg acg gca ggt aca gct tct tct aaa tct att gct gtt aca ggt gtt 3474
Val Thr Ala Gly Thr Ala Ser Ser Lys Ser Ile Ala Val Thr Gly Val
        835                 840                 845
gct gcc gtg agc gca act act tcg caa tac gaa gct gag gat gca tcg 3522
Ala Ala Val Ser Ala Thr Thr Ser Gln Tyr Glu Ala Glu Asp Ala Ser
    850                 855                 860
ctt tct ggc aat tcg gtt gct gca aag gcg tcc ata aac acg aat cat 3570
Leu Ser Gly Asn Ser Val Ala Ala Lys Ala Ser Ile Asn Thr Asn His
865                 870                 875                 880
acc gga tat acg gga act gga ttt gta gat ggt ttg ggg aat gat ggc 3618
Thr Gly Tyr Thr Gly Thr Gly Phe Val Asp Gly Leu Gly Asn Asp Gly
                885                 890                 895
gct ggt gtc acc ttc tat cca aag gtg aaa act ggc ggt gac tac aat 3666
Ala Gly Val Thr Phe Tyr Pro Lys Val Lys Thr Gly Gly Asp Tyr Asn
            900                 905                 910
gtc tcc ttg cgt tat gcg aat gct tca ggc acg gct aag tca gtc agt 3714
Val Ser Leu Arg Tyr Ala Asn Ala Ser Gly Thr Ala Lys Ser Val Ser
        915                 920                 925
att ttt gtt aat gga aaa aga gtg aag tcc acc tcg ctc gct aat ctc 3762
Ile Phe Val Asn Gly Lys Arg Val Lys Ser Thr Ser Leu Ala Asn Leu
    930                 935                 940
gca aat tgg gac act tgg tct aca caa tct gag aca ctg ccg ttg acg 3810
 
Ala Asn Trp Asp Thr Trp Ser Thr Gln Ser Glu Thr Leu Pro Leu Thr
945                 950                 955                 960
gca ggt gtg aat gtt gtg acc tat aaa tat tac tcc gat gcg gga gat    3858
Ala Gly Val Asn Val Val Thr Tyr Lys Tyr Tyr Ser Asp Ala Gly Asp
                965                 970                 975
aca ggc aat gtt aac atc gac aac atc acg gta cct ttt gcg cca att    3906
Thr Gly Asn Val Asn Ile Asp Asn Ile Thr Val Pro Phe Ala Pro Ile
            980                 985                 990
atc ggt aag tat gaa gca gag agt gct gag ctt tct ggt ggc agc tca    3954
Ile Gly Lys Tyr Glu Ala Glu Ser Ala Glu Leu Ser Gly Gly Ser Ser
        995                 1000                1005
ttg aac acg aac cat tgg tac tac agt ggt acg gct ttt gta gac ggt    4002
Leu Asn Thr Asn His Trp Tyr Tyr Ser Gly Thr Ala Phe Val Asp Gly
    1010                1015                1020
ttg agt gct gta ggc gcg cag gtg aaa tac aac gtg aat gtc cct agc    4050
Leu Ser Ala Val Gly Ala Gln Val Lys Tyr Asn Val Asn Val Pro Ser
1025                1030                1035                1040
gca gga agt tat cag gta gcg ctg cga tat gcg aat ggc agt gca gcg    4098
Ala Gly Ser Tyr Gln Val Ala Leu Arg Tyr Ala Asn Gly Ser Ala Ala
                1045                1050                1055
acg aaa acg ttg agt act tat atc aat gga gcc aag ctg ggg caa acc    4146
Thr Lys Thr Leu Ser Thr Tyr Ile Asn Gly Ala Lys Leu Gly Gln Thr
            1060                1065                1070
agt ttt acg agt cct ggt acg aat tgg aat gtt tgg cag gat aat gtg    4194
Ser Phe Thr Ser Pro Gly Thr Asn Trp Asn Val Trp Gln Asp Asn Val
        1075                1080                1085
caa acg gtg acg tta aat gca ggg gca aac acg att gcg ttt aaa tac    4242
Gln Thr Val Thr Leu Asn Ala Gly Ala Asn Thr Ile Ala Phe Lys Tyr
    1090                1095                1100
gac gcc gct gac agc ggg aac atc aac gta gat cgt ctg ctt ctt tca    4290
Asp Ala Ala Asp Ser Gly Asn Ile Asn Val Asp Arg Leu Leu Leu Ser
1105                1110                1115                1120
act tcg gca gcg gga acg ccg gtt tct gag cag aac ctg cta gac aat    4338
Thr Ser Ala Ala Gly Thr Pro Val Ser Glu Gln Asn Leu Leu Asp Asn
                1125                1130                1135
ccc ggt ttc gag cgt gac acg agt caa acc aat aac tgg att gag tgg    4386
 
Pro Gly Phe Glu Arg Asp Thr Ser Gln Thr Asn Asn Trp Ile Glu Trp
            1140                1145                1150
cat cca ggc acg caa gct gtt gct ttt ggc gtt gat agc ggc tca acc 4434
His Pro Gly Thr Gln Ala Val Ala Phe Gly Val Asp Ser Gly Ser Thr
        1155                1160                1165
acc aat ccg ccg gaa tcc ccg tgg tcg ggt gat aag cgt gcc tac ttc 4482
Thr Asn Pro Pro Glu Ser Pro Trp Ser Gly Asp Lys Arg Ala Tyr Phe
    1170                1175                1180
ttt gca gca ggt gcc tat caa caa agc atc cat caa acc att agt gtt 4530
Phe Ala Ala Gly Ala Tyr Gln Gln Ser Ile His Gln Thr Ile Ser Val
1185                1190                1195                1200
cct gtt aat aat gta aaa tac aaa ttt gaa gcc tgg gtc cgc atg aag 4578
Pro Val Asn Asn Val Lys Tyr Lys Phe Glu Ala Trp Val Arg Met Lys
                1205                1210                1215
aat acg acg ccg acg acg gca aga gcc gaa att caa aac tat ggc gga 4626
Asn Thr Thr Pro Thr Thr Ala Arg Ala Glu Ile Gln Asn Tyr Gly Gly
            1220                1225                1230
tca gcc att tat gcg aac ata agt aac agc ggt gtt tgg aaa tat atc 4674
Ser Ala Ile Tyr Ala Asn Ile Ser Asn Ser Gly Val Trp Lys Tyr Ile
        1235                1240                1245
agc gta agt gat att atg gtg acc aat ggt cag ata gat gtt gga ttt 4722
Ser Val Ser Asp Ile Met Val Thr Asn Gly Gln Ile Asp Val Gly Phe
    1250                1255                1260
tac gtg gat tca cct ggt gga act acg ctt cac att gat gat gtg cgc 4770
Tyr Val Asp Ser Pro Gly Gly Thr Thr Leu His Ile Asp Asp Val Arg
1265                1270                1275                1280
gta acc aaa caa taa 4785
Val Thr Lys Gln
acaaacaacc agctctcccg ttaatgggag ggctggttgt ttgttatgat aatccatcta 4845
tttagagtgg attaaacgtt ttgaagtgct tgctgaactt cttgcacaat ggataacgcc 4905
gcggtgcggg cacttgagaa agcacgttct gcaagctctc ccttacctgt acagccgtct 4965
ccgcagaagt agaaaggaac gttttccacg cgtatcggca gcagattatt ggaagcaatg 5025
tttttcacgc tggaaaccat cgctttcttg gaaacccgtt tcacggctgt gacatcgcgc 5085
cagcctggat aatgtttatc aaataaggct tccatttgga ggttcttctc ttccaggtac 5145
gctttgcgct gctcctcgtt atcaaagcgg tcgcttaagt atgcgatacc ttgcagcagc 5205
tgcccgcctt ctggtactag tgtgtgatc 5234
<210> 2
   <211> 3869
   <212> DNA
   <213> Microorganism
<220>
   <221> CDS
   <222> (241)...(3522)
<400> 2

tcatcgctac tggcaatcgg attcaaacaa atggctgcag ctcgcacaga cgattgtgga 60
aagggaatat ctgatttaac catacggcgg tcgcgattga ttgaatagga ttcgtggccg 120
cctaatattg aaagggggga tgcgtggagc agcgcatgca cggcgaggaa taactgttgt 180
tggagcctct aagtcattca tgtttagcaa acaaatttcg gtacgaaagg ggaaatgttt 240
atg tat gta agg aat cta aca ggt tca ttc cga ttt tct ctc tct ttt 288
Met Tyr Val Arg Asn Leu Thr Gly Ser Phe Arg Phe Ser Leu Ser Phe
1               5                   10                  15
ttg ctc tgt ttc tgt ctc ttc gtc ccc tct att tat gcc att gat ggt 336
Leu Leu Cys Phe Cys Leu Phe Val Pro Ser Ile Tyr Ala Ile Asp Gly
            20                  25                  30
gtt tat cat gcg cca tac gga atc gat gat ctg tac gag att cag gcg 384
Val Tyr His Ala Pro Tyr Gly Ile Asp Asp Leu Tyr Glu Ile Gln Ala
        35                  40                  45
acg gag cgg agt cca aga gat ccc gtt gca ggc gat act gtg tat atc 432
Thr Glu Arg Ser Pro Arg Asp Pro Val Ala Gly Asp Thr Val Tyr Ile
    50                  55                  60
aag ata aca acg tgg ccc att gaa tca gga caa acg gct tgg gtg acc 480
Lys Ile Thr Thr Trp Pro Ile Glu Ser Gly Gln Thr Ala Trp Val Thr
65                  70                  75                  80
tgg acg aaa aac ggt gtc aat caa gct gct gtc gga gca gca ttc aaa 528
Trp Thr Lys Asn Gly Val Asn Gln Ala Ala Val Gly Ala Ala Phe Lys
                85                  90                  95
tac aac agc ggc aac aac act tac tgg gaa gcg aac ctt ggc act ttt 576
Tyr Asn Ser Gly Asn Asn Thr Tyr Trp Glu Ala Asn Leu Gly Thr Phe
            100                 105                 110

gca aaa ggg gac gtg atc agt tat acc gtt cat ggc aac aag gat ggc 624
Ala Lys Gly Asp Val Ile Ser Tyr Thr Val His Gly Asn Lys Asp Gly
        115                 120                 125
gcg aat gag aag gtt atc ggt cct ttt act ttt acc gta acg gga tgg 672
Ala Asn Glu Lys Val Ile Gly Pro Phe Thr Phe Thr Val Thr Gly Trp
    130                 135                 140
gaa tcc gtt agc agt atc agc tct att acg gat aat acg aac cgt gtt 720
Glu Ser Val Ser Ser Ile Ser Ser Ile Thr Asp Asn Thr Asn Arg Val
145                 150                 155                 160
gtg ctg aat gcg gtg ccg aat aca ggc aca ttg aag cca aag atc aac 768
Val Leu Asn Ala Val Pro Asn Thr Gly Thr Leu Lys Pro Lys Ile Asn
                165                 170                 175
ctt tcc ttt acg gcg gat gat gtc ctc cgc gta cag gtt tct cca acc 816
Leu Ser Phe Thr Ala Asp Asp Val Leu Arg Val Gln Val Ser Pro Thr
            180                 185                 190
gga aca gga acg tta agc agt gga ctt agt aat tac aca gtt tca gat 864
Gly Thr Gly Thr Leu Ser Ser Gly Leu Ser Asn Tyr Thr Val Ser Asp
        195                 200                 205
acc gcc tca acc act tgg ctt aca act tcc aag ctg aag gtg aag gtg 912
Thr Ala Ser Thr Thr Trp Leu Thr Thr Ser Lys Leu Lys Val Lys Val
    210                 215                 220
gat aag aat cca ttc aaa ctt agt gtg tat aag cct gat gga acg acg 960
Asp Lys Asn Pro Phe Lys Leu Ser Val Tyr Lys Pro Asp Gly Thr Thr
225                 230                 235                 240
ttg att gcc cgt caa tat gac agc act acg aat cgt aac att gcc tgg 1008
Leu Ile Ala Arg Gln Tyr Asp Ser Thr Thr Asn Arg Asn Ile Ala Trp
                245                 250                 255
tta acc aat ggc agt aca atc atc gac aag gta gaa gat cat ttt tat 1056
Leu Thr Asn Gly Ser Thr Ile Ile Asp Lys Val Glu Asp His Phe Tyr
            260                 265                 270
tca ccg gct tcc gag gag ttt ttt ggc ttt gga gag cat tac aac aac 1104
Ser Pro Ala Ser Glu Glu Phe Phe Gly Phe Gly Glu His Tyr Asn Asn
        275                 280                 285
ttc cgt aaa cgc gga aat gat gtg gac acc tat gtg ttc aac cag tat 1152
Phe Arg Lys Arg Gly Asn Asp Val Asp Thr Tyr Val Phe Asn Gln Tyr
    290                 295                 300

aag aat caa aat gac cgc acc tac atg gca att cct ttt atg ctt aac 1200
Lys Asn Gln Asn Asp Arg Thr Tyr Met Ala Ile Pro Phe Met Leu Asn
305                 310                 315                 320
agc agc ggt tat ggc att ttc gta aat tca acg tat tat tcc aaa ttt 1248
Ser Ser Gly Tyr Gly Ile Phe Val Asn Ser Thr Tyr Tyr Ser Lys Phe
                325                 330                 335
cgg ttg gca acc gaa cgc acc gat atg ttc agc ttt acg gct gat aca 1296
Arg Leu Ala Thr Glu Arg Thr Asp Met Phe Ser Phe Thr Ala Asp Thr
            340                 345                 350
ggg ggt agt gcc gcc tcg atg ctg gat tat tat ttc att tac ggt aat 1344
Gly Gly Ser Ala Ala Ser Met Leu Asp Tyr Tyr Phe Ile Tyr Gly Asn
        355                 360                 365
gat ttg aaa aat gtg gtg agt aac tac gct aac att acc ggt aag cca 1392
Asp Leu Lys Asn Val Val Ser Asn Tyr Ala Asn Ile Thr Gly Lys Pro
    370                 375                 380
aca gcg ctg ccg aaa tgg gct ttc ggg tta tgg atg tca gct aac gag 1440
Thr Ala Leu Pro Lys Trp Ala Phe Gly Leu Trp Met Ser Ala Asn Glu
385                 390                 395                 400
tgg gat cgt caa acc aag gtg aat aca gcc att aat aac gcg aac tcc 1488
Trp Asp Arg Gln Thr Lys Val Asn Thr Ala Ile Asn Asn Ala Asn Ser
                405                 410                 415
aat aat att ccg gct aca gcg gtt gtg ctc gaa cag tgg agt gat gag 1536
Asn Asn Ile Pro Ala Thr Ala Val Val Leu Glu Gln Trp Ser Asp Glu
            420                 425                 430
aac acg ttt tat att ttc aat gat gcc acc tat acc ccg aaa acg ggc 1584
Asn Thr Phe Tyr Ile Phe Asn Asp Ala Thr Tyr Thr Pro Lys Thr Gly
        435                 440                 445
agt gct gcg cat gcc tat acc gat ttc act ttc ccg aca tct ggg aga 1632
Ser Ala Ala His Ala Tyr Thr Asp Phe Thr Phe Pro Thr Ser Gly Arg
    450                 455                 460
tgg acg gat cca aaa gcg atg gca gac aat gtg cat aac aat ggg atg 1680
Trp Thr Asp Pro Lys Ala Met Ala Asp Asn Val His Asn Asn Gly Met
465                 470                 475                 480
aag ctg gtg ctt tgg cag gtc cct att cag aaa tgg act tca acg ccc 1728
Lys Leu Val Leu Trp Gln Val Pro Ile Gln Lys Trp Thr Ser Thr Pro
                485                 490                 495

tat acc cag aaa gat aat gat gaa gcc tat atg acg gct cag aat tat 1776
Tyr Thr Gln Lys Asp Asn Asp Glu Ala Tyr Met Thr Ala Gln Asn Tyr
            500                 505                 510
gca gtt ggc aac ggt agc gga ggc cag tac agg ata cct tca gga caa 1824
Ala Val Gly Asn Gly Ser Gly Gly Gln Tyr Arg Ile Pro Ser Gly Gln
        515                 520                 525
tgg ttc gag aac agt ttg ctg ctt gat ttt acg aat acg gcc gcc aaa 1872
Trp Phe Glu Asn Ser Leu Leu Leu Asp Phe Thr Asn Thr Ala Ala Lys
    530                 535                 540
aac tgg tgg atg tct aaa cgc gct tat ctg ttt gat ggt gtg ggt atc 1920
Asn Trp Trp Met Ser Lys Arg Ala Tyr Leu Phe Asp Gly Val Gly Ile
545                 550                 555                 560
gac ggc ttc aaa aca gat ggc ggt gaa atg gta tgg ggt cgc tca aat 1968
Asp Gly Phe Lys Thr Asp Gly Gly Glu Met Val Trp Gly Arg Ser Asn
                565                 570                 575
act ttc tca aac ggt aag aaa ggc aat gaa atg cgc aat caa tac ccg 2016
Thr Phe Ser Asn Gly Lys Lys Gly Asn Glu Met Arg Asn Gln Tyr Pro
            580                 585                 590
aat gag tat gtg aaa gcc tat aac gag tac gcg cgc tcg aag aaa gcc 2064
Asn Glu Tyr Val Lys Ala Tyr Asn Glu Tyr Ala Arg Ser Lys Lys Ala
        595                 600                 605
gat gcg gtc tcc ttt agc cgt tcc ggc acg caa ggc gca cag gcg aat 2112
Asp Ala Val Ser Phe Ser Arg Ser Gly Thr Gln Gly Ala Gln Ala Asn
    610                 615                 620
cag att ttc tgg tcc ggt gac caa gag tcg acg ttt ggt gct ttt caa 2160
Gln Ile Phe Trp Ser Gly Asp Gln Glu Ser Thr Phe Gly Ala Phe Gln
625                 630                 635                 640
caa gct gtg aat gca ggg ctt acg gca agt atg tct ggc gtt cct tat 2208
Gln Ala Val Asn Ala Gly Leu Thr Ala Ser Met Ser Gly Val Pro Tyr
                645                 650                 655
tgg agc tgg gat atg gca ggc ttt aca ggc act tat cca acg gct gag 2256
Trp Ser Trp Asp Met Ala Gly Phe Thr Gly Thr Tyr Pro Thr Ala Glu
            660                 665                 670
ttg tac aaa cgt gct act gaa atg gct gct ttt gca ccg gtc atg cag 2304
Leu Tyr Lys Arg Ala Thr Glu Met Ala Ala Phe Ala Pro Val Met Gln
        675                 680                 685

ttt cat tcc gag tct aac ggc agc tct ggt atc aac gag gaa cgt tct 2352
Phe His Ser Glu Ser Asn Gly Ser Ser Gly Ile Asn Glu Glu Arg Ser
    690                 695                 700
cca tgg aac gca caa gcg cgt aca ggc gac aat acg atc att agt cat 2400
Pro Trp Asn Ala Gln Ala Arg Thr Gly Asp Asn Thr Ile Ile Ser His
705                 710                 715                 720
ttt gcc aaa tat acg aat acg cgc atg aat ttg ctt cct tat att tat 2448
Phe Ala Lys Tyr Thr Asn Thr Arg Met Asn Leu Leu Pro Tyr Ile Tyr
                725                 730                 735
agc gaa gcg aag atg gct agt gat act ggc gtt ccc atg atg cgc gcc 2496
Ser Glu Ala Lys Met Ala Ser Asp Thr Gly Val Pro Met Met Arg Ala
            740                 745                 750
atg gcg ctt gaa tat ccg aag gac acg aac acg tac ggt ttg aca caa 2544
Met Ala Leu Glu Tyr Pro Lys Asp Thr Asn Thr Tyr Gly Leu Thr Gln
        755                 760                 765
cag tat atg ttc gga ggt aat tta ctt att gct cct gtt atg aat cag 2592
Gln Tyr Met Phe Gly Gly Asn Leu Leu Ile Ala Pro Val Met Asn Gln
    770                 775                 780
gga gaa aca aac aag agt att tat ctt ccg cag ggg gat tgg atc gat 2640
Gly Glu Thr Asn Lys Ser Ile Tyr Leu Pro Gln Gly Asp Trp Ile Asp
785                 790                 795                 800
ttc tgg ttc ggt gct cag cgt cct ggc ggt cga aca atc agc tac acg 2688
Phe Trp Phe Gly Ala Gln Arg Pro Gly Gly Arg Thr Ile Ser Tyr Thr
                805                 810                 815
gcc ggc atc gat gat cta ccg gtt ttt gtg aag ttt ggc agt att ctt 2736
Ala Gly Ile Asp Asp Leu Pro Val Phe Val Lys Phe Gly Ser Ile Leu
            820                 825                 830
ccg atg aat ttg aac gcg caa tat caa gtg ggc ggg acc att ggc aac 2784
Pro Met Asn Leu Asn Ala Gln Tyr Gln Val Gly Gly Thr Ile Gly Asn
        835                 840                 845
agc ttg acg agc tac acg aat ctc gcg ttc cgc att tat ccg ctt ggg 2832
Ser Leu Thr Ser Tyr Thr Asn Leu Ala Phe Arg Ile Tyr Pro Leu Gly
    850                 855                 860
aca aca acg tac gac tgg aat gat gat att ggc ggt tcg gtg aaa acc 2880
Thr Thr Thr Tyr Asp Trp Asn Asp Asp Ile Gly Gly Ser Val Lys Thr
865                 870                 875                 880

ata act tct aca gag caa tat ggg ttg aat aaa gaa acc gtg act gtt 2928
Ile Thr Ser Thr Glu Gln Tyr Gly Leu Asn Lys Glu Thr Val Thr Val
                885                 890                 895
cca gcg att aat tct acc aag aca ttg caa gtg ttt acg act aag cct 2976
Pro Ala Ile Asn Ser Thr Lys Thr Leu Gln Val Phe Thr Thr Lys Pro
            900                 905                 910
tcc tct gta acg gtg ggt ggt tct gtg atg aca gag tac agt act tta 3024
Ser Ser Val Thr Val Gly Gly Ser Val Met Thr Glu Tyr Ser Thr Leu
        915                 920                 925
act gcc cta acg gga gcg tcg aca ggc tgg tac tat gat act gta cag 3072
Thr Ala Leu Thr Gly Ala Ser Thr Gly Trp Tyr Tyr Asp Thr Val Gln
    930                 935                 940
aaa ttc act tac gtc aag ctt ggt tca agt gca tct gct caa tcc gtt 3120
Lys Phe Thr Tyr Val Lys Leu Gly Ser Ser Ala Ser Ala Gln Ser Val
945                 950                 955                 960
gtg cta aat ggc gtt aat aag gtg gaa tat gaa gca gaa ttc ggc gtg 3168
Val Leu Asn Gly Val Asn Lys Val Glu Tyr Glu Ala Glu Phe Gly Val
                965                 970                 975
caa agc ggc gtt tca acg aac acg aac cat gca ggt tat act ggt aca 3216
Gln Ser Gly Val Ser Thr Asn Thr Asn His Ala Gly Tyr Thr Gly Thr
            980                 985                 990
gga ttt gtg gac ggc ttt gag act ctt gga gac aat gtt gct ttt gat 3264
Gly Phe Val Asp Gly Phe Glu Thr Leu Gly Asp Asn Val Ala Phe Asp
        995                 1000                1005
gtt tcc gtc aaa gcc gca ggt act tat acg atg aag gtt cgg tat tca 3312
Val Ser Val Lys Ala Ala Gly Thr Tyr Thr Met Lys Val Arg Tyr Ser
    1010                1015                1020
tcc ggt gca ggc aat ggc tca aga gcc atc tat gtg aat aac acc aaa 3360
Ser Gly Ala Gly Asn Gly Ser Arg Ala Ile Tyr Val Asn Asn Thr Lys
1025                1030                1035                1040
gtg acg gac ctt gcc ttg ccg caa aca aca agc tgg gat aca tgg ggg 3408
Val Thr Asp Leu Ala Leu Pro Gln Thr Thr Ser Trp Asp Thr Trp Gly
                1045                1050                1055
act gct acg ttt agc gtc tcg ctg agt aca ggt ctc aac acg gtg aaa 3456
Thr Ala Thr Phe Ser Val Ser Leu Ser Thr Gly Leu Asn Thr Val Lys
            1060                1065                1070

gtc agc tat gat ggt acc agt tca ctt ggc att aat ttc gat aac atc 3504
Val Ser Tyr Asp Gly Thr Ser Ser Leu Gly Ile Asn Phe Asp Asn Ile
        1075                1080                1085
gcg att gta gag caa taa 3522
Ala Ile Val Glu Gln
    1090
aaggtcggga gggcaagtcc ctcccttaat ttctaatcga aagggagtat ccttgatgcg 3582
tccaccaaac aaagaaattc cacgtattct tgcttttttt acagcgttta cgttgtttgg 3642
ttcaaccctt gccttgcttc ctgctccgcc tgcgcatgcc tatgtcagca gcctagggga 3702
aaatctcatt tcttcgagtg tcaccggaga taccttgacg ctaactgttg ataacggtgc 3762
gccgagtgat gacctcttga ttgttcaagc ggtgcaaaac ggtattttga aggtggatta 3822
tcgtccaaat agcataacgc cgagcgcgaa gacgccgatg ctggatc 3869